(19) Europäisches Patentamt
European Patent Office
Office européen des brevets

(11) EP 2 694 963 B1

(12) # EUROPEAN PATENT SPECIFICATION

(45) Date of publication and mention of the grant of the patent:
**02.08.2017 Bulletin 2017/31**

(21) Application number: **12765931.6**

(22) Date of filing: **02.04.2012**

(51) Int Cl.:
***G01N 33/50*** *(2006.01)* ***C12Q 1/68*** *(2006.01)*
***C40B 40/08*** *(2006.01)*

(86) International application number:
**PCT/US2012/031880**

(87) International publication number:
**WO 2012/135845 (04.10.2012 Gazette 2012/40)**

(54) **GENE EXPRESSION SIGNATURE FOR WNT/B-CATENIN SIGNALING PATHWAY AND USE THEREOF**

GENEXPRESSIONSSIGNATUR FÜR DEN WNT/B-CATENIN-SIGNALWEG UND VERWENDUNG DAVON

SIGNATURE D'EXPRESSION GÉNIQUE POUR LA VOIE DE SIGNALISATION Wnt/CATÉNINE ET UTILISATION DE CELLE-CI

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **01.04.2011 US 201161470919 P**

(43) Date of publication of application:
**12.02.2014 Bulletin 2014/07**

(73) Proprietor: **Qiagen**
**Gaithersburg, Maryland 20878 (US)**

(72) Inventors:
- **WU, Zhong**
  **Poolesville,MD 20837 (US)**
- **DICARLO, John**
  **Bethesda, Maryland 20817 (US)**
- **WANG, Yexun**
  **Ellicott City, Maryland 21042 (US)**

(74) Representative: **Boult Wade Tennant**
**Verulam Gardens**
**70 Gray's Inn Road**
**London WC1X 8BT (GB)**

(56) References cited:
**US-A1- 2008 318 234** **US-A1- 2010 169 025**
**US-B2- 7 939 263**

- **HUANG MOLI ET AL: "Identification of genes regulated by Wnt/[beta]-catenin pathway and involved in apoptosis via microarray analysis", BMC CANCER, BIOMED CENTRAL, LONDON, GB, vol. 6, no. 1, 7 September 2006 (2006-09-07), page 221, XP021016219, ISSN: 1471-2407, DOI: 10.1186/1471-2407-6-221**
- **LEE ET AL: "Novel candidate targets of Wnt/beta-catenin signaling in hepatoma cells", LIFE SCIENCES, PERGAMON PRESS, OXFORD, GB, vol. 80, no. 7, 13 January 2007 (2007-01-13), pages 690-698, XP005830179, ISSN: 0024-3205, DOI: 10.1016/J.LFS.2006.10.024**
- **SHIN H ET AL: "Identification of transcriptional targets of Wnt/beta-catenin signaling in dermal papilla cells of human scalp hair follicles: EP2 is a novel transcriptional target of Wnt3a", JOURNAL OF DERMATOLOGICAL SCIENCE, ELSEVIER SCIENCE PUBLISHERS, SHANNON, IE, vol. 58, no. 2, 1 May 2010 (2010-05-01), pages 91-96, XP027019046, ISSN: 0923-1811 [retrieved on 2010-03-06]**
- **HULIT ET AL.: 'p27Kip1 repression of ErbB2-induced mammary tumor growth in transgenic mice involves Skp2 and Wnt/beta-catenin signaling.' CANCER RES. vol. 66, no. 17, 01 September 2006, pages 8529 - 8541, XP055128328**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- LIVAK ET AL.: 'Analysis of relative gene expression data using real-time quantitative PCR and the 2(-Delta Delta C(T)) Method' METHODS vol. 25, no. 4, December 2001, pages 402 - 408, XP003015637
- TIAN ET AL.: 'Loss of CHSY1, a Secreted FRINGE Enzyme, Causes Syndromic Brachydactyly in Humans via Increased NOTCH Signaling.' AMERICAN JOURNAL OF HUMAN GENETICS vol. 87, no. 6, 10 December 2010, pages 768 - 778, XP055128329
- PORTER ET AL.: 'Bodl, a novel kinetochore protein required for chromosome biorientation.' JOURNAL OF CELL BIOLOGY vol. 179, no. 2, 22 October 2007, pages 187 - 197, XP055128330
- PONGRAC ET AL.: 'Heat shock protein 12A shows reduced expression in the prefrontal cortex of subjects with schizophrenia.' BIOLOGICAL PSYCHIATRY vol. 56, no. 12, 15 December 2004, pages 943 - 950, XP004682240
- TONDERA ET AL.: 'The mitochondrial protein MTP18 contributes to mitochondrial fission in mammalian cells.' J CELL SCI vol. 118, no. 14, 15 July 2005, pages 3049 - 3059, XP055128332
- WANG ET AL.: 'Genomic and Biochemical Approaches in the discovery of mechanisms for selective neuronal vulnerability to oxidative stress.' BMC NEUROSCIENCE vol. 10, no. 12, 19 February 2009, page 12, XP021048323

**Description**

## BACKGROUND

### 1. Field of the Invention

The present invention relates to a novel set of markers, microarrays containing, and an expression signature comprising 16 genes or a subset thereof and the use thereof in determining the regulation status of Wnt/β-catenin signaling pathway in a cell sample or subject. The regulation status of Wnt/β-catenin signaling pathway in a cell sample or subject may be assayed based on the level of expression of one or more of these genes. More specifically, the invention provides a set of genes which can be used as biomarkers and as gene signatures for evaluating Wnt/β-catenin pathway deregulation status in a sample; classifying a cell sample as having a deregulated or regulated Wnt/β-catenin signaling pathway; determining whether an agent modulates the Wnt/β-catenin signaling pathway in sample; predicting response of a subject to an agent that modulates the Wnt/β-catenin signaling pathway; assigning treatment to a subject; and evaluating the pharmacodynamic effects of therapies designed to regulate Wnt/β-catenin pathway signaling. In the invention expression of the provided biomarkers is preferably determined by RT-PCR using SYBR green and the expression data analyzed and compared to a control sample by use of the support vector machine method.

### 2. Description of Related Art

Upon Wnt receptor activation, three different signaling cascades are activated (Huelsken and Birchmeier, 2001, Curr. Opin. Genet. Dev. 11:547-553): 1) the Wnt/Ca2+ pathway, which leads to activation of the protein kinase C and the Ca2+ calmodulin dependent protein kinase II; 2) the cytoskeleton pathway, which regulates organization and formation of the cytoskeleton and planar cell polarity; and 3) the canonical Wnt pathway, which controls the intracellular level of the proto-oncoprotein β-catenin. The canonical pathway, also known as the "Wnt/b-catenin" pathway or "β-catenin" pathway is the most studied and the best understood among the three Wnt pathways (Clevers, 2006, Cell 127:469-480).

The key protein of the Wnt/β-catenin pathway is the proto-oncoprotein β-catenin, which can switch between two different intracellular pools. In the absence of the Wnt signal, β-catenin is bound to the cytoplasmic domain of the membrane anchored E-cadherin where it forms together with alpha.-cadherin a connecting bridge to the cytoskeletal protein actin. The β-catenin level in the cytosol is kept low by the so-called destruction complex, which is formed by the active serine-threonin kinase glycogen synthase kinase-3β (GSK3b) and several other cytosolic proteins including the tumor suppressor proteins APC (Adenomatous Polyposis coli) and Axin/Conductin. Phosphorylation of β-catenin by GSK3b leads to its ubiquitinylation via b-TrCP (β-Transducin repeat Containing Protein) and to its degradation by the proteasomal degradation machinery. The activation of the Wnt/β-catenin pathway begins with the hetero-dimerization of the Wnt receptor Frizzled (Fz) with its co-receptor LRP5/6 (low-density lipoprotein receptor related protein). The subsequent hyperphosphorylation of Dishevelled (Dsh) by the activated casein kinase 2 (CK2) leads to the inhibition of GSK3b (Willert et al., 1997, EMBO J. 16:3089-3096). As a consequence, the destruction complex disassembles, β-catenin is not phosphorylated any more, and the level of cytosolic and nuclear β-catenin increases. Nuclear β-catenin interacts with T-cell factor/Lymphoid enhancer factor (TCF/Lef) and displaces co-repressors (Staal et al., 2002, EMBO Rep. 3:63-68). The β-catenin/TCF complex activates transcription of many different target genes.

Products of Wnt target genes unfold a large variety of biochemical functions including cell cycle kinase regulation, cell adhesion, hormone signaling, and transcription regulation. The plurality and diversity of the biochemical functions reflect the variety of different biological effects of the Wnt/b-catenin pathway, including activation of cell-cycle progression and proliferation, inhibition of apoptosis, regulation of embryonic development, cell differentiation, cell growth, and cell migration (reviewed in Vlad et al., 2008, Cellular Signaling 20:795-802). Numerous target genes of the b-catenin/TCF complex have been identified and may be found on the Wnt homepage, http://www.stanford.edu/~rnusse/wntwindow.html.

Wnt/β-catenin signaling is involved in adult tissue self-renewal. The Wnt/β-catenin cascade may be required for establishment of the progenitor compartment in the intestinal epithelium (Korinek et al., 1998, Nat. Genet. 19:1-5). Wnt proteins also promote the terminal differentiation of Paneth cells at the base of the intestinal crypts (Van Es et al., 2005, Nature 435:959-963). Wnt/β-catenin signaling is required for the establishment of the hair follicle (van Genderen et al., 1994, Genes Dev. 8:2691-2703) Wnt/β-catenin signals in hair follicles activates bulge stem cells, promotes entry into the hair lineage, and recruits the cells to the transit-amplifying matrix compartment (Lowry et al., 2005, 19:1596-1611; Huelsken et al., 2001, 105:533-545). The Wnt/β-catenin pathway is also an important regulator of hematopoietic stem and progenitor cells and bone homeostasis (reviewed in Clevers, 2006, Cell 469-480).

Wnt/β-catenin signaling is also implicated in cancer. Germline APC mutation is the genetic cause of Familiar Adenomatous Polyposis (FAP) (Kinzler et al., 1991; Nishisho et al., 1991). Loss of both APC alleles occurs in a large majority of sporadic colorectal cancers. β-catenin is inappropriately stabilized as a consequence of the loss of APC (Rubinfeld et al., 1996). These mutations activate β-catenin signaling, inhibit cellular differentiation, increase cellular proliferation, and ultimately result in the formation of precancerous intestinal polyps (Gregorieff and Clevers, 2005, Genes Dev. 19:877-890; Logan and Nusse, 2004, Annu. Rev. Cell Dev. Biol. 20:781-810). In rare cases of colorectal cancer where APC is not mutated, Axin 2 is mutant (Liu et al., 2000), or β-catenin has an activating point mutation that removes its N-terminal Ser/Thr destruction motif (Morin et al., 1997). Activating Wnt/β-catenin pathway mutations are not limited to intestinal cancer. Loss-of-function Axin mutations have also been found in hepatocellular carcinomas, and oncogenic β-catenin mutations occur in a wide variety of solid tumors (reviewed in Reya and Clevers, 2005). Mutational activation of the Wnt/β-catenin cascade may also be involved in hair follicle tumors (reviewed in Clevers, 2006, Cell 469-480). Inactivating mutations in the Wnt/β-catenin signaling pathway have also been identified in human sebaceous tumors, which carried LEF1 mutations (Takeda et al, 2006). Wnt/β-catenin signaling is also implicated in cancer stem cell regulation (Malanchi et al., 2008, 452:650-653; reviewed by Fodde and Brabletz, 2007, Curr. Opin. Cell Biol. 19:150-158).

The identification of patient subpopulations most likely to respond to therapy is a central goal of modern molecular medicine. This notion is particularly important for cancer due to the large number of approved and experimental therapies (Rothenberg et al., 2003, Nat. Rev. Cancer 3:303-309), low response rates to many current treatments, and clinical importance of using the optimal therapy in the first treatment cycle (Dracopoli, 2005, Curr. Mol. Med. 5:103-110). In addition, the narrow therapeutic index and severe toxicity profiles associated with currently marketed cytotoxics results in a pressing need for accurate response prediction. Although recent studies have identified gene expression signatures associated with response to cytotoxic chemotherapies (Folgueria et al., 2005, Clin. Cancer Res. 11:7434-7443; Ayers et al., 2004, 22:2284-2293; Chang et al., 2003, Lancet 362:362-369; Rouzier et al., 2005, Proc. Natl. Acad. Sci. USA 102: 8315-8320), these examples (and others from the literature) remain unvalidated and have not yet had a major effect on clinical practice. In addition to technical issues, such as lack of a standard technology platform and difficulties surrounding the collection of clinical samples, the myriad of cellular processes affected by cytotoxic chemotherapies may hinder the identification of practical and robust gene expression predictors of response to these agents. One exception may be the recent finding by microarray that low mRNA expression of the microtubule-associate protein Tau is predictive of improved response to paclitaxel (Rouzier et al., supra).

To improve on the limitations of cytotoxic chemotherapies, current approaches to drug design in oncology are aimed at modulating specific cell signaling pathways important for tumor growth and survival (Hahn and Weinberg, 2002, Nat. Rev. Cancer 2:331-341; Hanahan and Weinberg, 2000, Cell 100:57-70; Trosko et al., 2004, Ann. N.Y. Acad. Sci. 1028:192-201). In cancer cells, these pathways become deregulated resulting in aberrant signaling, inhibition of apoptosis, increased metastasis, and increased cell proliferation (reviewed in Adjei and Hildalgo, 2005, J. Clin. Oncol. 23:5386-5403). Although normal cells integrate multiple signaling pathways for controlled growth and proliferation, tumors seem to be heavily reliant on activation of one or two pathways ("oncogene activation"). Aberrant Wnt/β-catenin pathway signaling can cause cancer and a number of genetic defects in this pathway may contribute to tumor promotion and progression (reviewed in Polakis, 2000, Genes Dev. 14:1837-1851). Hyperactivation of the Wnt/β-catenin pathway is one of the most frequent signaling abnormalities in several human cancers, including colorectal carcinomas (Morin et al., 1997, Science 275:1787-1790), melanomas (Rubinfeld et al., 1997, Science 275:1790-1792), hepatoblastomas (Koch et al., 1999, Cancer Res. 59:269-273), medulloblastomas (Zurawel et al, 1998, Cancer Res. 58:896-899), prostatic carcinomas (Voeller et al, 1998, Cancer Res. 58:2520-2523), and uterine and ovarian endometrioid adenocarcinomas (Schlosshauer et al., 2000, Mod. Pathol. 13:1066-1071; Mirabelli-Primdahl et al, 1999, Cancer Res. 59:3346-3351; Saegusa and Okayasu, 2001, J. Pathol. 194:59-67; Wu et al., 2001, Cancer Res. 61:8247-8255). Wnt/β-catenin pathway activation is also common in metaplastic carcinomas of the breast (Hayes et al., 2008, Clin. Cancer Res. 14:4038-4044). The components of these aberrant signaling pathways represent attractive selective targets for new anticancer therapies. In addition, responder identification for target therapies may be more achievable than for cytotoxics, as it seems logical that patients with tumors that are "driven" by a particular pathway will respond to therapeutics targeting components of that pathway. Therefore, it is crucial that we develop methods to identify which pathways are active in which tumors and use this information to guide therapeutic decisions. One way to enable this is to identify gene expression profiles that are indicative of pathway activation status.

A multitude of pathway components may activate, modify, or inhibit Wnt/β-catenin signaling at multiple points or may be involved in crosstalk to other pathways. Measuring pathway activity by testing only a few well-characterized pathway components may miss other important pathway mediators. Given its involvement in numerous biological functions and diseases, a gene expression signature-based readout of pathway activation may be more appropriate than relying on a single indicator of pathway activity, as the same signature of gene expression may be elicited by activation of multiple components of the pathway. In addition, by integrating expression data from multiple genes, a quantitative assessment of pathway activity may be possible. In addition to using gene expression signatures for classification cell samples, including but not limited to tumors, by assessing pathway activation status, gene expression signatures for pathway activation may also be used as pharmacodynamic biomarkers, i.e. monitoring pathway inhibition in patient tumors or peripheral tissues post-treatment; as response prediction biomarkers, i.e. prospectively identifying patients harboring tumors that have high levels of a particular pathway activity before treating the patients with inhibitors targeting the pathway; and as early efficacy biomarkers, i.e. an early readout of efficacy. A gene expression signature for pathway activity may also be used to screen for agents that modulate pathway signaling.

A recent patent application, United States Patent Application 20100169025, entitled "METHODS AND GENE EXPRESSION SIGNATURE FOR WNT/B-CATENIN SIGNALING PATHWAY" by Arthur et al. and assigned to Merck which published on July 1, 2010 purports to disclose a set of genes the expression of which are purported to correlate to Wnt/catenin signaling and regulation. However, this patent application relates to a distinct set of 38 genes none of which overlap with the present invention. While Applicants do not wish to be bound by their theory this may be because in their methods the inventors used a different type of sample and method to identify the putative Wnt/β-catenin signature. Particularly they used cell lines with constitutive active Wnt/β-catenin signaling (DLD1, SW480 and SW620) which were transfected with β-catenin siRNA. As disclosed in detail infra, the present inventors instead utilized cell lines wherein Wnt/β-catenin signaling status was altered by use of a specific ligand (Wnt3a) stimulation and LiCl treatment or β-catenin siRNA treatment.

In addition they used different mathematical methods for deriving and validating their Wnt/β-catenin signature genes. They used a Monte-Carlo technique to calculate the significance of the set of biomarker genes. In this method validation of the marker set may be accomplished by leaving one out and survival model. Examples of classification (pattern recognition) methods include: profile similarity; artificial neural network; support vector machine (SVM); logic regression, linear or quadratic discriminant analysis, decision trees, clustering, principal component analysis, and nearest neighbor classifier analysis. In contrast to Merck, the present inventors relied on the support vector machine (SVM) method to derive the unique Wnt/β-catenin gene signature disclosed herein.

Still further in the Merck patent application examples they used as the early time point (24h) of siRNA and compared gene expression in colon tumor samples vs normal samples. By contrast, the present invention identified the gene signature using cell lines stimulated with Wnt3a and LiCl or inhibited with /β-catenin siRNA.

Huang et al. (BMC Cancer, 2006, 6(1), 221) used a stably integrated, inducible RNA interference (RNAi) vector to specifically inhibit the expression and the transcriptional activity of beta-catenin in HeLa cells followed by microarray analysis and RT-PCT to identify differentially expressed genes. Lee et al. (Life Sciences, 2007, 80(7), 690-698) describes analysis of the transcriptome profile of human hepatoma cell lines using cDNA microarrays representing 15,127 unique, liver-enriched gene loci. Shin et al. (Journal of dermatological Science, 2010, 58(2), 91-96) describes an investigation to assess the response to Wnt3a in dermal papilla cells.

DETAILED DESCRIPTION OF THE FIGURES

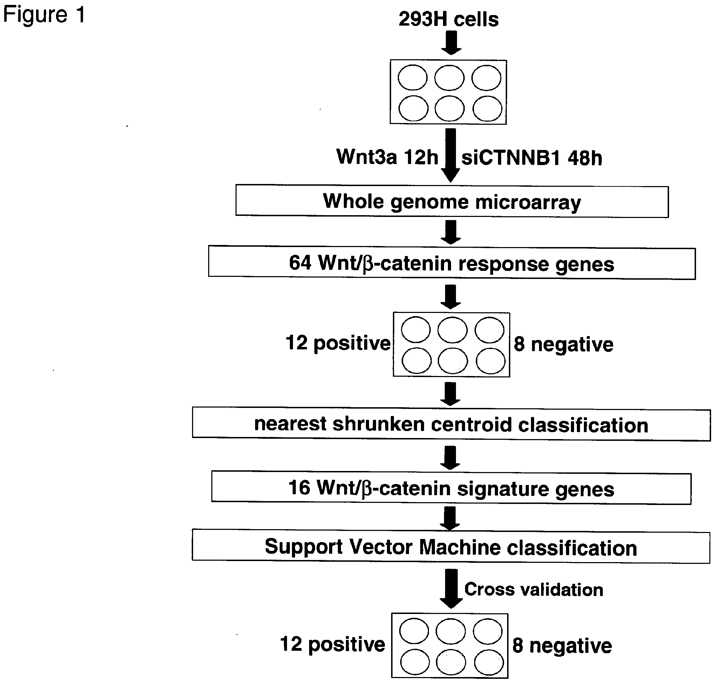

Figure 1 contains a schematic depicting an overview of the experiments that resulted in the identification of a unique gene signature profile that may be used to screen for agents that modulate pathway signaling and/or to assay the regulatory status of Wnt/β-catenin pathway activity in a cell sample.

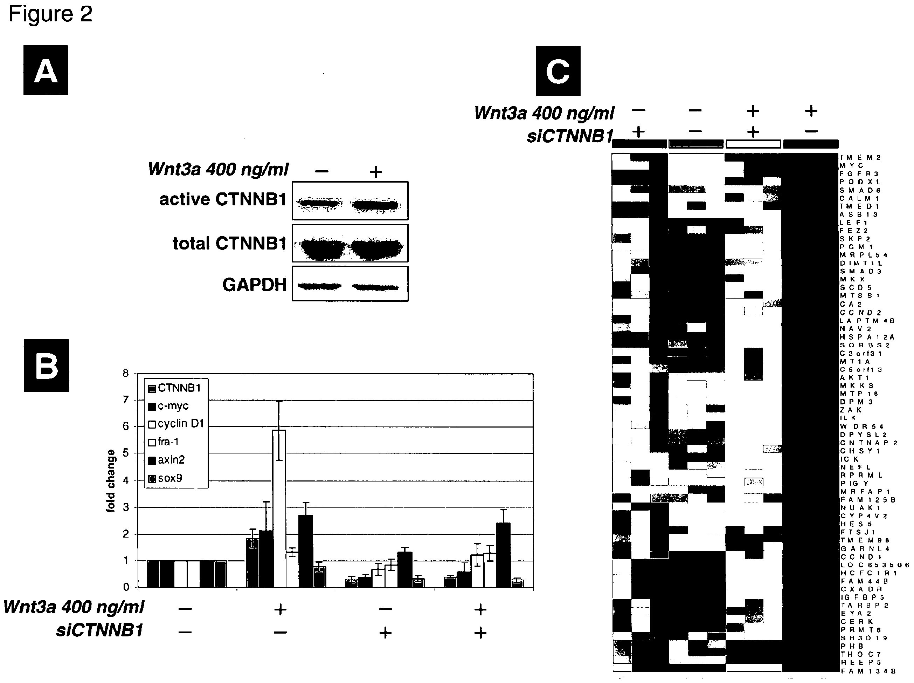

Figure 2A-C depict the results of experiments wherein 293H cells were transfected with siRNA targeting β-catenin (CTNNB1) for 48 hours and Wnt3a was added in the last 12 hours. Activation of the Wnt/β-catenin pathway was confirmed by increased protein levels of active β-catenin (Panel A) and upregulation of target genes (Panel B). Whole genome microarray analysis identified the shown 64 Wnt/β-catenin response genes (Panel C).

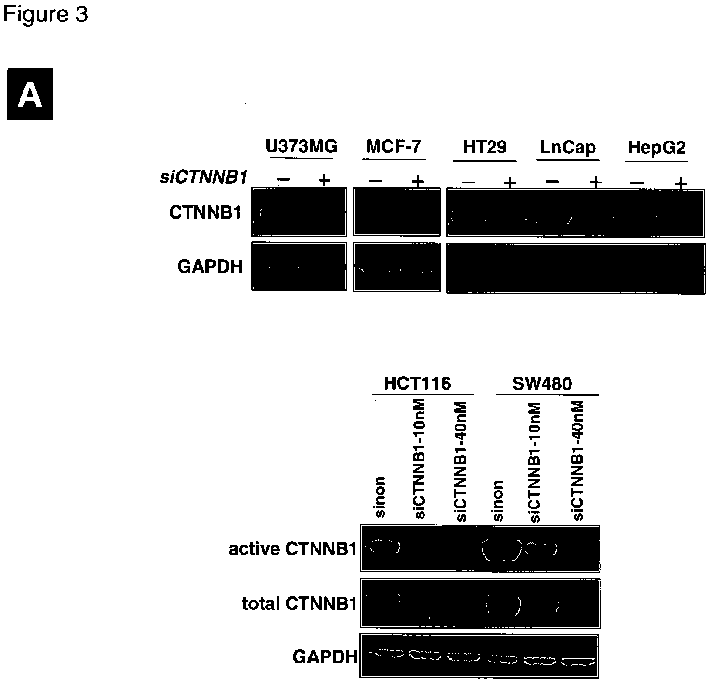

Figure 3A-B shows alteration in expression of CTNNB1 (the gene encoding β-catenin) for a training dataset of 20 samples which were used for Wnt/β-catenin pathway signature identification. In the experiments twelve samples were stimulated with either Wnt3a or LiCl (Panel B shows 11 samples) while eight samples were transfected with siRNA targeting β-catenin (CTNNB1) (Panel A shows 7 samples).

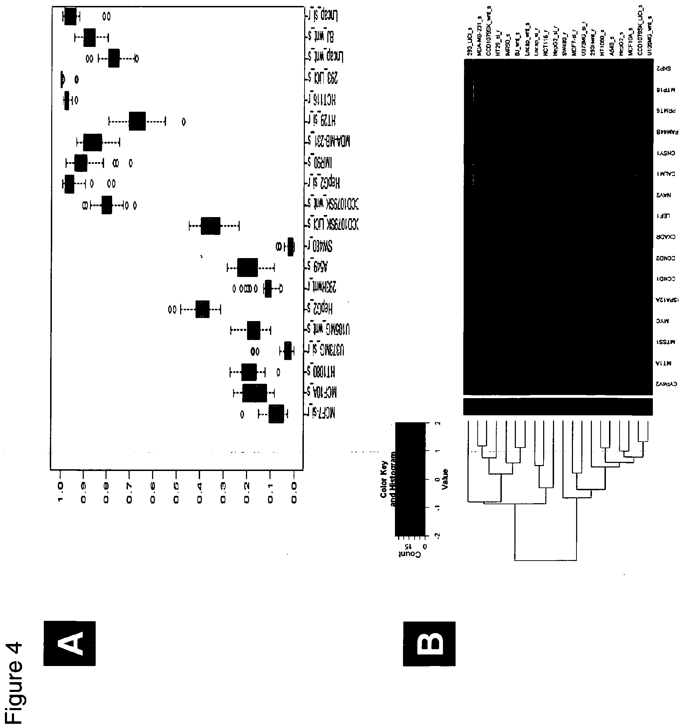

Figure 4A-B contain results of cross validation of the 16 Wnt/β-catenin gene signature. In these experiments the 16 Wnt/β-catenin gene signature was verified by cross validation on twenty samples with Support Vector Machine classification method (Panel A). As can be seen, the 16 gene signature correctly predicated the regulatory status of twenty samples in cross validation process (Panel A). A heatmap showing the PCR expression data of 16 signature genes across 20 samples is also shown (Panel B).

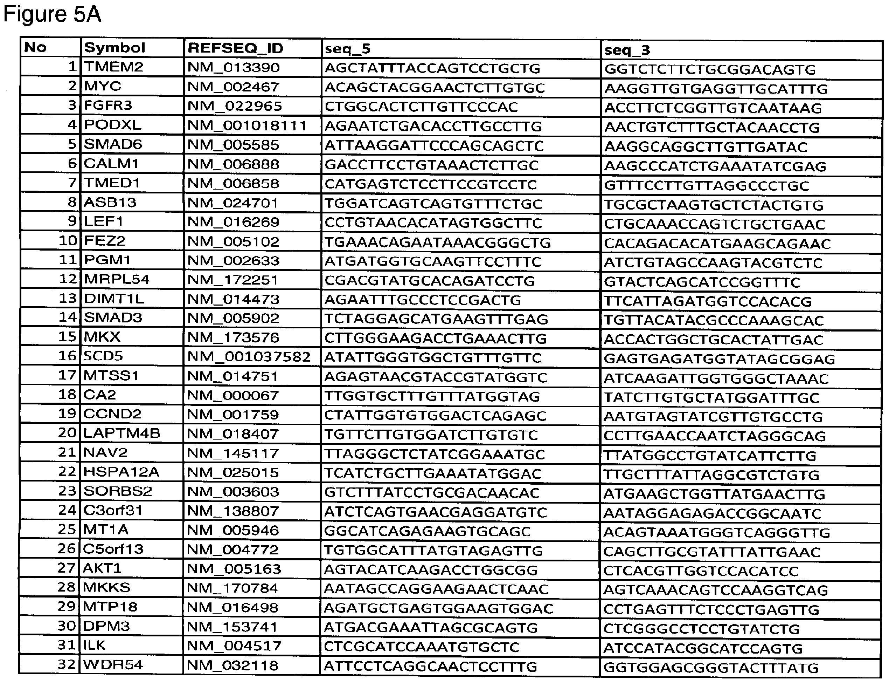

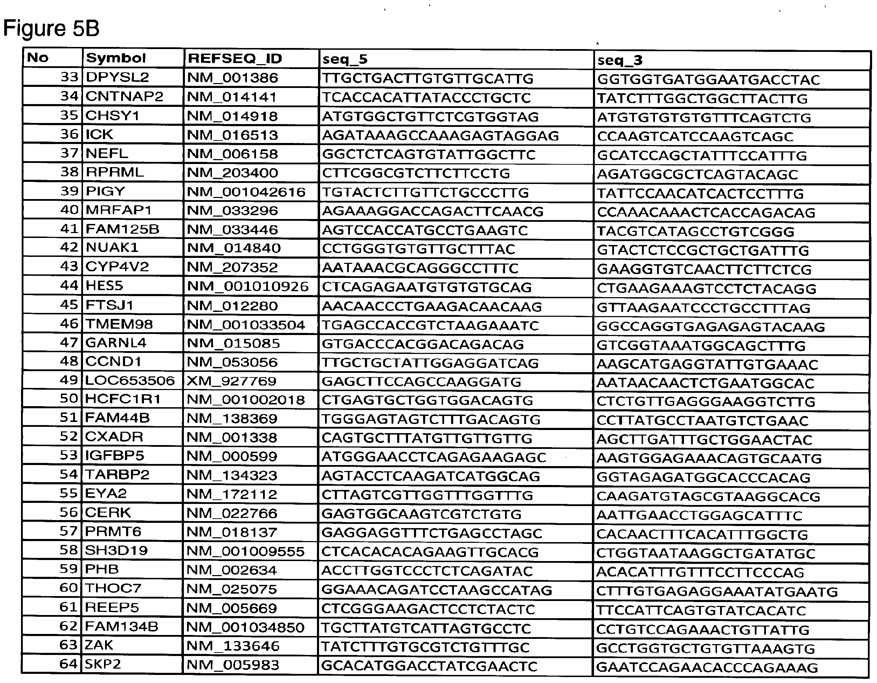

Figure 5A-B contains the sequence information for all primers used to validate 64 Wnt/β-catenin response genes with real-time PCR.

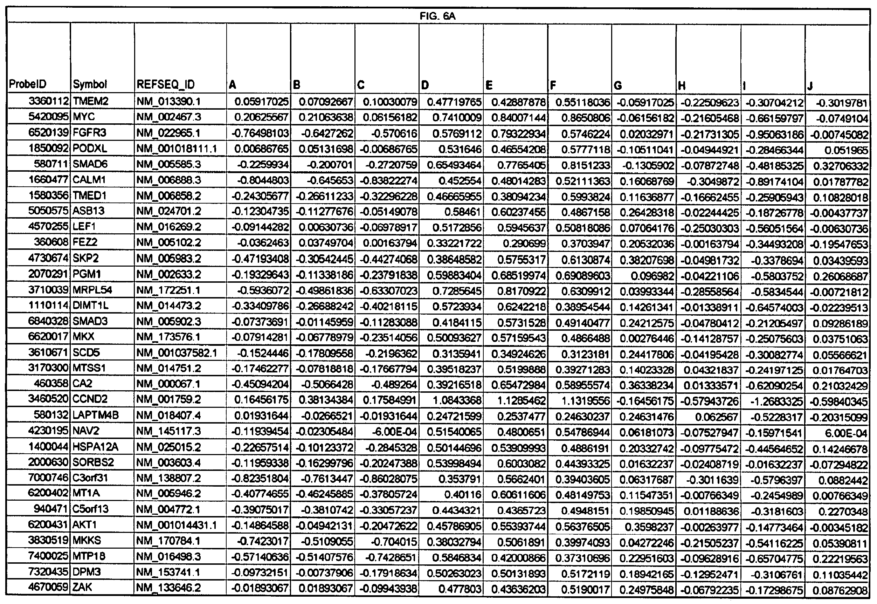

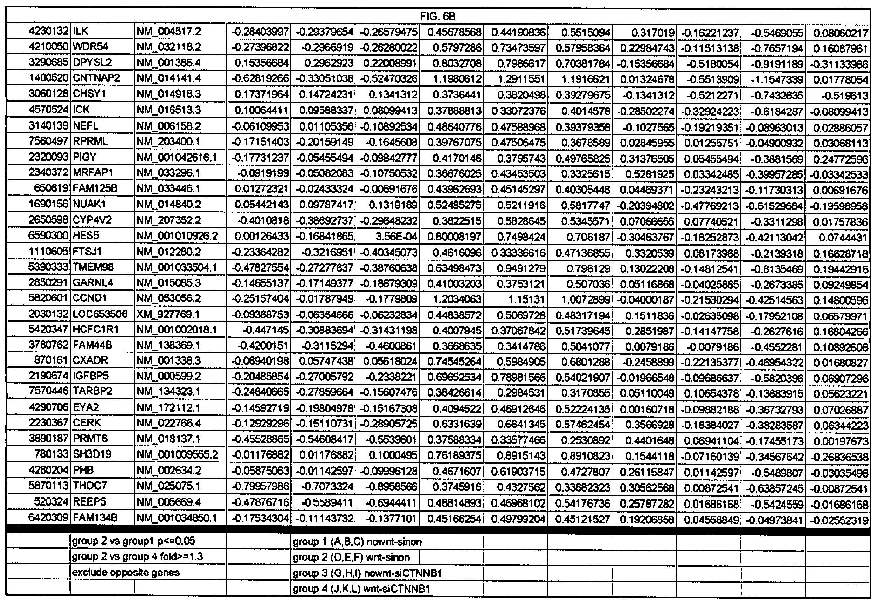

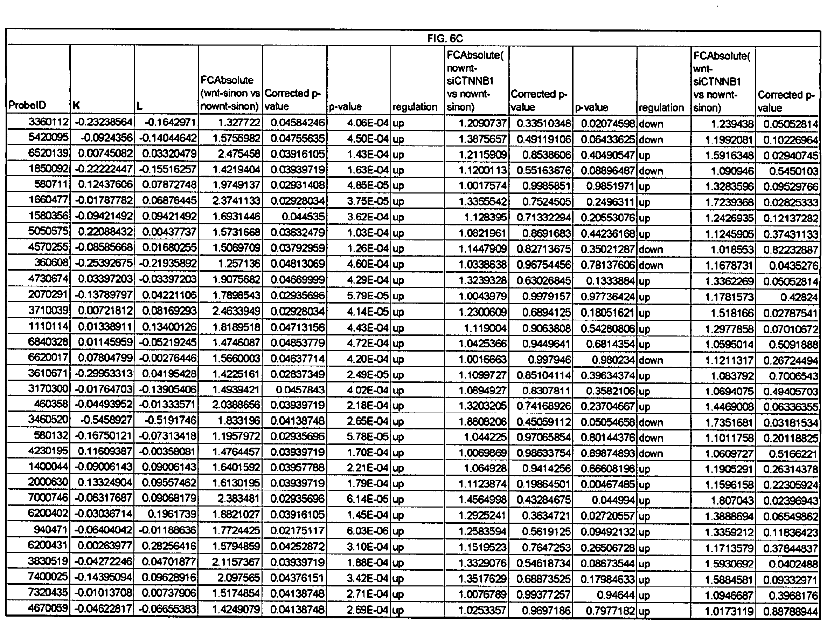

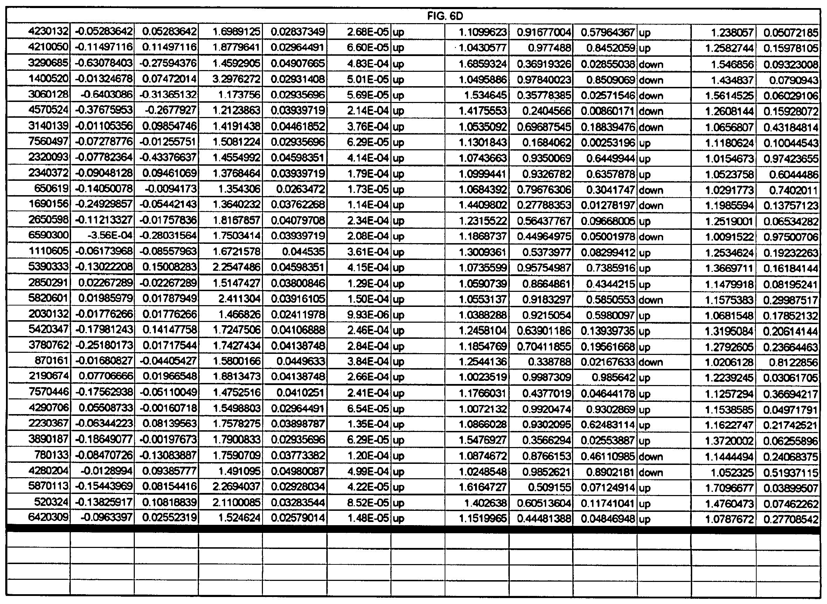

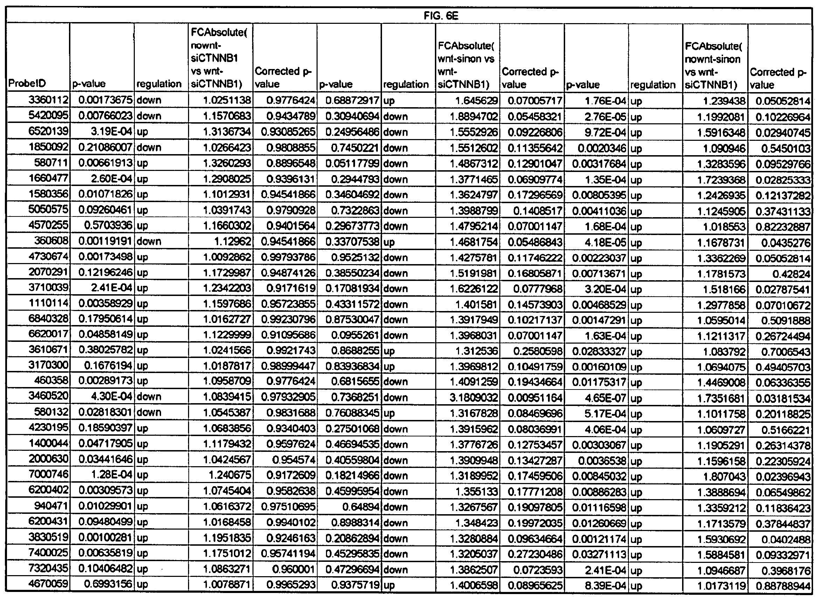

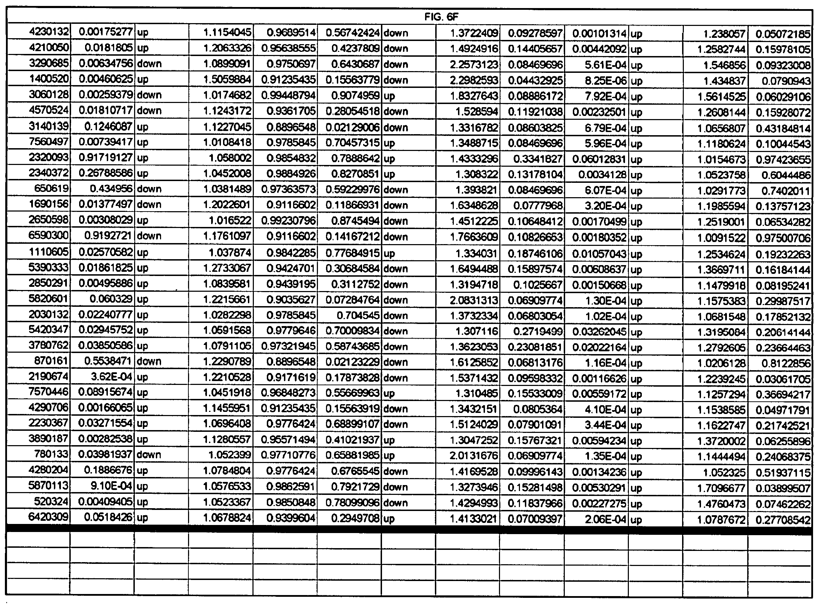

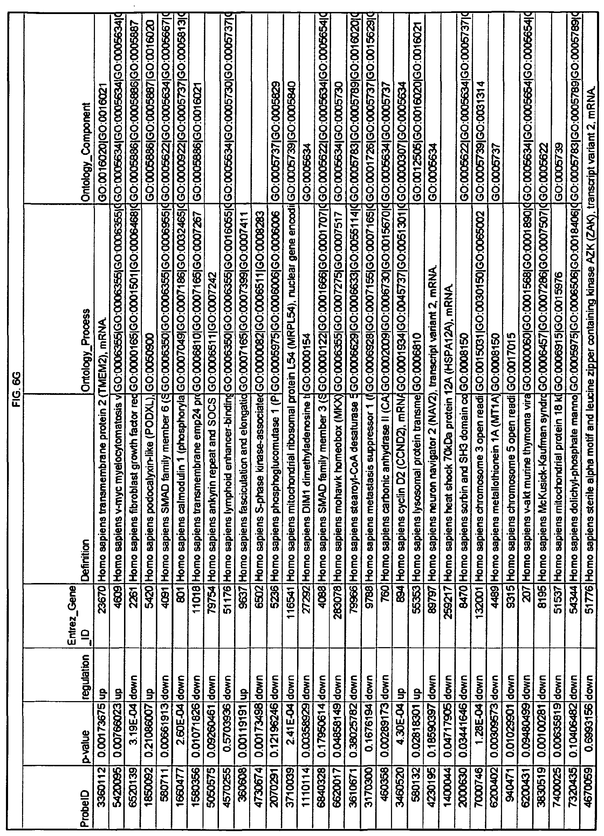

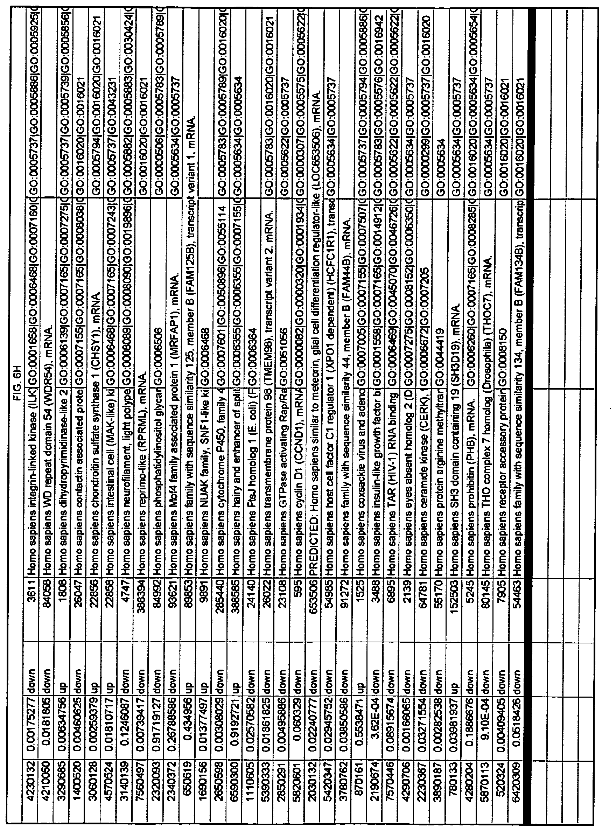

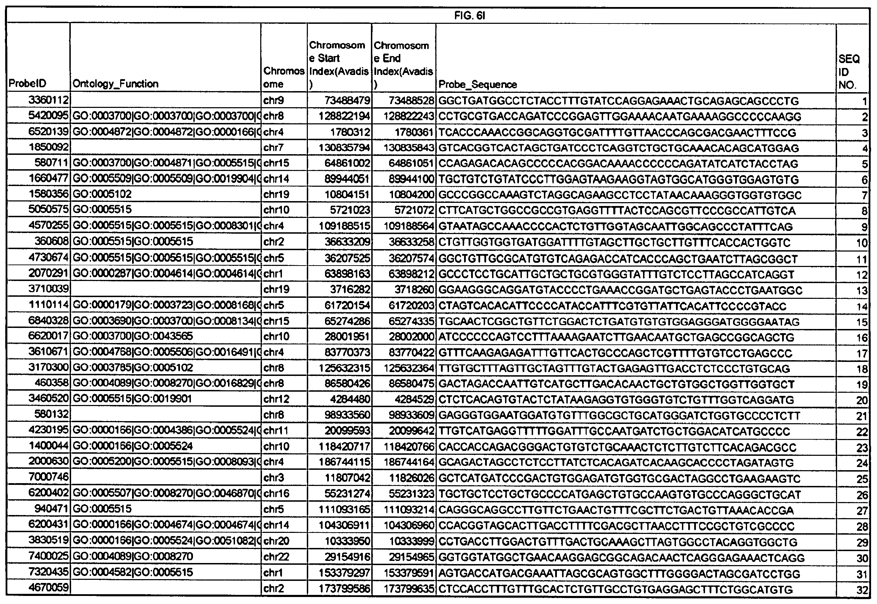

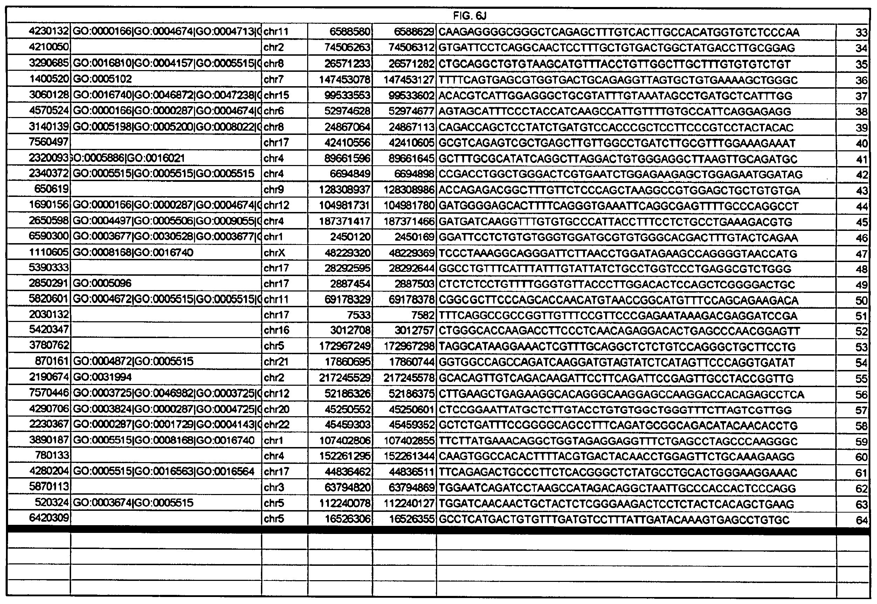

Figure 6 shows results of gene expression profiling and identification of Wnt/b-catenin response genes. 64 genes were identified as Wnt/β-catenin response genes due to their expression being consistently affected by Wnt3a and siRNA treatment.

## DETAILED DESCRIPTION

Prior to disclosing the invention in detail the following definitions are provided. Unless defined otherwise, all technical and scientific terms used herein have the same meaning as commonly understood to one of ordinary skill in the art to which this invention belongs.

As used herein, oligonucleotide sequences that are complementary to one or more of the genes described herein, refers to oligonucleotides that are capable of hybridizing under stringent conditions to at least part of the nucleotide sequence of said genes. Such hybridizable oligonucleotides will typically exhibit at least about 75% sequence identity at the nucleotide level to said genes, preferably about 80% or 85% sequence identity or more preferably about 90% or 95% or more sequence identity to said genes.

"Bind(s) substantially" refers to complementary hybridization between a probe nucleic acid and a target nucleic acid and embraces minor mismatches that can be accommodated by reducing the stringency of the hybridization media to achieve the desired detection of the target polynucleotide sequence.

The phrase "hybridizing specifically to" refers to the binding, duplexing or hybridizing of a molecule substantially to or only to a particular nucleotide sequence or sequences under stringent conditions when that sequence is present in a complex mixture (e.g., total cellular) DNA or RNA.

"Biomarker" means any gene, protein, or an EST derived from that gene, the expression or level of which changes between certain conditions. Where the expression of the gene correlates with a certain condition, the gene is a biomarker for that condition.

"Biomarker-derived polynucleotides" means the RNA transcribed from a biomarker gene, any cDNA or cRNA produced therefrom, and any nucleic acid derived therefrom, such as synthetic nucleic acid having a sequence derived from the gene corresponding to the biomarker gene.

A gene marker is "informative" for a condition, phenotype, genotype or clinical characteristic if the expression of the gene marker is correlated or anti-correlated with the condition, phenotype, genotype or clinical characteristic to a greater degree than would be expected by chance.

As used herein, the term "gene" has its meaning as understood in the art. However, it will be appreciated by those of ordinary skill in the art that the term "gene" may include gene regulatory sequences (e.g., promoters, enhancers, etc.) and/or intron sequences. It will further be appreciated that definitions of gene include references to nucleic acids that do not encode proteins but rather encode functional RNA molecules such as tRNAs. For clarity, the term gene generally refers to a portion of a nucleic acid that encodes a protein; the term may optionally encompass regulatory sequences. This definition is not intended to exclude application of the term "gene" to non-protein coding expression units but rather to clarify that, in most cases, the term as used in this document refers to a protein coding nucleic acid. In some cases, the gene includes regulatory sequences involved in transcription, or message production or composition. In other embodiments, the gene comprises transcribed sequences that encode for a protein, polypeptide or peptide. In keeping with the terminology described herein, an "isolated gene" may comprise transcribed nucleic acid(s), regulatory sequences or coding sequences, isolated substantially away from other such sequences, such as other naturally occurring genes, regulatory sequences, polypeptide or peptide encoding sequences, etc. In this respect, the term "gene" is used for simplicity to refer to a nucleic acid comprising a nucleotide sequence that is transcribed, and the complement thereof. In particular embodiments, the transcribed nucleotide sequence comprises at least one functional protein, polypeptide and/or peptide encoding unit. As will be understood by those in the art, this functional term "gene" includes both genomic sequences, RNA or cDNA sequences, or smaller engineered nucleic acid segments, including nucleic acid segments of a non-transcribed part of a gene, including but not limited to the non-transcribed promoter or enhancer regions of a gene. Smaller engineered gene nucleic acid segments may express, or may be adapted to express using nucleic acid manipulation technology, proteins, polypeptides, domains, peptides, fusion proteins, mutants and/or such like. The sequences which are located 5' of the coding region and which are present on the mRNA are referred to as 5' untranslated sequences ("5'UTR"). The sequences which are located 3' or downstream of the coding region and which are present on the mRNA are referred to as 3' untranslated sequences, or ("3'UTR").

"Signature" refers to the differential expression pattern. It could be expressed as the number of individual unique probes whose expression is detected when a cRNA product is used in microarray analysis. It could also be expressed as the number of individual genes whose expression is detected with real time RT-PCR. A signature may be exemplified by a particular set of biomarkers.

A "similarity value" is a number that represents the degree of similarity between two things being compared. For example, a similarity value may be a number that indicates the overall similarity between a cell sample expression profile using specific phenotype-related biomarkers and a control specific to that template (for instance, the similarity to a "deregulated Wnt/b-catenin signaling pathway" template, where the phenotype is deregulated Wnt/b-catenin signaling pathway status). The similarity value may be expressed as a similarity metric, such as a correlation coefficient, or a classification probability or may simply be expressed as the expression level difference, or the aggregate of the expression level differences, between a cell sample expression profile and a baseline template.

As used herein, the terms "measuring expression levels," "obtaining expression level," and "detecting an expression level", includes methods that quantify a gene expression level of, for example, a transcript of a gene, or a protein encoded by a gene, as well as methods that determine whether a gene of interest is expressed at all. Thus, an assay which provides a "yes" or "no" result without necessarily providing quantification, of an amount of expression is an assay that "measures expression" as that term is used herein. Alternatively, a measured or obtained expression level may be expressed as any quantitative value, for example, a fold-change in expression, up or down, relative to a control gene or relative to the same gene in another sample, or a log ratio of expression, or any visual representation thereof, such as, for example, a "heatmap" where a color intensity is representative of the amount of gene expression detected. The genes identified as being differentially expressed in tumor cells having Wnt/b-catenin signaling pathway deregulation may be used in a variety of nucleic acid or protein detection assays to detect or quantify the expression level of a gene or multiple genes in a given sample. Exemplary methods for detecting the level of expression of a gene include, but are not limited to, Northern blotting, dot or slot blots, reporter gene matrix (see for example, U.S. Pat. No. 5,569,588) nuclease protection, RT-PCR, microarray profiling, differential display, 2D gel electrophoresis, SELDI-TOF, ICAT, enzyme assay, antibody assay and MNAzyme-based detection methods (see U.S. Ser. No. 61/470,919, US 2011/0143338; US 2007/0231810; WO WO/2008/122084; WO/2007/041774; and Mokany et al., J Am Chem Soc. 2010 January 27; 132(3): 1051-1059,). Optionally a gene whose level of expression is to be detected may be amplified, for example by methods that may include one or more of: polymerase chain reaction (PCR), strand displacement amplification (SDA), loop-mediated isothermal amplification (LAMP), rolling circle amplification (RCA), transcription-mediated amplification (TMA), self-sustained sequence replication (3SR), nucleic acid sequence based amplification (NASBA), or reverse transcription polymerase chain reaction (RT-PCR). In the preferred embodiment gene expression will be detected by RT-PCR, preferably using SYBR green.

A "patient" can mean either a human or non-human animal, preferably a mammal.

As used herein, "subject", as refers to an organism or to a cell sample, tissue sample or organ sample derived therefrom, including, for example, cultured cell lines, biopsy, blood sample, or fluid sample containing a cell. In many instances, the subject or sample derived therefrom, comprises a plurality of cell types. In one embodiment, the sample includes, for example, a mixture of tumor and normal cells. In one embodiment, the sample comprises at least 10%, 15%, 20%, et seq., 90%, or 95% tumor cells. The organism may be an animal, including but not limited to, an animal, such as a cow, a pig, a mouse, a rat, a chicken, a cat, a dog, etc., and is usually a mammal, such as a human.

As used herein, the term "pathway" is intended to mean a set of system components involved in two or more sequential molecular interactions that result in the production of a product or activity. A pathway can produce a variety of products or activities that can include, for example, intermolecular interactions, changes in expression of a nucleic acid or polypeptide, the formation or dissociation of a complex between two or more molecules, accumulation or destruction of a metabolic product, activation or deactivation of an enzyme or binding activity. Thus, the term "pathway" includes a variety of pathway types, such as, for example, a biochemical pathway, a gene expression pathway, and a regulatory pathway. Similarly, a pathway can include a combination of these exemplary pathway types.

"Wnt signaling pathway," also known as the "Wnt/b-catenin signaling pathway" or "b-catenin signaling pathway" refers to one of the intracellular signaling pathways activated upon Wnt receptor activation, the canonical Wnt/b-catenin signaling pathway, which controls the intracellular level of the proto-oncoproteinb-catenin. On activation of the Wnt signaling pathway by binding with the Wnt ligand (including, but not limited to, Wnt1, Wnt2, Wnt2B/13, Wnt3, Wnt3A, Wnt4, Wnt5A, Wnt5B, Wnt6, Wnt7A, Wnt8A, Wnt8B, Wnt9A, Wnt9B, Wnt10A, Wnt10B, Wnt11, and Wnt16), the Wnt receptor, Frizzled, hetero-dimerizes with its co-receptor LRP5/6 (low-density lipoprotein receptor related protein). Subsequently, activated Casein kinase 2 hyperphosphorylates Dishevelled, leading to the inhibition of GSK3b, a component of the destruction complex (including APC and Axin/Conductin) which regulates b-catenin levels in the cytosol. Phosphorylation of fb-catenin by GSK3b leads to its ubiquitinylation via b-TrCP and to its degradation by the proteasomal degradation machinery. As a result of GSK3b inhibition, the destruction complex dissembles, b-catenin is no longer phosphorylated, and the level of cytosolic and nuclear b-catenin increases. Nuclear b-catenin interacts with T-cell factor/Lymphoid enhancer factor (TCF/Lef) and displaces co-repressors. The b-catenin/TCF complex activates transcription of many different target genes. (See also Clevers, 2006, Cell 127:469-480 for a review of the Wnt/b-catenin signaling cascade). The Wnt/b-catenin signaling pathway includes, but is not limited to, the genes, and proteins encoded thereby, listed in the Tables in this application.

Wnt/b-catenin agent," "Wnt agent," or "b-catenin agent" refers to a drug or agent that modulates the canonical Wnt/b-catenin signaling pathway. A Wnt/b-catenin pathway inhibitor inhibits the canonical Wnt/b-catenin pathway signaling. Molecular targets of such agents may include b-catenin, TCF4, APC, axin, GSK3b, and any of the genes listed herein. Such agents are known in the art and include, but are not limited to: thiazolidinediones (Wang et al., 2008, J. Surg. Res. Jun. 27, 2008 e-publication ahead of print); PKF115-584 (Doghman et al., 2008, J. Clin. Endocrinol. Metab. doi: 10.1210/jc.2008-0247); bis[2-(acylamino)phenyl]disulfide (Yamakawa et al., 2008, Biol Pharm. Bull. 31:916-920);

FH535 (Handeli and Simon, 2008, Mol. Cancer Ther. 7:521-529); suldinac (Han et al., 2008, Eur. J. Pharmacol. 583:26-31); cyclooxygenase-2 inhibitor celecoxib (Tuynman et al., 2008, Cancer Res. 68:1213-1220); reverse-turn mimetic compounds (U.S. Pat. No. 7,232,822); b-catenin inhibitor compound 1 (WO2005021025); fusicoccin analog (WO2007062243); and FZD10 modulators (WO2008061020).

The term "deregulated Wnt/b-catenin pathway" is used herein to mean that the Wnt/b-catenin signaling pathway is either hyperactivated or hypoactivated. A Wnt/b-catenin signaling pathway is hyperactivated in a sample (for example, a tumor sample) if it has at least 10%, 20%, 30%, 40%, 50%, 75%, 100%, 200%, 500%, 1000% greater activity/signaling than the Wnt/b-catenin signaling pathway in a normal (regulated) sample. A Wnt/b-catenin signaling pathway is hypoactivated if it has at least 10%, 20%, 30%, 40%, 50%, 75%, 100% less activity/signaling in a sample (for example, a tumor sample) than the Wnt/b-catenin signaling pathway in a normal (regulated) sample. The normal sample with the regulated Wnt/b-catenin signaling pathway may be from adjacent normal tissue, may be other tumor samples which do not have deregulated Wnt/b-catenin signaling, or may be a pool of samples. Alternatively, comparison of samples' Wnt/b-catenin signaling pathway status may be done with identical samples which have been treated with a drug or agent vs. vehicle. The change in activation status may be due to a mutation of one or more genes in the Wnt/b-catenin signaling pathway (such as point mutations, deletion, or amplification), changes in transcriptional regulation (such as methylation, phosphorylation, or acetylation changes), or changes in protein regulation (such as translation or post-translational control mechanisms).

The term "oncogenic pathway" is used herein to mean a pathway that when hyperactivated or hypoactivated contributes to cancer initiation or progression. In one embodiment, an oncogenic pathway is one that contains an oncogene or a tumor suppressor gene.

The term "treating" in its various grammatical forms in relation to the present invention refers to preventing (i.e. chemoprevention), curing, reversing, attenuating, alleviating, minimizing, suppressing, or halting the deleterious effects of a disease state, disease progression, disease causative agent (e.g. bacteria or viruses), or other abnormal condition. For example, treatment may involve alleviating a symptom (i.e., not necessarily all the symptoms) of a disease of attenuating the progression of a disease.

"Treatment of cancer," as used herein, refers to partially or totally inhibiting, delaying, or preventing the progression of cancer including cancer metastasis; inhibiting, delaying, or preventing the recurrence of cancer including cancer metastasis; or preventing the onset or development of cancer (chemoprevention) in a mammal, for example, a human. In addition, the methods of the present invention may be practiced for the treatment of human patients with cancer. However, it is also likely that the methods would also be effective in the treatment of cancer in other mammals.

As used herein, the term "therapeutically effective amount" is intended to qualify the amount of the treatment in a therapeutic regiment necessary to treat cancer. This includes combination therapy involving the use of multiple therapeutic agents, such as a combined amount of a first and second treatment where the combined amount will achieve the desired biological response. The desired biological response is partial or total inhibition, delay, or prevention of the progression of cancer including cancer metastasis; inhibition, delay, or prevention of the recurrence of cancer including cancer metastasis; or the prevention of the onset of development of cancer (chemoprevention) in a mammal, for example, a human.

"Displaying or outputting a classification result, prediction result, or efficacy result" means that the results of a gene expression based sample classification or prediction are communicated to a user using any medium, such as for example, orally, writing, visual display, etc., computer readable medium or computer system. It will be clear to one skilled in the art that outputting the result is not limited to outputting to a user or a linked external component(s), such as a computer system or computer memory, but may alternatively or additionally be outputting to internal components, such as any computer readable medium. Computer readable media may include, but are not limited to hard drives, floppy disks, CD-ROMs, DVDs, DATs. Computer readable media does not include carrier waves or other wave forms for data transmission. It will be clear to one skilled in the art that the various sample classification methods disclosed and claimed herein, can, but need not be, computer-implemented, and that, for example, the displaying or outputting step can be done by, for example, by communicating to a person orally or in writing (e.g., in handwriting).

As noted above the present invention identifies a novel set of genes, i.e., a gene signature, the levels of expression of which in a cell sample may be used to assess the regulation status of Wnt/β-catenin signaling pathway in a cell sample or subject. This is significant because, due to limitations of cytotoxic based chemotherapies, current oncology drug development is designed to target specific cellular signaling pathways critical for tumor growth and progression. Such targeted drug development requires specific biomarkers to monitor the activity status of pathway. Compared to more traditional methods which rely on detecting the expression of one or a few indicators within the pathway constituents, multi-gene expression based methods measure pathway alteration as a function of the downstream effect of pathway regulation on multiple gene expression changes. These downstream gene expression alterations can potentially capture all changes related to any upstream alteration of a pathway component.

The Wnt/β-catenin pathway is one of the most frequently altered pathways in a number of human cancers including colorectal carcinomas, melanomas, breast cancer and hepatocellular carcinomas and therefore is of major interest to cancer researchers. Activation of the Wnt/β-catenin pathway results in a variety of downstream biological effects and this functional diversity is reflected in gene expression changes. By utilizing Wnt3a stimulation and siRNA mediated β-catenin knockdown in 293H cells followed by gene expression profiling analysis, the inventors have identified a list of 64 genes whose expression was upregulated in response to Wnt3a and whose increased expression levels were diminished by treatment with β-catenin siRNA. These 64 Wnt/β-catenin response genes were further evaluated by real-time PCR with 20 samples from a panel of 16 different cell lines either stimulated with Wnt3a and LiCl and/or inhibited with β-catenin siRNA. Sixteen genes were identified as a specific panel of indicators for Wnt/β-catenin pathway regulation using a nearest shrunken centroid classifier method. The 16 gene signature predicted the regulatory status of Wnt/β-catenin pathway in these 20 samples with an accuracy of 100% during cross validation process with support vector machine method. Therefore, the inventors have verified that they have identified a novel gene expression signature comprising a specific set of 16 genes the expression of which may be assayed (preferably by RT-PCR) to monitor the regulatory status of cellular Wnt/β-catenin pathway activity and related applications involving the modulation of this important signaling pathway.

In particular, the inventors discovered that the 13 genes listed below are up-regulated after activation of Wnt/β-catenin pathway:

CALM1
CCND1
CCND2
CHSY1
CXADR
FAM44B
HSPA12A
LEF1
MTP18
MYC
NAV2
SKP2
PRMT6

In addition, the inventors discovered that another three genes, i.e., CYP4V2, MT1A and MTSS1 are down-regulated after activation of Wnt/β-catenin pathway.

As disclosed in detail in the Experimental Section this gene expression signature has been developed from cell lines in response to specific pathway manipulation with microarray analysis. Few previous studies have verified their signature genes in terms of different cell lines and real-time PCR platform. Therefore, by developing and verifying a unique gene expression signature correlated to the activation of the Wnt/β-catenin pathway, the inventors provide a novel and improved workflow for pathway gene expression signature for the identification and verification of cells and samples wherein this pathway is affected using a real-time PCR platform.

The inventive gene expression signature, because of the manner by which it was determined, should accurately reflect the regulatory status of Wnt/β-catenin pathway activity and be useful in different assays such as screening for compounds that modulate Wnt signaling and for identifying cells wherein Wnt/β-catenin signaling is abnormal as in malignancy.

As discussed above and in detail in the Experimental Section the present inventors identified this signature gene set from an initial list of 64 Wnt/β-catenin response genes identified with microarray analysis in 293H cells treated with Wnt3a and β-catenin targeting siRNA. The Wnt/β-catenin response genes were validated with real-time PCR in a training set of 20 samples and 16 Wnt/β-catenin signature genes were identified by a support vector machine method.

The accuracy and predictive value of this 16 gene signature was later verified by cross validation in those 20 samples using real-time PCR in which 12 samples were stimulated with Wnt3a or LiCl and the rest comprised cells transfected with β-catenin siRNA. As shown infra and in the Figures referenced in the Experimental Section this 16 gene signature had an accuracy of 100% in predicting the regulatory status of Wnt/β-catenin pathway activity in these 20 samples. Therefore, the 16 gene signature and the genes in this signature may be used as biomarkers for monitoring the regulatory status of Wnt/β-catenin pathway activity.

In a preferred embodiment, the expression of these 16 genes may be determined in samples by Microarray and/or RT-PCR. In an especially preferred embodiment the expression of these 16 genes may be determined by use of SYBR Green based real-time PCR and the gene expression data is analyzed by ΔΔCt method and the pathway activity is determined with the support vector machine method.

In these methods the regulatory status of a cell sample may be determined by comparing the expression profile of one or more of these 16 genes to a control samples (cells having a normal Wnt/β-catenin pathway activity). The assayed cell sample for which regulatory status may be evaluated according to the invention may comprise any cell or cell sample wherein Wnt/β-catenin pathway activity is desirably assayed. This includes by way of example potentially malignant cells, cells which have been obtained from a patient subjected to a chemotherapy regimen which potentially affects Wnt/β-catenin pathway activity, cells wherein Wnt/β-catenin pathway deregulation status is desirably evaluated in a sample; cell samples which are to be classified as having a deregulated or regulated Wnt/β-catenin signaling pathway; and a cell sample wherein it is to be determined whether an agent modulates the Wnt/β-catenin signaling pathway in sample. In addition the present signature and biomarkers comprised therein can be used to predict the response of a subject to an agent that modulates the Wnt/β-catenin signaling pathway; assigning treatment to a subject; and evaluating the pharmacodynamic effects of therapies designed to regulate Wnt/β-catenin pathway signaling.

Because the present invention relies upon a comparison of the levels of expression by different genes in cell samples, practice of the invention typically requires control and treatment samples to determine the relative regulatory status of a target cell sample vs control. The target cell sample e.g., may be one manipulated by different means that may affect Wnt/β-catenin pathway regulation status such as contacting with siRNA(s) or drug treatment and the control will be the appropriate control for that manipulation. For example the control cells will be treated identically (culture conditions, time, excipients, vehicles) except for the absence of the particular tested manipulation agent such as exposure to a chemotherapeutic agent.

In the present invention, target polynucleotide molecules are typically extracted from a sample taken from an individual afflicted with cancer or tumor cell lines, and corresponding normal/control tissues or cell lines, respectively. Samples may also be taken from primary cell lines or ex vivo cultures of cells taken from an animal or patient. The sample may be collected in any clinically acceptable manner, but must be collected such that biomarker-derived polynucleotides (i.e., RNA) are preserved. mRNA or nucleic acids derived therefrom (i.e., cDNA or amplified DNA) are preferably labeled distinguishably from standard or control polynucleotide molecules, and both are simultaneously or independently hybridized to a microarray comprising some or all of the biomarkers or biomarker sets or subsets described above. Alternatively, mRNA or nucleic acids derived therefrom may be labeled with the same label as the standard or control polynucleotide molecules, wherein the intensity of hybridization of each at a particular probe is compared. A sample may comprise any clinically relevant tissue sample, such as a tumor biopsy, fine needle aspirate, or hair follicle, or a sample of bodily fluid, such as blood, plasma, serum, lymph, ascitic fluid, cystic fluid, urine. The sample may be taken from a human, or, in a veterinary context, from non-human animals such as ruminants, horses, swine or sheep, or from domestic companion animals such as felines and canines. Additionally, the samples may be from frozen or archived formalin-fixed, paraffin-embedded (FFPE) tissue samples.

Methods for preparing total and poly(A)+ RNA are well known and are described generally in Sambrook et al., MOLECULAR CLONING--A LABORATORY MANUAL (2ND ED.), Vols. 1-3, Cold Spring Harbor Laboratory, Cold Spring Harbor, N.Y. (1989)) and Ausubel et al., CURRENT PROTOCOLS IN MOLECULAR BIOLOGY, vol. 2, Current Protocols Publishing, New York (1994)).

RNA may be isolated from eukaryotic cells by procedures that involve lysis of the cells and denaturation of the proteins contained therein. Cells of interest include wild-type cells (i.e., non-cancerous), drug-exposed wild-type cells, tumor- or tumor-derived cells, modified cells, normal or tumor cell line cells, and drug-exposed modified cells.

Additional steps may be employed to remove DNA. Cell lysis may be accomplished with a nonionic detergent, followed by microcentrifugation to remove the nuclei and hence the bulk of the cellular DNA. In one embodiment, RNA is extracted from cells of the various types of interest using guanidinium thiocyanate lysis followed by CsCl centrifugation to separate the RNA from DNA (Chirgwin et al., Biochemistry 18:5294-5299 (1979)). Poly(A)+ RNA is selected by selection with oligo-dT cellulose (see Sambrook et al, MOLECULAR CLONING--A LABORATORY MANUAL (2ND ED.), Vols. 1-3, Cold Spring Harbor Laboratory, Cold Spring Harbor, N.Y. (1989). Alternatively, separation of RNA from DNA can be accomplished by organic extraction, for example, with hot phenol or phenol/chloroform/isoamyl alcohol. If desired, RNase inhibitors may be added to the lysis buffer. Likewise, for certain cell types, it may be desirable to add a protein denaturation/digestion step to the protocol.

For many applications, it is desirable to preferentially enrich mRNA with respect to other cellular RNAs, such as transfer RNA (tRNA) and ribosomal RNA (rRNA). Most mRNAs contain a poly(A) tail at their 3' end. This allows them to be enriched by affinity chromatography, for example, using oligo(dT) or poly(U) coupled to a solid support, such as cellulose or Sephadex. (see Ausubel et al., CURRENT PROTOCOLS IN MOLECULAR BIOLOGY, vol. 2, Current Protocols Publishing, New York (1994). Once bound, poly(A)+ mRNA is eluted from the affinity column using 2 mM EDTA/0.1% SDS.

The sample of RNA can comprise a plurality of different mRNA molecules, each different mRNA molecule having a different nucleotide sequence. In a specific embodiment, the mRNA molecules in the RNA sample comprise at least 100 different nucleotide sequences. More preferably, the mRNA molecules of the RNA sample comprise mRNA molecules corresponding to each of the biomarker genes. In another specific embodiment, the RNA sample is a mammalian RNA sample.

In a specific embodiment, total RNA or mRNA from cells is used in the methods of the invention. The source of the RNA can be cells of a plant or animal, human, mammal, primate, non-human animal, dog, cat, mouse, rat, bird, yeast, eukaryote, prokaryote, etc. In specific embodiments, the method of the invention is used with a sample containing total mRNA or total RNA from 1 X $10^6$ cells or less. In another embodiment, proteins can be isolated from the foregoing sources, by methods known in the art, for use in expression analysis at the protein level.

Probes to the homologs of the biomarker sequences disclosed herein can be employed preferably wherein non-human nucleic acid is being assayed.

In a preferred embodiment of the invention the Wnt/β-catenin pathway regulation status will be determined based on the expression levels of all of the 16 genes in the Wnt/β-catenin pathway signature versus the control sample. However, it is envisioned that Wnt/β-catenin pathway regulation status may be in addition be determined by assaying the expression of a subset of these 16 genes or biomarkers, i.e., any combination of at least 2 of these genes, at least 3 of these genes, at least 4 of these genes, at least 6 of these genes, at least 7 of these genes,...or all of these 16 genes. In addition, it is within the scope of the invention to further assay the expression if additional genes which affect or correlate to Wnt/β-catenin pathway regulation status.

Therefore, one aspect of the invention provides a set of 16 biomarkers whose expression is correlated with Wnt/b-catenin signaling pathway deregulation. These biomarkers identified as useful for classifying subjects according to regulation status of the Wnt/b-catenin signaling pathway, predicting response of a subject to a compound that modulates the Wnt/b-catenin signaling pathway, measuring pharmacodynamic effect on the Wnt/b-catenin signaling pathway of a therapeutic agent.

Another aspect of the invention provides a method of using these biomarkers or a microarray containing to distinguish tumor types in diagnosis or to predict response to therapeutic agents.

Yet other aspects of the invention provide methods of using these biomarkers or a microarray containing as pharmacodynamic biomarkers, i.e. monitoring pathway inhibition in patient tumors or peripheral tissues post-treatment; as response prediction biomarkers, i.e. prospectively identifying patients harboring tumors that have high levels of a particular pathway activity before treating the patients with inhibitors targeting the pathway; and as early efficacy biomarkers, i.e. an early readout of efficacy. In one embodiment of the invention, the 16 biomarker set may be split into two opposing "arms"--the "up" arm, which are the 13 genes that are upregulated, and the "down" arm which are the 3 genes that are downregulated, as signaling through the Wnt/b-catenin pathway increases.

In yet another aspect the invention provides a set of 16 biomarkers or a microarray containing that can classify subjects by Wnt/b-catenin signaling pathway regulation status, i.e. distinguish between subjects having regulated and deregulated Wnt/b-catenin signaling pathways. These biomarkers are listed herein. The invention also provides subsets drawn from the set of 16 genes, that can distinguish between subjects having deregulated and regulated Wnt/b-catenin signaling pathways. Alternatively, a subset of the biomarkers, can distinguish between subjects having deregulated and regulated Wnt/b-catenin signaling pathways. The invention also provides a method of using the above biomarkers to distinguish between subjects having deregulated or regulated Wnt/b-catenin signaling pathway.

In another embodiment, the invention provides a set of 16 biomarkers or a microarray containing them that can be used to predict response of a subject to a Wnt/b-catenin signaling pathway agent. In a more specific embodiment, the invention provides a subset of the disclosed set of 16 biomarkers that can be used to predict the response of a subject to an agent that modulates the Wnt/b-catenin signaling pathway. In another embodiment, the invention provides a set of 16 biomarkers that can be used to select a Wnt/b-catenin pathway agent for treatment of a subject with cancer. In a more specific embodiment, the invention provides a subset of the set of 16 biomarkers that can be used to select a Wnt/b-catenin pathway agent for treatment of a subject with cancer. Alternatively, a subset of these biomarkers can be used to predict response of a subject to a Wnt/b-catenin signaling pathway agent or to select a Wnt/b-catenin signaling pathway agent for treatment of a subject with cancer.

In another embodiment, the invention provides a set of 16 genetic biomarkers or a microarray containing them that can be used to determine whether an agent has a pharmacodynamic effect on the Wnt/b-catenin signaling pathway in a subject. The biomarkers provided may be used to monitor inhibition of the Wnt/b-catenin signaling pathway at various time points following treatment of a subject with said agent. In a more specific embodiment, the invention provides a subset of the disclosed 16 biomarkers that can be used to monitor pharmacodynamic activity of an agent on the Wnt/b-catenin signaling pathway.

The subject biomarkers may be used alone or in combination with biomarkers outside the set. For example, biomarkers that distinguish Wnt/b-catenin pathway regulation status may be used in combination with biomarkers that distinguish growth factor signaling pathway regulation status. Any of the biomarker sets provided herein also may also be used in combination with other biomarkers for cancer, or for any other clinical or physiological condition.

As noted in a preferred embodiment the expression value of all 16 genes is assayed by realtime PCR to determine the Wnt pathway regulatory status. To ensure accuracy the expression value of these 16 genes plus control genes (i.e., 1 or more house keeping genes, e.g., 5 house keeping genes) is measured on both the control cell sample and the treatment sample and the ΔΔCt is calculated. This ΔΔCt value of those 16 genes is then compared to ΔΔCt value of 16 genes in our training data pool that contains 20 samples (8 negatively regulated and 12 positively regulated in terms of pathway activity). In the exemplified embodiments the support vector machine method is used to determine the regulatory status of the particular target cell sample.

The present invention provides kits and kit components for effecting the described gene expression assay methods. In a preferred exemplary embodiment the kit will comprise a Wnt signaling PCR array product comprising at least 5 sequences corresponding to these 16 genes, preferably all 16 of these genes or the majority thereof. With respect thereto the Applicant company Qiagen has an existing Wnt PCR array which contains 4 of the genes comprised within the 16 gene signature reported herein. In a preferred embodiment this existing Wnt PCR array will be modified by the incorporation of the remaining 12 signature genes. Therefore this product will be similar to the existing Wnt PCR array in terms of physical component except that it will comprise 12 new genes. The invention further may preferably include a web based system for analysis of the gene expression data.

After running the preferred PCR array, a user will determine the regulatory status of a target sample and a control sample. In a preferred embodiment a user will effect comparison and analysis by the use of an available Qiagen web based analysis tool or equivalent. This web-based analysis tool and data analysis is currently being effected with the existing Qiagen Wnt PCR array. In the context of the present invention this tool will in addition provide users with a number (index, probability or analogous parameter) which will be used to assess the relative regulatory status of a particular treatment sample compared to an appropriate control sample. Therefore, the kit components and the protocol are similar to the currently available Wnt PCR array with the exception of an extra analysis (pathway regulatory status based on 16 genes expression level).

## APPLICATIONS OF PRESENT INVENTION

### Diagnostic/Sample Classification Methods

The invention provides for methods of using the biomarker sets to analyze a sample from an individual or subject so as to determine or classify the subject's sample at a molecular level, whether a sample has a deregulated or regulated Wnt/b-catenin pathway. The sample may or may not be derived from a tumor. The individual need not actually be afflicted with cancer. Essentially, the expression of specific biomarker genes in the individual, or a sample taken therefrom, is compared to a standard or control. For example, assume two cancer-related conditions, X and Y. One can compare the level of expression of Wnt/b-catenin pathway biomarkers for condition X in an individual to the level of the biomarker-derived polynucleotides in a control, wherein the level represents the level of expression exhibited by samples having condition X. In this instance, if the expression of the markers in the individual's sample is substantially (i.e., statistically) different from that of the control, then the individual does not have condition X. Where, as here, the choice is bimodal (i.e. a sample is either X or Y), the individual can additionally be said to have condition Y. Of course, the comparison to a control representing condition Y can also be performed. Preferably, both are performed simultaneously, such that each control acts as both a positive and a negative control. The distinguishing result may thus either be a demonstrable difference from the expression levels (i.e. the amount of marker-derived RNA, or polynucleotides derived therefrom) represented by the control, or no significant difference. Thus, in one embodiment, the method of determining a particular tumor-related status of an individual comprises the steps of (1) hybridizing labeled target polynucleotides from an individual to a microarray containing the above biomarker set or a subset of the biomarkers; (2) hybridizing standard or control polynucleotide molecules to the microarray, wherein the standard or control molecules are differentially labeled from the target molecules; and (3) determining the difference in transcript levels, or lack thereof, between the target and standard or control, wherein the difference, or lack thereof, determines the individual's tumor-related status. In a more specific embodiment, the standard or control molecules comprise biomarker-derived polynucleotides from a pool of samples from normal individuals, a pool of samples from normal adjacent tissue, or a pool of tumor samples from individuals with cancer. In a preferred embodiment, the standard or control is artificially-generated pool of biomarker-derived polynucleotides, which pool is designed to mimic the level of biomarker expression exhibited by clinical samples of normal or cancer tumor tissue having a particular clinical indication (i.e. cancerous or non-cancerous; Wnt/b-catenin pathway regulated or deregulated). In another specific embodiment, the control molecules comprise a pool derived from normal or cancer cell lines.

The present invention provides a set of biomarkers or a microarray containing useful for distinguishing deregulated from regulated Wnt/b-catenin pathway tumor types. Thus, in one embodiment of the above method, the level of polynucleotides (i.e., mRNA or polynucleotides derived therefrom) in a sample from an individual, expressed from the 16 biomarkers provided herein are compared to the level of expression of the same biomarkers from a control. If the purpose is to identify whether a compound affects Wnt signaling the control may comprise a sample treated by the same methods except in the absence of the compound.

The comparison alternatively may be to both deregulated and regulated Wnt/b-catenin signaling pathway tumor samples, and the comparison may be to polynucleotide pools from a number of deregulated and regulated Wnt/b-catenin signaling pathway tumor samples, respectively. Where the individual's biomarker expression most closely resembles or correlates with the deregulated control, and does not resemble or correlate with the regulated control, the individual is classified as having a deregulated Wnt/b-catenin signaling pathway. Where the pool is not pure deregulated or regulated Wnt/b-catenin signaling pathway type tumors samples, for example, a sporadic pool is used, a set of experiments using individuals with known Wnt/b-catenin signaling pathway status may be hybridized against the pool in order to define the expression templates for the deregulated and regulated group. Each individual with unknown Wnt/b-catenin signaling pathway status is hybridized against the same pool and the expression profile is compared to the template(s) to determine the individual's Wnt/b-catenin signaling pathway status. As noted in the preferred methods the expression of the biomarkers is effected by use of RT-PCR.

There is described a method comprising: (i) calculating a measure of similarity between a first expression profile and a deregulated Wnt/b-catenin signaling pathway template, or calculating a first measure of similarity between said first expression profile and said deregulated Wnt/b-catenin signaling pathway template and a second measure of similarity between said first expression profile and a regulated Wnt/b-catenin signaling pathway template, said first expression profile comprising the expression levels of a first plurality of genes in the cell sample, said deregulated Wnt/b-catenin signaling pathway template comprising expression levels of said first plurality of genes that are average expression levels of the respective genes in a plurality of cell samples having at least one or more components of said Wnt/b-catenin signaling pathway with abnormal activity, and said regulated Wnt/b-catenin signaling pathway template comprising expression levels of said first plurality of genes that are average expression levels of the respective genes in a plurality of cell samples not having at least one or more components of said Wnt/b-catenin signaling pathway with abnormal activity, said first plurality of genes consisting of at least 5 of the genes for which biomarkers are listed herein ;

(ii) classifying said cell sample as having said deregulated Wnt/b-catenin signaling pathway if said first expression profile has a high similarity to said deregulated Wnt/b-catenin signaling pathway template or has a higher similarity to said deregulated Wnt/b-catenin signaling pathway template than to said regulated Wnt/b-catenin signaling pathway template, or classifying said cell sample as having said regulated Wnt/b-catenin signaling pathway if said first expression profile has a low similarity to said deregulated Wnt/b-catenin signaling pathway template or has a higher similarity to said regulated Wnt/b-catenin signaling pathway template than to said deregulated Wnt/b-catenin signaling pathway template; wherein said first expression profile has a high similarity to said deregulated Wnt/b-catenin signaling pathway template if the similarity to said deregulated Wnt/b-catenin signaling pathway template is above a predetermined threshold, or has a low similarity to said deregulated Wnt/b-catenin signaling pathway template if the similarity to said deregulated Wnt/b-catenin signaling pathway template is below said predetermined threshold; and

(iii) displaying; or outputting to a user interface device, a computer readable storage medium, or a local or remote computer system; the classification produced by said classifying step (ii).

In another specific embodiment, the set of biomarkers may be used to classify a sample from a subject by the Wnt/b-catenin signaling pathway regulation status. The sample may or may not be derived from a tumor. Thus, in one embodiment of the above method, the level of polynucleotides (i.e., mRNA or polynucleotides derived therefrom) in a sample from an individual, expressed from the biomarkers provided herein are compared to the level of expression of the same biomarkers from a control, wherein the control comprises biomarker-related polynucleotides derived from deregulated Wnt/b-catenin signaling pathway samples, regulated Wnt/b-catenin signaling pathway samples, or both. The comparison may be to both deregulated and regulated Wnt/b-catenin signaling pathway samples, and the comparison may be to polynucleotide pools from a number of deregulated and regulated Wnt/b-catenin signaling pathway samples, respectively. The comparison may also be made to a mixed pool of samples with deregulated and regulated Wnt/b-catenin signaling pathway or unknown samples.

For the above embodiments, the fullest of biomarkers may be used (i.e., the complete set of 16 biomarkers). In other embodiments, subsets of at least 5 of the 16 biomarkers may be used or subsets of the "up" and "down" arms of the biomarkers may be used.

The expression profile may be a differential expression profile comprising differential measurements of said plurality of genes in a sample derived from a subject versus measurements of said plurality of genes in a control sample. The differential measurements can be xdev, log(ratio), error-weighted log(ratio), or a mean subtracted log(intensity) (see, e.g., PCT publication WO00/39339, published on Jul. 6, 2000; PCT publication WO2004/065545, published Aug. 5, 2004). The similarity between the biomarker expression profile of a sample or an individual and that of a control can be assessed a number of ways using any method known in the art. For example, Dai et al. describe a number of different ways of calculating gene expression templates and corresponding biomarker genets useful in classifying breast cancer patients (U.S. Pat. No. 7,171,311; WO2002/103320; WO2005/086891; WO2006015312; WO2006/084272). Similarly, Linsley et al. (US2003/0104426) and Radish et al. (US20070154931) disclose gene biomarker genesets and methods of calculating gene expression templates useful in classifying chronic myelogenous leukemia patients. In the simplest case, the profiles can be compared visually in a printout of expression difference data. Alternatively, the similarity can be calculated mathematically.

The similarity may be represented by a correlation coefficient between the patient or sample profile and the template. In one embodiment, a correlation coefficient above a correlation threshold indicates high similarity, whereas a correlation coefficient below the threshold indicates low similarity. In some embodiments, the correlation threshold is set as 0.3, 0.4, 0.5, or 0.6. In another embodiment, similarity between a sample or patient profile and a template is represented by a distance between the sample profile and the template. In one embodiment, a distance below a given value indicates a high similarity, whereas a distance equal to or greater than the given value indicates low similarity.

Thus, the above method of determining a particular tumor-related status of an individual may comprise the steps of (1) hybridizing labeled target polynucleotides from an individual to a microarray containing one of the above marker sets; (2) hybridizing standard or control polynucleotides molecules to the microarray, wherein the standard or control molecules are differentially labeled from the target molecules; and (3) determining the ratio (or difference) of transcript levels between two channels (individual and control), or simply the transcript levels of the individual; and (4) comparing the results from (3) to the predefined templates, wherein said determining is accomplished by any means known in the art, and wherein the difference, or lack thereof, determines the individual's tumor-related status. The method can use the complete set of 16 biomarkers. However, subsets of the 16 biomarkers, or the "up" (13 genes which are upregulated) or "down" (the 3 genes which are down regulated may also be used.

The signature score of a sample may be defined as the average expression level (such as mean log(ratio)) of the complete set of 16 biomarkers or a subset of these biomarkers, regardless of "arm." If the signature score for a sample is above a pre-determined threshold, then the sample is considered to have deregulation of the Wnt/b-catenin signaling pathway. The pre-determined threshold may be 0, or may be the mean, median, or a percentile of signature scores of a collection of samples or a pooled sample used as a standard or control.

The use of the biomarkers is not limited to distinguishing or classifying particular tumor types, such as colon cancer, as having deregulated or regulated Wnt/b-catenin signaling pathway. The biomarkers may be used to classify cell samples from any cancer type, where aberrant Wnt/b-catenin signaling may be implicated. Aberrant Wnt/b-catenin pathway signaling has been discovered in a wide variety of cancers, including melanoma, hepatocellular carcinoma, osteosarcoma, and many tumors (uterine, ovarian, lung, gastric, and renal) (Luu et al., 2004, Curr. Cancer Drug Targets 4:653-671; Reya and Clevers, 2005, Nature 434:843-850; Moon et al., 2004, Nat. Rev. Genet. 5:691-701).

The use of the biomarkers is also not restricted to distinguishing or classifying cell samples as having deregulated or regulated Wnt/b-catenin signaling pathway for cancer-related conditions, and may be applied in a variety of phenotypes or conditions, in which aberrant Wnt/b-catenin signaling plays a role, or the level of Wnt/b-catenin signaling activity is sought. For example, the biomarkers may be useful for classifying cell samples for bone and joint disorders, such as, but not limited to, osteoporosis, rheumatoid arthritis, sclerosteosis, van Buchem syndrome, osteoporosis pseudoglioma syndrome. The Wnt/b-catenin signaling pathway has previously been implicated in bone and joint formation and regeneration (Boyden et al, 2002, N. Engl. J. Med. 346:1513-1521; Gong et al., 2001, Cell 107:513-523; Little et al., 2002, Am. J. Hum. Genet. 70:11-19; Diana et al., 2007, Nat. Med. 13:156-163; Baron and Rawadi, 2007, Endocrin. 148:2635-2643; Kim et al., 2007, J. Bone Mineral Res-- 22:1913-1923). Wnt/b-catenin signaling has also been implicated in the development of diabetes (Jin, 2008, Diabetologia, e-publication ahead of print, Aug. 12, 2008); retinal development and disease (Lad et al., 2008, Stem Cells Dev. E-publication ahead of print Aug. 8, 2008); neurodegernative disorders (Caraci et al, 2008, Neurochem. Res., E-publication ahead of print, Apr. 22, 2008).

Methods of Predicting Response to Treatment and Assigning Treatment

The invention provides a set of biomarkers useful for distinguishing samples from those patients who are predicted to respond to treatment with an agent that modulates the Wnt/b-catenin signaling pathway from patients who are not predicted to respond to treatment an agent that modulates the Wnt/b-catenin signaling pathway. Thus, the invention further provides a method for using these biomarkers for determining whether an individual with cancer is a predicted responder to treatment with an agent that modulates the Wnt/b-catenin signaling pathway. In one embodiment, the invention provides for a method of predicting response of a cancer patient to an agent that modulates the Wnt/b-catenin signaling pathway comprising (1) comparing the level of expression of the 16 biomarkers in a sample taken from the individual to the level of expression of the same biomarkers in a standard or control, where the standard or control levels represent those found in a sample having a deregulated Wnt/b-catenin signaling; and (2) determining whether the level of the biomarker-related polynucleotides in the sample from the individual is significantly different than that of the control, wherein if no substantial difference is found, the patient is predicted to respond to treatment with an agent that modulates the Wnt/b-catenin signaling pathway, and if a substantial difference is found, the patient is predicted not to respond to treatment with an agent that modulates the Wnt/b-catenin signaling pathway. Persons of skill in the art will readily see that the standard or control levels may be from a sample having a regulated Wnt/b-catenin signaling pathway. In a more specific embodiment, both controls are run. In case the pool is not pure "Wnt/b-catenin regulated" or "Wnt/b-catenin deregulated," a set of experiments of individuals with known responder status may be hybridized against the pool to define the expression templates for the predicted responder and predicted non-responder group. Each individual with unknown outcome is hybridized against the same pool and the resulting expression profile is compared to the templates to predict its outcome.

Wnt/b-catenin signaling pathway deregulation status of a tumor may indicate a subject that is responsive to treatment with an agent that modulates the Wnt/b-catenin signaling pathway. Therefore, the invention provides for a method of determining or assigning a course of treatment of a cancer patient, comprising determining whether the level of expression of the 16 biomarkers, or a subset of at least 5 thereof, correlates with the level of these biomarkers in a sample representing deregulated Wnt/b-catenin signaling pathway status or regulated Wnt/b-catenin signaling pathway status; and determining or assigning a course of treatment, wherein if the expression correlates with the deregulated Wnt/b-catenin signaling pathway status pattern, the tumor is treated with an agent that modulates the Wnt/b-catenin signaling pathway.

As with the diagnostic biomarkers, the method can preferably use the complete set of 16 biomarkers. However, subsets of at least 5 of the 16 biomarkers may also be used.

Classification of a sample as "predicted responder" or "predicted non-responder" is accomplished substantially as for the diagnostic biomarkers described above, wherein a template is generated to which the biomarker expression levels in the sample are compared.

In another embodiment, the above method for measuring the effect of an agent on the Wnt/β-catenin signaling pathway is preferably determined after real-time PCR measuring expression levels of 16 biomarker genes using SYBR Green, and a ΔΔCT method employed to analysis the data. The average CT values of house keeping genes in each sample is calculated as house keeping gene CT value for that sample. ΔCT was calculated by subtracting house keeping CT value from individual assay CT value of same sample. ΔΔCT value was derived by further subtracting ΔCT value of control samples of each assay from its corresponding ΔCT value of treatment sample. This ΔΔCt value of those 16 genes is then compared to ΔΔCt value of 16 genes in our training data pool that contains 20 samples (8 negatively regulated and 12 positively regulated in terms of pathway activity). A support vector machine method is preferably used to analyze the ΔΔCT values of the samples and the expression thereof used to assess the regulatory status of the Wnt/β-catenin pathway activity in the sample.

The use of the biomarkers is not restricted to predicting response to agents that modulate Wnt/b-catenin signaling pathway for cancer-related conditions, and may be applied in a variety of phenotypes or conditions, clinical or experimental, in which gene expression plays a role. Where a set of biomarkers has been identified that corresponds to two or more phenotypes, the biomarker sets can be used to distinguish these phenotypes. For example, the phenotypes may be the diagnosis and/or prognosis of clinical states or phenotypes associated with cancers and other disease conditions, or other physiological conditions, prediction of response to agents that modulate pathways other than the Wnt/b-catenin signaling pathway, wherein the expression level data is derived from a set of genes correlated with the particular physiological or disease condition.

The use of the biomarkers is not limited to predicting response to agents that modulate Wnt/b-catenin signaling pathway for a particular cancer type, such as colon cancer. The biomarkers may be used to predict response to agents in any cancer type, where aberrant Wnt/b-catenin signaling may be implicated. Aberrant Wnt/b-catenin pathway signaling has been discovered in a wide variety of cancers, including melanoma, hepatocellular carcinoma, osteosarcoma, and many tumors (uterine, ovarian, lung, gastric, and renal) (Luu et al., 2004, Curr. Cancer Drug Targets 4:653-671; Reya and Clevers, 2005, Nature 434:843-850; Moon et al., 2004, Nat. Rev. Genet. 5:691-701).

The use of the biomarkers is also not restricted to predicting response to agents that modulate Wnt/b-catenin signaling pathway for cancer-related conditions, and may be applied in a variety of phenotypes or conditions, in which aberrant Wnt/b-catenin signaling plays a role, or the level of Wnt/b-catenin signaling activity is sought. For example, the biomarkers may be useful for predicting response to agents that modulate the Wnt/b-catenin signaling pathway in subjects with bone or joint disorders, such as, but not limited to, osteoporosis, rheumatoid arthritis, sclerosteosis, van Buchem syndrome, osteoporosis pseudoglioma syndrome. The Wnt/b-catenin signaling pathway has previously been implicated in bone and joint formation and regeneration (Boyden et al, 2002, N. Engl. J. Med. 346:1513-1521; Gong et al., 2001, Cell 107:513-523; Little et al., 2002, Am. J. Hum. Genet. 70:11-19; Diarra et al., 2007, Nat. Med. 13:156-163; Baron and Rawadi, 2007, Endocrin. 148:2635-2643; Kim et al., 2007, J. Bone Mineral Res. 22:1913-1923). Wnt/b-catenin signaling has also been implicated in the development of diabetes (Jin, 2008, Diabetologia, e-publication ahead of print, Aug. 12, 2008); retinal development and disease (Lad et al., 2008, Stem Cells Dev. E-publication ahead of print Aug. 8, 2008); neurodegenerative disorders (Caraci et al, 2008, Neurochem. Res., Apr. 22, 2008).

Method of Determining Whether an Agent Modulates the Wnt/b-catenin Signaling Pathway

The invention provides a set of biomarkers useful for and methods of using the biomarkers for identifying or evaluating an agent that is predicted to modify or modulate the Wnt/b-catenin signaling pathway in a subject. "Wnt/b-catenin signaling pathway" is initiated by binding of the Writ ligands (including, but not limited to Wnt1, Wnt2, Wnt2B/13, Wnt3, Wnt3A, Wnt4, Wnt5A, Wnt5B, Wnt6, Wnt7A, Wnt8A, Wnt8B, Wnt9A, Wnt9B, Wnt10A, Wnt10B, Wnt11, and Wnt16) to the co-receptor Frizzled/LRP5/6 complex. Frizzled interacts with Dishevelled, a cytoplasmic protein that functions upstream of b-catenin and GSK3b, leading to the inactivation of the destruction complex. Upon destruction complex inactivation, stabilized b-catenin is transported to the nucleus where it regulates the activity of TCF/LEF family transcription factors. b-catenin induces expression of a large number of genes, including genes involved in proliferation (c-Myc and Cyclin D1) and feedback regulation of the pathway (Axin-2 and LEF1). In this application, unless otherwise specified, it will be understood that "Wnt/b-catenin signaling pathway" refers to signaling through canonical Wnt/b-catenin signaling pathway, which controls the intracellular level of the proto-oncoprotein b-catenin.

Agents affecting the Wnt/b-catenin signaling pathway include small molecule compounds; proteins or peptides (including antibodies); siRNA, shRNA, or microRNA molecules; or any other agents that modulate one or more genes or proteins that function within the Wnt/b-catenin signaling pathway or other signaling pathways that interact with the Wnt/b-catenin signaling pathway, such as the Notch pathway.

"Wnt/b-catenin pathway agent" refers to an agent which modulates the canonical Wnt/b-catenin pathway signaling. A Wnt/b-catenin pathway inhibitor inhibits the canonical Wnt/b-catenin pathway signaling. Molecular targets of such agents may include b-catenin, TCF4, APC, axin, and GSK3b. Such agents are known in the art and include, but are not limited to: thiazolidinediones (Wang et al., 2008, J. Surg. Res. Jun. 27, 2008 e-publication ahead of print); PKF115-584 (Doghman et al., 2008, J. Clin. Endocrinol. Metab. 10.1210/jc.2008-0247); bis[2-(acylamino)phenyl]disulfide (Yamakawa et al., 2008, Biol Pharm. Bull. 31:916-920); FH535 (Handeli and Simon, 2008, Mol. Cancer Ther. 7:521-529); suldinac (Han et al., 2008, Fur. J. Pharmacol. 583:26-31); cyclooxygenase-2 inhibitor celecoxib (Tuynman et al., 2008, Cancer Res. 68:1213-1220); reverse-turn mimetic compounds (U.S. Pat. No. 7,232,822); b-catenin inhibitor compound 1 (WO2005021025); fusicoccin analog (WO2007062243); and FZD10 modulators (WO2008061020).

In one embodiment, the method for measuring the effect or determining whether an agent modulates the Wnt/b-catenin signaling pathway comprises: (1) comparing the level of expression of the 16 biomarkers in a sample treated with an agent to the level of expression of the same biomarkers in a standard or control, wherein the standard or control levels represent those found in a vehicle-treated sample; and (2) determining whether the level of the biomarker-related polynucleotides in the treated sample is significantly different than that of the vehicle-treated control, wherein if no substantial difference is found, the agent is predicted not to have an modulate the Wnt/b-catenin signaling pathway, and if a substantial difference is found, the agent is predicted to modulate the Wnt/b-catenin signaling pathway.

In another embodiment, the above method for measuring the effect of an agent on the Wnt/β-catenin signaling pathway is preferably determined after real-time PCR measuring expression levels of 16 biomarker genes (e.g., using SYBR green), and a $\Delta\Delta$CT method employed to analysis the data. The average CT values of house keeping genes in each sample is calculated as house keeping gene CT value for that sample. $\Delta$CT was calculated by subtracting house keeping CT value from individual assay CT value of same sample. $\Delta\Delta$CT value was derived by further subtracting $\Delta$CT value of control samples of each assay from its corresponding $\Delta$CT value of treatment sample. This $\Delta\Delta$Ct value of those 16 genes is then compared to $\Delta\Delta$Ct value of 16 genes in our training data pool that contains 20 samples (8 negatively regulated and 12 positively regulated in terms of pathway activity). A support vector machine method is preferably used to analyze the $\Delta\Delta$CT values of the samples and the expression thereof used to assess the regulatory status of the Wnt/β-catenin pathway activity in the sample.

The use of the biomarkers is not restricted to determining whether an agent modulates Wnt/b-catenin signaling pathway for cancer-related conditions, and may be applied in a variety of phenotypes or conditions, clinical or experimental, in which gene expression plays a role. Where a set of biomarkers has been identified that corresponds to two or more phenotypes, the biomarker sets can be used to distinguish these phenotypes. For example, the phenotypes may be the diagnosis and/or prognosis of clinical states or phenotypes associated with cancers and other disease conditions, or other physiological conditions, prediction of response to agents that modulate pathways other than the Wnt/b-catenin signaling pathway, wherein the expression level data is derived from a set of genes correlated with the particular physiological or disease condition.

The use of the biomarkers is not limited to determining whether an agent modulates the Wnt/b-catenin signaling pathway for a particular cancer type, such as colon cancer. The biomarkers may be used to determine whether an agent modulates the Wnt/b-catenin for any cancer type, where aberrant Wnt/b-catenin signaling may be implicated. Aberrant Wnt/b-catenin pathway signaling has been discovered in a wide variety of cancers, including melanoma, hepatocellular carcinoma, osteosarcoma, and many tumors (uterine, ovarian, lung, gastric, and renal) (Luu et al., 2004, Curr. Cancer Drug Targets 4:653-671; Reya and Clevers, 2005, Nature 434:843-850; Moon et al., 2004, Nat. Rev. Genet. 5:691-701).

The use of the biomarkers is also not restricted determining whether an agent modulates the Wnt/b-catenin signaling pathway for cancer-related conditions, and may be applied for agents for a variety of phenotypes or conditions, in which aberrant Wnt/b-catenin signaling plays a role, or the level of Wnt/b-catenin signaling activity is sought. For example, the biomarkers may be useful for determining whether an agent modulates the Wnt/b-catenin signaling pathway, for treatment of bone or joint disorders, such as, but not limited to, osteoporosis, rheumatoid arthritis, sclerosteosis, van Buchem syndrome, osteoporosis pseudoglioma syndrome. The Wnt/b-catenin signaling pathway has previously been implicated in bone and joint formation and regeneration (Boyden et al, 2002, N. Engl. J. Med. 346:1513-1521; Gong et al., 2001, Cell 107:513-523; Little et al., 2002, Am. J. Hum. Genet. 70:11-19; Diarra et al., 2007, Nat. Med. 13:156-163; Baron and Rawadi, 2007, Endocrin. 148:2635-2643; Kim et al., 2007, J. Bone Mineral Res. 22:1913-1923). Wnt/b-catenin signaling has also been implicated in the development of diabetes (Jin, 2008, Diabetologia, e-publication ahead of print, Aug. 12, 2008); retinal development and disease (Lad et al., 2008, Stem Cells Dev. Aug. 8, 2008); neurodegernative disorders (Caraci et al, 2008, Neurochem. Res., Apr. 22, 2008).

Method of Measuring Pharmacodynamic Effect of an Agent

The invention provides a set of biomarkers useful for measuring the pharmacodynamic effect of an agent on the Wnt/b-catenin signaling pathway. The biomarkers provided may be used to monitor modulation of the Wnt/b-catenin signaling pathway at various time points following treatment with said agent in a patient or sample. Thus, the invention further provides a method for using these biomarkers as an early evaluation for efficacy of an agent which modulates the Wnt/b-catenin signaling pathway. In one embodiment, the invention provides for a method of measuring pharmacodynamic effect of an agent that modulates the Wnt/b-catenin signaling pathway in patient or sample comprising: (1) comparing the level of expression of the 16 biomarkers in a sample treated with an agent to the level of expression of the same biomarkers in a standard or control, wherein the standard or control levels represent those found in a vehicle-treated sample; and (2) determining whether the level of the biomarker-related polynucleotides in the treated sample is significantly different than that of the vehicle-treated control, wherein if no substantial difference is found, the agent is predicted not to have an pharmacodynamic effect on the Wnt/b-catenin signaling pathway, and if a substantial difference is found, the agent is predicted to have an pharmacodynamic effect on the Wnt/b-catenin signaling pathway. In another specific embodiment, the invention provides a subset of at least 5, or 10 biomarkers, drawn from the set of 16 that can be used to monitor pharmacodynamic activity of an agent on the Wnt/b-catenin signaling pathway.

In another embodiment, the above method for measuring the effect of an agent on the Wnt/β-catenin signaling pathway is preferably determined after real-time PCR measuring expression levels of 16 biomarker genes (e.g., using SYBR green detection), and a ΔΔCT method employed to analysis the data. The average CT values of house keeping genes in each sample is calculated as house keeping gene CT value for that sample. ΔCT was calculated by subtracting house keeping CT value from individual assay CT value of same sample. ΔΔCT value was derived by further subtracting ΔCT value of control samples of each assay from its corresponding ΔCT value of treatment sample. This ΔΔCt value of those 16 genes is then compared to ΔΔCt value of 16 genes in our training data pool that contains 20 samples (8 negatively regulated and 12 positively regulated in terms of pathway activity). A support vector machine method is preferably used to analyze the ΔΔCT values of the samples and the expression thereof used to assess the regulatory status of the Wnt/β-catenin pathway activity in the sample.

Improving Sensitivity to Expression Level Differences

In using the biomarkers disclosed herein, and, indeed, using any sets of biomarkers to differentiate an individual or subject having one phenotype from another individual or subject having a second phenotype, one can compare the absolute expression of each of the biomarkers in a sample to a control; for example, the control can be the average level of expression of each of the biomarkers, respectively, in a pool of individuals or subjects. To increase the sensitivity of the comparison, however, the expression level values are preferably transformed in a number of ways.

For example, the expression level of each of the biomarkers can be normalized by the average expression level of all markers the expression level of which is determined, or by the average expression level of a set of control genes. Thus, in one embodiment, the biomarkers are represented by probes on a microarray, and the expression level of each of the biomarkers is normalized by the mean or median expression level across all of the genes represented on the microarray, including any non-biomarker genes. In a specific embodiment, the normalization is carried out by dividing the median or mean level of expression of all of the genes on the microarray. In another embodiment, the expression levels of the biomarkers is normalized by the mean or median level of expression of a set of control biomarkers. In a specific embodiment, the control biomarkers comprise a set of housekeeping genes. In another specific embodiment, the normalization is accomplished by dividing by the median or mean expression level of the control genes.

The sensitivity of a biomarker-based assay will also be increased if the expression levels of individual biomarkers are compared to the expression of the same biomarkers in a pool of samples. Preferably, the comparison is to the mean or median expression level of each the biomarker genes in the pool of samples. Such a comparison may be accomplished, for example, by dividing by the mean or median expression level of the pool for each of the biomarkers from the expression level each of the biomarkers in the sample. This has the effect of accentuating the relative differences in expression between biomarkers in the sample and markers in the pool as a whole, making comparisons more sensitive and more likely to produce meaningful results that the use of absolute expression levels alone. The expression level data may be transformed in any convenient way; preferably, the expression level data for all is log transformed before means or medians are taken.

In performing comparisons to a pool, two approaches may be used. First, the expression levels of the markers in the sample may be compared to the expression level of those markers in the pool, where nucleic acid derived from the sample and nucleic acid derived from the pool are hybridized during the course of a single experiment. Such an approach requires that new pool nucleic acid be generated for each comparison or limited numbers of comparisons, and is therefore limited by the amount of nucleic acid available. Alternatively, and preferably, the expression levels in a pool, whether normalized and/or transformed or not, are stored on a computer, or on computer-readable media, to be used in comparisons to the individual expression level data from the sample (i.e., single-channel data).

Thus, there is described the following method of classifying a first cell or organism as having one of at least two different phenotypes, where the different phenotypes comprise a first phenotype and a second phenotype. The level of expression of each of a plurality of genes in a first sample from the first cell or organism is compared to the level of expression of each of said genes, respectively, in a pooled sample from a plurality of cells or organisms, the plurality of cells or organisms comprising different cells or organisms exhibiting said at least two different phenotypes, respectively, to produce a first compared value. The first compared value is then compared to a second compared value, wherein said second compared value is the product of a method comprising comparing the level of expression of each of said genes in a sample from a cell or organism characterized as having said first phenotype to the level of expression of each of said genes, respectively, in the pooled sample. The first compared value is then compared to a third compared value, wherein said third compared value is the product of a method comprising comparing the level of expression of each of the genes in a sample from a cell or organism characterized as having the second phenotype to the level of expression of each of the genes, respectively, in the pooled sample. Optionally, the first compared value can be compared to additional compared values, respectively, where each additional compared value is the product of a method comprising comparing the level of expression of each of said genes in a sample from a cell or organism characterized as having a phenotype different from said first and second phenotypes but included among the at least two different phenotypes, to the level of expression of each of said genes, respectively, in said pooled sample. Finally, a determination is made as to which of said second, third, and, if present, one or more additional compared values, said first compared value is most similar, wherein the first cell or organism is determined to have the phenotype of the cell or organism used to produce said compared value most similar to said first compared value.

The compared values may each be ratios of the levels of expression of each of said genes. Each of the levels of expression of each of the genes in the pooled sample may be normalized prior to any of the comparing steps. The normalization of the levels of expression may be carried out by dividing by the median or mean level of the expression of each of the genes or dividing by the mean or median level of expression of one or more housekeeping genes in the pooled sample from said cell or organism. The normalized levels of expression may be subjected to a log transform, and the comparing steps comprise subtracting the log transform from the log of the levels of expression of each of the genes in the sample. The two or more different phenotypes may be different regulation status of the Wnt/b-catenin signaling pathway. The two or more different phenotypes may be different predicted responses to treatment with an agent that modulates the Wnt/b-catenin signaling pathway. The levels of expression of each of the genes, respectively, in the pooled sample or said levels of expression of each of said genes in a sample from the cell or organism characterized as having the first phenotype, second phenotype, or said phenotype different from said first and second phenotypes, respectively, may be stored on a computer or on a computer-readable medium.

The two phenotypes may be deregulated or Wnt/b-catenin signaling pathway status. The two phenotypes may be predicted Wnt/b-catenin signaling pathway-agent responder status. The two phenotypes may be pharmacodynamic effect and no pharmacodynamic effect of an agent on the Wnt/b-catenin signaling pathway.

The comparison may be made between the expression of each of the genes in the sample and the expression of the same genes in a pool representing only one of two or more phenotypes. In the context of Wnt/b-catenin signaling pathway status-correlated genes, for example, one can compare the expression levels of Wnt/b-catenin signaling pathway regulation status-related genes in a sample to the average level of the expression of the same genes in a "deregulated" pool of samples (as opposed to a pool of samples that include samples from patients having regulated and deregulated Wnt/b-catenin signaling pathway status). Thus, in this method, a sample is classified as having a deregulated Wnt/b-catenin signaling pathway status if the level of expression of prognosis-correlated genes exceeds a chosen coefficient of correlation to the average "deregulated Wnt/b-catenin signaling pathway" expression profile (i.e., the level of expression of Wnt/b-catenin signaling pathway status-correlated genes in a pool of samples from patients having a "deregulated Wnt/b-catenin signaling pathway status." Patients or subjects whose expression levels correlate more poorly with the "deregulated Wnt/b-catenin signaling pathway" expression profile (i.e., whose correlation coefficient fails to exceed the chosen coefficient) are classified as having a regulated Wnt/b-catenin signaling pathway status.

Of course, single-channel data may also be used without specific comparison to a mathematical sample pool. For example, a sample may be classified as having a first or a second phenotype, wherein the first and second phenotypes are related, by calculating the similarity between the expression of at least 5 markers in the sample, where the markers are correlated with the first or second phenotype, to the expression of the same markers in a first phenotype template and a second phenotype template, by (a) labeling nucleic acids derived from a sample with a fluorophore to obtain a pool of fluorophore-labeled nucleic acids; (b) contacting said fluorophore-labeled nucleic acid with a microarray under conditions such that hybridization can occur, detecting at each of a plurality of discrete loci on the microarray a flourescent emission signal from said fluorophore-labeled nucleic acid that is bound to said microarray under said conditions; and (c) determining the similarity of marker gene expression in the individual sample to the first and second templates, wherein if said expression is more similar to the first template, the sample is classified as having the first phenotype, and if said expression is more similar to the second template, the sample is classified as having the second phenotype.

## Methods for Classification of Expression Profiles

In preferred embodiments, the methods of the invention use a classifier for predicting Wnt/b-catenin signaling pathway regulation status of a sample, predicting response to agents that modulate the Wnt/b-catenin signaling pathway, assigning treatment to a subject, and/or measuring pharmacodynamic effect of an agent. The classifier can be based on any appropriate pattern recognition method that receives an input comprising a biomarker profile and provides an output comprising data indicating which patient subset the patient belongs. The classifier can be trained with training data from a training population of subjects. Typically, the training data comprise for each of the subjects in the training population a training marker profile comprising measurements of respective gene products of a plurality of genes in a suitable sample taken from the patient and outcome information, i.e., deregulated or regulated Wnt/b-catenin signaling pathway status.

In preferred embodiments, the classifier can be based on a classification (pattern recognition) method described below, e.g., profile similarity; artificial neural network; support vector machine (SVM); logic regression, linear or quadratic discriminant analysis, decision trees, clustering, principal component analysis, nearest neighbor classifier analysis, nearest shrunken centroid. Such classifiers can be trained with the training population using methods described in the relevant sections, infra.

The biomarker profile can be obtained by measuring the plurality of gene products in a cell sample from the subject using a method known in the art, e.g., a method described infra.

Various known statistical pattern recognition methods can be used in conjunction with the present invention. A classifier based on any of such methods can be constructed using the biomarker profiles and Wnt/b-catenin pathway signalling status data of training patients. Such a classifier can then be used to evaluate the Wnt/b-catenin pathway signalling status of a patient based on the patient's biomarker profile. The methods can also be used to identify biomarkers that discriminate between different Wnt/b-catenin signalling pathway regulation status using a biomarker profile and Wnt/b-catenin signalling pathway regulation data of training patients.

## Profile Matching

A subject can be classified by comparing a biomarker profile obtained in a suitable sample from the subject with a biomarker profile that is representative of a particular phenotypic state. Such a marker profile is also termed a "template profile" or a "template." The degree of similarity to such a template profile provides an evaluation of the subject's phenotype. If the degree of similarity of the subject marker profile and a template profile is above a predetermined threshold, the subject is assigned the classification represented by the template. For example, a subject's outcome prediction can be evaluated by comparing a biomarker profile of the subject to a predetermined template profile corresponding to a given phenotype or outcome, e.g., a Wnt/b-catenin signalling pathway template comprising measurements of the plurality of biomarkers which are representative of levels of the biomarkers in a plurality of subjects that have tumors with deregulated Wnt/b-catenin signalling pathway status.

In one embodiment, the similarity is represented by a correlation coefficient between the subject's profile and the template. In one embodiment, a correlation coefficient above a correlation threshold indicates a high similarity, whereas a correlation coefficient below the threshold indicates a low similarity.

## Artificial Neural Network

In some embodiments, a neural network is used. A neural network can be constructed for a selected set of molecular markers of the invention. A neural network is a two-stage regression or classification model. A neural network has a layered structure that includes a layer of input units (and the bias) connected by a layer of weights to a layer of output units. For regression, the layer of output units typically includes just one output unit. However, neural networks can handle multiple quantitative responses in a seamless fashion. In multilayer neural networks, there are input units (input layer), hidden units (hidden layer), and output units (output layer). There is, furthermore, a single bias unit that is connected to each unit other than the input units. Neural networks are described in Duda et al., 2001, Pattern Classification, Second Edition, John Wiley & Sons, Inc., New York; and Hastie et al., 2001, The Elements of Statistical Learning, Springer-Verlag, New York.

Support Vector Machine

In some embodiments of the present invention, support vector machines (SVMs) are used to classify subjects using expression profiles of marker genes described in the present invention. General description of SVM can be found in, for example, Cristianini and Shawe-Taylor, 2000, An Introduction to Support Vector Machines, Cambridge University Press, Cambridge, Baser et al., 1992, "A training algorithm for optimal margin classifiers, in Proceedings of the 5th Annual ACM Workshop on Computational Learning Theory, ACM Press, Pittsburgh, Pa., pp. 142-152; Vapnik, 1998, Statistical Learning Theory, Wiley, New York; Duda, Pattern Classification, Second Edition, 2001, John Wiley & Sons, Inc.; Hastie, 2001, The Elements of Statistical Learning, Springer, N.Y.; and Furey et al, 2000, Bioinformatics 16, 906-914. Applications of SVM in biological applications are described in Jaakkola et al., Proceedings of the 7th International Conference on Intelligent Systems for Molecular Biology, AAAI Press, Menlo Park, Calif. (1999); Brown et al., Proc. Natl. Acad. Sci. 97(1):262-67 (2000); Zien et al., Bioinformatics, 16(9):799-807 (2000); Furey et al., Bioinformatics, 16(10):906-914 (2000).

In some embodiments, the classifier is based on a regression model, preferably a logistic regression model. Such a regression model includes a coefficient for each of the molecular markers in a selected set of molecular biomarkers of the invention. In such embodiments, the coefficients for the regression model are computed using, for example, a maximum likelihood approach. In particular embodiments, molecular biomarker data from two different classification or phenotype groups, e.g., deregulated or regulated Wnt/b-catenin signaling pathway, response or non-response to treatment to an agent that modulates the Wnt/b-catenin signaling pathway, is used and the dependent variable is the phenotypic status of the patient for which molecular marker characteristic data are from.

Some embodiments of the present invention provide generalizations of the logistic regression model that handle multicategory (polychotomous) responses. Such embodiments can be used to discriminate an organism into one or three or more classification groups, e.g., good, intermediate, and poor therapeutic response to treatment with Wnt/b-catenin signaling pathway agents. Such regression models use multicategory logic models that simultaneously refer to all pairs of categories, and describe the odds of response in one category instead of another. Once the model specifies logits for a certain (J-1) pairs of categories, the rest are redundant. See, for example, Agresti, An Introduction to Categorical Data Analysis, John Wiley & Sons, Inc., 1996, New York, Chapter 8.

Discriminant Analysis

Linear discriminant analysis (LDA) attempts to classify a subject into one of two categories based on certain object properties. In other words, LDA tests whether object attributes measured in an experiment predict categorization of the objects. LDA typically requires continuous independent variables and a dichotomous categorical dependent variable. In the present invention, the expression values for the selected set of molecular markers of the invention across a subset of the training population serve as the requisite continuous independent variables. The clinical group classification of each of the members of the training population serves as the dichotomous categorical dependent variable.

LDA seeks the linear combination of variables that maximizes the ratio of between-group variance and within-group variance by using the grouping information. Implicitly, the linear weights used by LDA depend on how the expression of a molecular biomarker across the training set separates in the two groups (e.g., a group that has deregulated Wnt/b-catenin signaling pathway and a group that have regulated Wnt/b-catenin signaling pathway status) and how this gene expression correlates with the expression of other genes. In some embodiments, LDA is applied to the data matrix of the N members in the training sample by K genes in a combination of genes described in the present invention. Then, the linear discriminant of each member of the training population is plotted. Ideally, those members of the training population representing a first subgroup (e.g. those subjects that have deregulated Wnt/b-catenin signaling pathway status) will cluster into one range of linear discriminant values (e.g., negative) and those member of the training population representing a second subgroup (e.g. those subjects that have regulated Wnt/b-catenin signaling pathway status) will cluster into a second range of linear discriminant values (e.g., positive). The LDA is considered more successful when the separation between the clusters of discriminant values is larger. For more information on linear discriminant analysis, see Duda, Pattern Classification, Second Edition, 2001, John Wiley & Sons, Inc; and Hastie, 2001, The Elements of Statistical Learning, Springer, N.Y.; Venables & Ripley, 1997, Modern Applied Statistics with s-plus, Springer, N.Y. Quadratic discriminant analysis (QDA) takes the same input parameters and returns the same results as LDA. QDA uses quadratic equations, rather than linear equations, to produce results. LDA and QDA are interchangeable, and which to use is a matter of preference and/or availability of software to support the analysis. Logistic regression takes the same input parameters and returns the same results as LDA and QDA.

Decision Trees

In some embodiments of the present invention, decision trees are used to classify subjects using expression data for a selected set of molecular biomarkers of the invention. Decision tree algorithms belong to the class of supervised learning algorithms. The aim of a decision tree is to induce a classifier (a tree) from real-world example data. This tree can be used to classify unseen examples which have not been used to derive the decision tree.

A decision tree is derived from training data. An example contains values for the different attributes and what class the example belongs. In one embodiment, the training data is expression data for a combination of genes described in the present invention across the training population.

Clustering

In some embodiments, the expression values for a selected set of molecular markers of the invention are used to cluster a training set. For example, consider the case in which ten gene biomarkers described in one of the genes of the present invention are used. Each member m of the training population will have expression values for each of the ten biomarkers. Such values from a member m in the training population define the vector: Those members of the training population that exhibit similar expression patterns across the training group will tend to cluster together. A particular combination of genes of the present invention is considered to be a good classifier in this aspect of the invention when the vectors cluster into the trait groups found in the training population. For instance, if the training population includes patients with good or poor prognosis, a clustering classifier will cluster the population into two groups, with each group uniquely representing either a deregulated Wnt/b-catenin signalling pathway status or a regulated Wnt/b-catenin signalling pathway status.

Clustering is described on pages 211-256 of Duda and Hart, Pattern Classification and Scene Analysis, 1973, John Wiley & Sons, Inc., New York. As described in Section 6.7 of Duda, the clustering problem is described as one of finding natural groupings in a dataset. To identify natural groupings, two issues are addressed. First, a way to measure similarity (or dissimilarity) between two samples is determined. This metric (similarity measure) is used to ensure that the samples in one cluster are more like one another than they are to samples in other clusters. Second, a mechanism for partitioning the data into clusters using the similarity measure is determined.

Similarity measures are discussed in Section 6.7 of Duda, where it is stated that one way to begin a clustering investigation is to define a distance function and to compute the matrix of distances between all pairs of samples in a dataset. If distance is a good measure of similarity, then the distance between samples in the same cluster will be significantly less than the distance between samples in different clusters. However, as stated on page 215 of Duda, clustering does not require the use of a distance metric. For example, a nonmetric similarity function s(x, x') can be used to compare two vectors x and x'. Conventionally, s(x, x') is a symmetric function whose value is large when x and x' are somehow "similar". An example of a nonmetric similarity function s(x, x') is provided on page 216 of Duda.

Once a method for measuring "similarity" or "dissimilarity" between points in a dataset has been selected, clustering requires a criterion function that measures the clustering quality of any partition of the data. Partitions of the data set that extremize the criterion function are used to cluster the data. See page 217 of Duda. Criterion functions are discussed in Section 6.8 of Duda. More recently, Duda et al., Pattern Classification, 2nd edition, John Wiley & Sons, Inc. New York, has been published. Pages 537-563 describe clustering in detail. More information on clustering techniques can be found in Kaufman and Rousseeuw, 1990, Finding Groups in Data: An Introduction to Cluster Analysis, Wiley, New York, N.Y.; Everitt, 1993, Cluster analysis (3d ed.), Wiley, New York, N.Y.; and Backer, 1995, Computer-Assisted Reasoning in Cluster Analysis, Prentice Hall, Upper Saddle River, N.J. Particular exemplary clustering techniques that can be used in the present invention include, but are not limited to, hierarchical clustering (agglomerative clustering using nearest-neighbor algorithm, farthest-neighbor algorithm, the average linkage algorithm, the centroid algorithm, or the sum-of-squares algorithm), k-means clustering, fuzzy k-means clustering algorithm, and Jarvis-Patrick clustering.

Principal Component Analysis

Principal component analysis (PCA) has been proposed to analyze gene expression data. Principal component analysis is a classical technique to reduce the dimensionality of a data set by transforming the data to a new set of variable (principal components) that summarize the features of the data. See, for example, Jolliffe, 1986, Principal Component Analysis, Springer, N.Y. Principal components (PCs) are uncorrelate and are ordered such that the kth PC has the kth largest variance among PCs. The kth PC can be interpreted as the direction that maximizes the variation of the projections of the data points such that it is orthogonal to the first k-1 PCs. The first few PCs capture most of the variation in the data set. In contrast, the last few PCs are often assumed to capture only the residual 'noise' in the data.

PCA can also be used to create a classifier in accordance with the present invention. In such an approach, vectors for a selected set of molecular biomarkers of the invention can be constructed in the same manner described for clustering above. In fact, the set of vectors, where each vector represents the expression values for the select genes from a particular member of the training population, can be considered a matrix. In some embodiments, this matrix is represented in a Free-Wilson method of qualitative binary description of monomers (Kubinyi, 1990, 3D QSAR in drug design theory methods and applications, Pergamon Press, Oxford, pp 589-638), and distributed in a maximally compressed space using PCA so that the first principal component (PC) captures the largest amount of variance information possible, the second principal component (PC) captures the second largest amount of all variance information, and so forth until all variance information in the matrix has been accounted for.

Then, each of the vectors (where each vector represents a member of the training population) is plotted. Many different types of plots are possible. In some embodiments, a one-dimensional plot is made. In this one-dimensional plot, the value for the first principal component from each of the members of the training population is plotted. In this form of plot, the expectation is that members of a first group will cluster in one range of first principal component values and members of a second group will cluster in a second range of first principal component values.

In one example, the training population comprises two classification groups. The first principal component is computed using the molecular biomarker expression values for the select genes of the present invention across the entire training population data set where the classification outcomes are known. Then, each member of the training set is plotted as a function of the value for the first principal component. In this example, those members of the training population in which the first principal component is positive represent one classification outcome and those members of the training population in which the first principal component is negative represent the other classification outcome. In some embodiments, the members of the training population are plotted against more than one principal component. For example, in some embodiments, the members of the training population are plotted on a two-dimensional plot in which the first dimension is the first principal component and the second dimension is the second principal component. In such a two-dimensional plot, the expectation is that members of each subgroup represented in the training population will cluster into discrete groups. For example, a first cluster of members in the two-dimensional plot will represent subjects in the first classification group, a second cluster of members in the two-dimensional plot will represent subjects in the second classification group, and so forth.

In some embodiments, the members of the training population are plotted against more than two principal components and a determination is made as to whether the members of the training population are clustering into groups that each uniquely represents a subgroup found in the training population. In some embodiments, principal component analysis is performed by using the R mva package (Anderson, 1973, Cluster Analysis for applications, Academic Press, New York 1973; Gordon, Classification, Second Edition, Chapman and Hall, CRC, 1999.). Principal component analysis is further described in Duda, Pattern Classification, Second Edition, 2001, John Wiley & Sons, Inc.

Nearest Neighbor Classifier Analysis

Nearest neighbor classifiers are memory-based and require no model to be fit. Given a query point x0, the k training points x(r), r, ... , k closest in distance to x0 are identified and then the point x0 is classified using the k nearest neighbors. Ties can be broken at random. In some embodiments, Euclidean distance in feature space is used to determine distance as:

$$d(i)=.parallel.x(i)-xo.parallel.$$

Typically, when the nearest neighbor algorithm is used, the expression data used to compute the linear discriminant is standardized to have mean zero and variance 1. In the present invention, the members of the training population are randomly divided into a training set and a test set. For example, in one embodiment, two thirds of the members of the training population are placed in the training set and one third of the members of the training population are placed in the test set. Profiles of a selected set of molecular biomarkers of the invention represents the feature space into which members of the test set are plotted. Next, the ability of the training set to correctly characterize the members of the test set is computed. In some embodiments, nearest neighbor computation is performed several times for a given combination of genes of the present invention. In each iteration of the computation, the members of the training population are randomly assigned to the training set and the test set. Then, the quality of the combination of genes is taken as the average of each such iteration of the nearest neighbor computation. The nearest neighbor rule can be refined to deal with issues of unequal class priors, differential misclassification costs, and feature selection. Many of these refinements involve some form of weighted voting for the neighbors. For more information on nearest neighbor analysis, see Duda, Pattern Classification, Second Edition, 2001, John Wiley & Sons, Inc; and Hastie, 2001, The Elements of Statistical Learning, Springer, N.Y.

Evolutionary Methods

Inspired by the process of biological evolution, evolutionary methods of classifier design employ a stochastic search for an optimal classifier. In broad overview, such methods create several classifiers--a population--from measurements of gene products of the present invention. Each classifier varies somewhat from the other. Next, the classifiers are scored on expression data across the training population. In keeping with the analogy with biological evolution, the resulting (scalar) score is sometimes called the fitness. The classifiers are ranked according to their score and the best classifiers are retained (some portion of the total population of classifiers). Again, in keeping with biological terminology, this is called survival of the fittest. The classifiers are stochastically altered in the next generation--the children or offspring. Some offspring classifiers will have higher scores than their parent in the previous generation, some will have lower scores. The overall process is then repeated for the subsequent generation: The classifiers are scored and the best ones are retained, randomly altered to give yet another generation, and so on. In part, because of the ranking, each generation has, on average, a slightly higher score than the previous one. The process is halted when the single best classifier in a generation has a score that exceeds a desired criterion value. More information on evolutionary methods is found in, for example, Duda, Pattern Classification, Second Edition, 2001, John Wiley & Sons, Inc.

Bagging, Boosting and the Random Subspace Method

Bagging, boosting and the random subspace method are combining techniques that can be used to improve weak classifiers. These techniques are designed for, and usually applied to, decision trees. In addition, Skurichina and Duin provide evidence to suggest that such techniques can also be useful in linear discriminant analysis.

In bagging, one samples the training set, generating random independent bootstrap replicates, constructs the classifier on each of these, and aggregates them by a simple majority vote in the final decision rule. See, for example, Breiman, 1996, Machine Learning 24, 123-140; and Efron & Tibshirani, An Introduction to Bootstrap, Chapman & Hall, New York, 1993.

In boosting, classifiers are constructed on weighted versions of the training set, which are dependent on previous classification results. Initially, all objects have equal weights, and the first classifier is constructed on this data set. Then, weights are changed according to the performance of the classifier. Erroneously classified objects (molecular biomarkers in the data set) get larger weights, and the next classifier is boosted on the reweighted training set. In this way, a sequence of training sets and classifiers is obtained, which is then combined by simple majority voting or by weighted majority voting in the final decision. See, for example, Freund & Schapire, "Experiments with a new boosting algorithm," Proceedings 13th International Conference on Machine Learning, 1996, 148-156.

In some embodiments, modifications of Freund and Schapire, 1997, Journal of Computer and System Sciences 55, pp. 119-139, are used. For example, in some embodiments, feature pre-selection is performed using a technique such as the nonparametric scoring methods of Park et al., 2002, Pac. Symp. Biocomput. 6, 52-63. Feature pre-selection is a form of dimensionality reduction in which the genes that discriminate between classifications the best are selected for use in the classifier. Then, the LogitBoost procedure introduced by Friedman et al., 2000, Ann Stat 28, 337-407 is used rather than the boosting procedure of Freund and Schapire. In some embodiments, the boosting and other classification methods of Ben-Dor et al., 2000, Journal of Computational Biology 7, 559-583 are used in the present invention. In some embodiments, the boosting and other classification methods of Freund and Schapire, 1997, Journal of Computer and System Sciences 55, 119-139, are used.

In the random subspace method, classifiers are constructed in random subspaces of the data feature space. These classifiers are usually combined by simple majority voting in the final decision rule. See, for example, Ho, "The Random subspace method for constructing decision forests," IEEE Trans Pattern Analysis and Machine Intelligence, 1998; 20(8): 832-844.

Other Algorithms

The pattern classification and statistical techniques described above are merely examples of the types of models that can be used to construct a model for classification. Moreover, combinations of the techniques described above can be used. Some combinations, such as the use of the combination of decision trees and boosting, have been described. However, many other combinations are possible. In addition, in other techniques in the art such as Projection Pursuit and Weighted Voting can be used to construct a classifier.

As discussed in the Experimental Section, expression of the subject biomarker genes is preferably determined after real-time PCR using SYBR Green, and a $\Delta\Delta$CT method employed to analysis the data. The average CT values of house keeping genes in each sample is calculated as house keeping gene CT value for that sample. $\Delta$CT was calculated by subtracting house keeping CT value from individual assay CT value of same sample. $\Delta\Delta$CT value was derived by further subtracting $\Delta$CT value of control samples of each assay from its corresponding $\Delta$CT value of treatment sample. A support vector machine method is preferably used to analyze the $\Delta\Delta$CT values of the samples and the expression thereof used to assess the regulatory status of the Wnt/$\beta$-catenin pathway activity in the sample.

## EXAMPLES

## EXPERIMENTAL METHODS USED TO IDENTIFY INVENTIVE WNT/β-CATENIN SIGNALING (16) GENE SIGNATURE

### Identification of Wnt/β-catenin response genes by gene expression profiling

The protocol that was used to identify the subject gene signature is depicted schematically in Figure 1. As depicted therein, human embryonic kidney 293H cells were plated in 6-well plate in a density of $10^5$ cells per well in 2 ml growth medium. Plated cells were incubated in a cell culture incubator at 37 degrees C with 5% CO2 supplied. Twenty four hours after plating, cells were transfected with siRNA specifically targeting β-catenin or non targeting siRNA as control. For each well, 6 μl of SureFECT transfection reagent (SABiosciences, a QIAGEN company) was diluted into 200 μl of OptiMEM medium (Invitrogen). The diluted transfection reagent was mixed with 20 nM -β catenin targeting siRNA duplex D (GTTCCGAATGTCTGAGGACAA (SEQ ID NO: 65)) (SABiosciences, a QIAGEN Company) or non-targeting siRNA (ACACTAAGTACGTCGTATTAC (SEQ ID NO: 66)) (SABiosciences, a QIAGEN owned company) as control. After incubation at room temperature for 20 min, the transfection mixture was added into 293H cells in 6-well plate with 2 ml growth medium. Twenty four hours after transfection, the medium with transfection mixture was replaced with 1 ml serum-free medium in each well and cells were incubated for 12 hours in serum-free medium. After 12 hours serum starvation, these cells were replaced with 1 ml serum-free medium with either 400 ng/ml mouse recombinant Wnt3a (R&D Systems) or PBS as control.

At the end of the 12 hour Wnt3a treatment, cells were lysed in 50 μl of Modified RIPA buffer (150 mM NaCl, 50 mM TrisHC1, 1% IGEPAL, 0.5% sodium deoxycholate, 1mM EDTA, 1% Triton X-100 and 0.1% SDS with protease and phosphatase inhibitor) to check the protein levels of active β-catenin with western blot according to cell lysis and western blot protocol described in the experimental protocols section. The mouse anti-active β-catenin (1:1000) (Millipore) was used to detect active-β-catenin level. The mouse anti-β-catenin (1:2000) (BD Transduction lab) and rabbit anti-GAPDH (1:2000) (Sigma) were used to detect total β-catenin and GAPDH levels as control. The effect of Wnt3a treatment was demonstrated by increased protein levels of active β-catenin in Wnt3a treated samples compared to untreated samples (See results contained in Figure 2A)

For RNA extraction, cells were lysed in 200 μl of G6 buffer (SABiosciences, a QIAGEN company) for each well. The lysates were further processed to RNA isolation with RT2 qPCR-Grade RNA Isolation Kit from SABiosciences according to protocol described in the experimental protocols section. At the end of isolation, 30 μl of RNase-free water was added to spin column to elute RNA off column. The concentration of RNA was measured with Nanodrop spectrophotometer (Thermo SCIENTIFIC) and the RNA was further processed for real-time RT PCR or microarray gene expression profiling analysis.

Real-time RT-PCR was employed to confirm the effect of Wnt3a treatment and β-catenin siRNA knockdown by measuring mRNA expression levels of Wnt/β-catenin target genes and β-catenin itself respectively. 1 μg of total RNA was reverse transcribed with RT2 EZ First Strand cDNA synthesis kit (SABiosciences, a QIAGEN company) according to protocol described in the experimental protocols section. The 20μl cDNA reaction was diluted to 100 μl of water for real-time PCR analysis. For each real-time PCR reaction mixture, 1 μl of cDNA was mixed with μl of 10 μM primer mixture (forward and reverse primers mixed), 12.5 μl of real-time PCR master mixture (SABiosciences, a QIAGEN company) and 10.5 μl water to a total volume of 25 μl. The primer sequences used for CTNNB1, MYC, CCND1, FRA-1, AXIN2 and SOX9 are in the Table below:

| | | | |
|---|---|---|---|
| CTNNB1 | NM_001904 | ACTTGCATTGTGATTGGCCTG (SEQ ID NO: 67) | AATCCATTTGTATTGTTACTCCTCG (SEQ ID NO: 68) |
| MYC | NM_002467 | AGATCCGGAGCGAATAGGG (SEQ ID NO: 69) | CCTTGCTCGGGTGTTGTAAGT (SEQ ID NO: 70) |
| CCND1 | NM_053056 | TTGTACCTGTAGGACTCTCATTCG (SEQ ID NO: 71) | CACTGTGAGCTGGCTTCATTG (SEQ ID NO: 72) |
| FOSL1 | NM_005438 | GGACAGTATCCCACATCCAAC (SEQ ID NO: 73) | CAAATTGTGCTAGAGAGGCCAG (SEQ ID NO: 74) |
| AXIN2 | NM_004655 | GGCCTGCTAGAATCACTGC (SEQ ID NO: 75) | CTCCCTACAACTGTTCATTTCTTTG (SEQ ID NO: 76) |
| SOX9 | NM_000346 | CCAGCTCCTACTACAGCCACG (SEQ ID NO: 77) | TGTGTGTAGACGGGTTGTTCC (SEQ ID NO: 78) |

The reaction mixture was added into 384-well real-time PCR plate in duplicate wells with 10 μl each well. The PCR plate was sealed with optical adhesive film (Applied Biosystems) and centrifuged for 2 minutes at 2000 rpm. The real-time PCR was run in ABI 7900 real-time PCR machine (Applied Biosystems) with PCR program as following, 95 degrees C for 10 min, 40 cycles of 95 degrees C 15 seconds and 60 degrees C 1 minutes following melting curve analysis. The effect of Wnt3a treatment was confirmed by upregulation of Wnt/β-catenin target genes such as β-catenin (CTNNB1), MYC, CCND1 and AXIN2 in Wnt3a treated samples compared to untreated samples (See Figure 2B). The siRNA knockdown of β-catenin was verified by decreased mRNA expression levels of β-catenin (See Figure 2B).

After the confirmation of Wnt3a treatment and β-catenin siRNA knockdown, RNA samples were processed to whole genome microarray gene profiling analysis. The 12 samples were split into four treatment groups in triplicates, sinon-no wnt, sinon-wnt, siCTNNB1-no wnt and siCTNNB1-wnt. 300 ng of total RNA was amplified and labeled with TargetAmp Nano-g Biotin-aRNA Labeling Kit (Epicentre Biotechnologies) according to manufacturer's protocol.

The amplification and labeling reagents and reaction parameters are shown below.

| **1-strand cDNA synthesis** | RNA con | RNA amount | H2O | T7-Oligo(dT)primer | total |
|---|---|---|---|---|---|
| Sample | ng/μl | 300 ng | | | |
| si-non wnt 0 12h (2) | 854.72 | 0.35 | 1.65 | 1 | 3.00 |
| si-non wnt 0 12h (3) | 866.62 | 0.35 | 1.65 | 1 | 3.00 |
| si-non wnt 0 12h (4) | 952.17 | 0.32 | 1.68 | 1 | 3.00 |
| si-non wnt 400 12h (17) | 582.53 | 0.51 | 1.49 | 1 | 3.00 |
| si-non wnt 400 12h (18) | 796.17 | 0.38 | 1.62 | 1 | 3.00 |
| si-non wnt 400 12h (20) | 845.58 | 0.35 | 1.65 | 1 | 3.00 |
| siCTNNB1-D wnt 0 12 hours (1) | 888.41 | 0.34 | 1.66 | 1 | 3.00 |
| siCTNNB1-D wnt 0 12 hours (2) | 932.97 | 0.32 | 1.68 | 1 | 3.00 |
| siCTNNB1-D wnt 0 12 hours (3) | 880.03 | 0.34 | 1.66 | 1 | 3.00 |
| siCTNNB1-D wnt 400 12 hours (18) | 955.61 | 0.31 | 1.69 | 1 | 3.00 |
| siCTNNB1-D wnt 400 12 hours (19) | 812.59 | 0.37 | 1.63 | 1 | 3.00 |
| siCTNNB1-D wnt 400 12 hours (20) | 1016.87 | 0.30 | 1.70 | 1 | 3.00 |

incubate 65°C for 5min, chill on ice 1 min, centrifuge briefly

| *1st strand cDNA synthesis master mix* | 14 | |
|---|---|---|
| 1st strand cDNA premix | 21 | |
| DTT | 3.5 | |
| superscript III (200u/μl) | 3.5 | |
| Total | 28 | gently mix |

add 2 μl to each reaction, gently mix, incubate 50°C for 30min.

**second strand cDNA synthesis**

| *second strand cDNA synthesis master mix* | 13 | |
|---|---|---|
| 2nd-strand cDNA premix | 58.5 | |
| 2nd-strand DNA polymerase | 6.5 | |
| Total | 65 | gently mix |

add 5 μl to each reaction, gently mix, incubate 65°C 10min, centrifuge briefly.
incubate at 80°C for 3min, centrifuge briefly, chill on ice.

**In vitro transcription of biotin-aRNA**

warm T7 RNA polymerase to RT and thaw other reagent at RT

| | | |
|---|---|---|
| *in vitro transcription master mix* | 13 | |
| setup at RT | | |
| T7 transcription buffer | 26 | |
| UTP/biotin-UTP | 39 | |
| NTP premix | 130 | |
| DTT | 39 | |
| T7 RNA polymerase | 26 | |
| Total | 260 | gently mix |

add 20 μl to each reaction, gently mix, incubate 42°C for 4 hours (don't exceed 4h)
add 2 μl Rnase-free Dnase I to each reaction, mix gently, incubate 37°C 15min.

**biotin-aRNA purification (SABio cRNA cleanup kit)**

*bind aRNA to spin column*

a. transfer entire reaction (32 μl) to 1.5 ml tube
b. add 112 μl lysis & binding buffer (G6) to each reaction, mix by pipetting 2-3x
c. add 112 μl RT 100% ETOH, mix by pipettting 5-6x
d. immediately load on spin column
e. centrifuge 8000g for 30sec
f. discard flow-through, put column back to collection

*washing spin column*

a. add 400-500 μl washing buffer (G17+ETOH) to each spin column
b. centrifuge 8000g 30sec
c. discard flow-through, put column back to collection tube
d. add 200 μl washing buffer (G17+ETOH) to each spin column
e. centrifuge 11000g 1 min
f. discard flow-through, put column back to collection tube
g. centrifuge 11000g 2min (180° rotate from previous orientation)

*elute aRNA from spin column*

a. transfer spin column to new elution tube
b. add 40 μl (<40 ug, 80 μl if >40ug) H2O into column
c. sit in RT 2min
d. centrifuge 8000g for 1 min
e. store aRNA -80°C

The concentration of labeled antisense RNA was measured with Nanodrop spectrophotometer (Thermo SCIENTIFIC). Total 750 ng of labeled antisense RNA was hybridized onto an Illumina Human HT-12 BeadChip (Illumina) according to manufacturer's protocol described in experimental protocol section for a 12 sample chip (Illumina Whole Genome Gene Expression Direct Hybridization Assay).

Hybridized BeadChip was washed and scanned on an iScan (Illumina) according to the manufacturer's standard protocol. The image file was processed with GenomeStudio software (Illumina) without background correction and normalization. The sample probe expression file was exported as GeneSpring format for further analysis with GeneSpring software (Agilent). The expression data was analyzed with GeneSpring with its guided workflow and fold changes and statistical analysis was computed between groups during the guided workflow analysis.

After effecting these protocols we selected from the identified Wnt/ β-catenin response genes, those whose expression levels were significantly changed with Wnt3a stimulation with an adjusted P value <= 0.05 when comparing Wnt3a treated to no Wnt3a treated samples in the absence of β-catenin siRNA. Also, further selection was effected based on the effect of β-catenin siRNA on those Wnt3a stimulated genes whose Wnt3a stimulated expression was diminished by at least 1.3 fold after siRNA transfection. After excluding genes whose expression change direction was inconsistent between Wnt3a and siRNA treatment, 64 genes were selected as Wnt/β-catenin response genes.

This data is contained in the Excel spreadsheet shown in FIG. 6.

**Identification of Wnt/β-catenin gene expression signature**

To validate these 64 Wnt/β-catenin response genes with real-time PCR,SYBR green based real-time PCR assay was designed for each individual gene. The sequence information for all primers is contained in Figure 5A-B.

Twenty samples were employed to test the expression of these 64 genes. The Wnt/β-catenin pathway activity was negatively regulated in eight samples including β-catenin siRNA treatment of human colon cancer HCT116 and SW480 cells harboring constitutively active β-catenin as well as 293H cells transfected with β-catenin siRNA in the presence of Wnt3a treatment (same sample used in microarray experiment). In contrast twelve samples had their Wnt/β-catenin pathway activity positively regulated including ten samples stimulated with Wnt3a and two sample treated with LiCl (Sigma), a compound known to activate Wnt/β-catenin pathway activity.

The β-catenin siRNA was reverse transfected into HCT116, SW480 , 293H, U373MG, MCF7, HT29, Lncap and HepG2 cells. For each well of 6-well plate, 6 μl of SureFECT transfection reagent (SABiosciences, a QIAGEN Company) was diluted into 200 μl of OptiMEM medium (Invitrogen). The diluted transfection reagent was mixed with 40 nM β-catenin targeting siRNA duplex A
(GCAGTTCGCCTTCACTATGGA (SEQ ID NO: 79)) or non-targeting siRNA (ACACTAAGTACGTCGTATTAC (SEQ ID NO: 80)) (SABiosciences, a QIAGEN Company) as control. Master transfection mixture for 4 wells was prepared for either β-catenin siRNA or non-target siRNA. After incubation at room temperature for 20 minutes, 200 μl of transfection mixture was added into each well in eight 6-well plates with one plate for each cell line including HCT116, SW480, 293H, U373MG, MCF7, HT29, Lncap and HepG2. Each plate had two wells containing β-catenin siRNA mixture and two wells containing non-target siRNA mixture. These two duplicate wells were for protein extraction and RNA isolation respectively. During the 20 minute incubation time, different cell lines were trypsinized, washed off plate and resuspended in 8 ml culture medium (MeCoy's 5A with 10% FBS) and cell numbers were counted with a hemocytometer.

Cells were diluted into culture medium in a concentration of $6x10^4$ cells per ml. For each well, 2 ml of cells (1.2x $10^5$) were plated in 6-well plate on top of 200 μl transfection mixture and the plate was mixed well. The cell culture plates were put back into incubator and incubated for 72 hours at 37 degrees C with 5% CO2 supplied. At the end of 72 hours incubation, cells were either lysed in 50 μl modified RIPA buffer for protein lysate extraction or in 200 μl lysis G6 buffer for RNA isolation. The protein extraction and western blot was carried out according to western blot protocol in the experimental protocols section with mouse anti-active β-catenin (1:1000) and mouse anti-β-catenin (1:2000) antibody. The decreased β-catenin protein levels in both active and total forms verified the effect of β-catenin siRNA (See Figure 3A) . The RNA was isolated with RT2 qPCR-Grade RNA Isolation Kit from SABiosciences according to manufacturer's protocol as described in experimental protocol section.

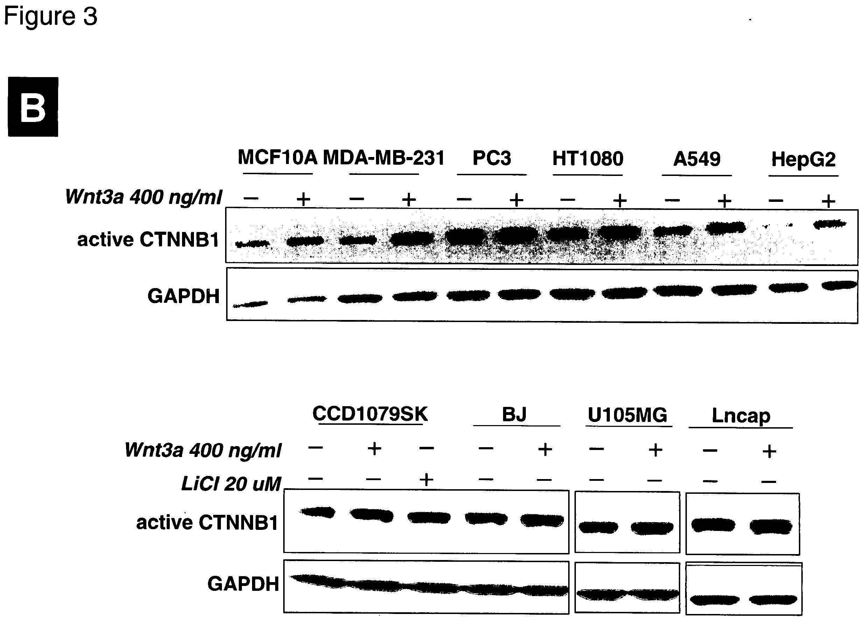

To obtain ten positively regulated samples with Wnt3a treatment, ten different cell lines were plated in 6-well plates in a density of $2x10^5$ cells/well/2ml. After 24h of plating, cells were switched to serum-free medium by removing normal culture medium, washing cells in PBS two times and replacing with 1 ml serum-free medium each well. After 12 hours in serum-free medium, cells were replaced with serum-free medium with or without 400 ng/ml Wnt3a in duplicate wells for an additional incubation of 12 h. At the end of 12 hours incubation, cells were either lysed in 50 μl modified RIPA buffer for protein lysate extraction or in 200 μl G6 lysis buffer for RNA isolation. The protein extraction and western blot was carried out according to western blot protocol with mouse anti-active β-catenin antibody (1:1000). The increased active β-catenin protein levels confirmed the effect of Wnt3a (See Figure 3B). The RNA was isolated with RT2 qPCR-Grade RNA Isolation Kit from SABiosciences according to manufacturer's protocol described in experimental protocol section.

For positive regulation with LiCl treatment, 293H and CCD1079SKcells were plated in 6-well plate in a density of 2x $10^5$ cells/well/2ml and cells were switched to serum-free medium 24 hours after plating. After 12 hours incubation in serum-free medium, cells were treated with or without 20 μM LiCl for 24h in serum-free medium. At the end of treatment, cells were lysed in 200 μl of G6 lysis buffer and RNA was isolated with RT2 qPCR-Grade RNA Isolation Kit from SABiosciences according to manufacturer's protocol described in experimental protocol section. The protein extraction and western blot was carried out according to western blot protocol with mouse anti-active β-catenin antibody (1:1000). The increased active β-catenin protein levels confirmed the effect of LiCl (See Figure 3B). The RNA was isolated with RT2 qPCR-Grade RNA Isolation Kit from SABiosciences according to manufacturer's protocol described in experimental protocol section.

To verify 64 Wnt/β-catenin response genes with SYBR green based real-time PCR on the described three negative and eight positive samples, 1 μg of total RNA was reversed transcribed with RT2 First Strand cDNA synthesis kit (SABiosciences, a QIAGEN company) according to manufacturer's protocol in experimental protocol section. The 20 μl of reverse transcription reaction was diluted to 200 μl with water. For each real-time PCR reaction, 1 μl of diluted cDNA was mixed with 5 μl of SYBR green PCR master mixture and 4 μl of water to give a final volume of 10 μl of each reaction. A master mixture of 90 real-time PCR reactions was prepared for each sample and added into 384-well plate with 10 μl for each well. Each sample had 72 reactions in 72 wells corresponding to 72 different PCR assays (64 Wnt/β-catenin response genes plus 8 house keeping genes) and each 384-well plate was loaded with reactions for 4 samples (72x4 wells). The 384-well plates were run in ABI 7900 real-time PCR machine (Applied Biosystems) with SYBR green based real-time PCR program as following, 95 degrees C for 10 min, 40 cycles of 95 degrees C 15 seconds and 60 degrees C 1 minutes following by melting curve analysis.

After real-time PCR, a ΔΔCT statistical analysis method was employed to analyze the data. The average CT values of 8 house keeping genes in each sample were calculated as house keeping gene CT value for that sample. ΔCT was calculated by subtracting house keeping CT value from individual assay CT value of same sample. The ΔΔCT value was derived by further subtracting ΔCT value of control samples of each assay from its corresponding ΔCT value of treatment sample. A nearest shrunken centroid classifier method (Proc Natl Acad Sci USA. 2002 May 14; 99(10):6567-72.) was used to analyze the ΔΔCT values of 8 negative and 12 positive samples. Sixteen genes were identified as a gene expression signature that differentially classified positive samples from negative samples (Figure 4B) . These 16 genes and their Accession numbers are listed in the table below.

| Gene Symbol | Genbank Accession Number |
|---|---|
| CALM1 | NM_006888.4 |
| CCND1 | NM_053056.2 |
| CCND2 | NM_001759.3 |
| CHSY1 | NM_014918.3 |
| CXADR | NM_001338.4 |
| CYP4V2 | NM_207352.3 |
| FAM44B | NM_138369.2 |
| HSPA12A | NM_025015.2 |
| LEF1 | NM_016269.4 |
| MT1A | NM_005946.2 |
| MTP18 | NM_016498.4 |
| MTSS1 | NM_014751.4 |
| MYC | NM_002467.4 |
| NAV2 | NM_145117.4 |
| PRMT6 | NM_018137.2 |
| SKP2 | NM_005983.3 |

The utility of the obtained 16 gene signature was verified on these 20 samples by cross validation with Support Vector Machine classification method. Using the described methods 20 out of 20 samples were classified correctly based on this 16 gene signature (See Figure 4A).

**Experimental Protocols**

**Cell culture and chemicals**

All cell culture medium was purchased from Invitrogen and different cell lines were purchased from ATCC. 293H, HepG2, U373MG, U105MG, MDA-MB415 and MDA-MB-231 cells were cultured in DMEM medium with 10% FBS, 1 mM sodium pyruvate and non-essential amino acid (Invitrogen). CCD1079SK, BJ, IMR90, MCF7 cells were cultured in MEM medium with 10% FBS. Lncap cells were cultured in RPMI 1640 medium with 10% FBS. HT29, HCT116, SW480 and SK-BR-3 were cultured in McCoy's 5A modified medium with 10% FBS. All cells were cultured in a cell culture incubator at 37 degrees C supplied with 5% CO2. All chemicals used in experiments were from Sigma unless indicated with other source.

**Protocol for cell lysis and western blot**

At the end of experimental treatment, cells were lysed in Modified RIPA buffer ( 150mM NaCl, 50mM TrisHC1, 1% IGEPAL, 0.5% sodium deoxycholate, 1mM EDTA, 1% Triton X-100 and 0.1% SDS with protease and phosphatase inhibitor) (all chemicals from Sigma). For each well in 6-well plate, cell culture medium was aspirated and washed with 1 ml PBS. 50 μl of Modified RIPA buffer was added to each well and cells were scrapped off wells in Modified RIPA buffer. Cell lysate was transferred to 1.5 ml microcentrifuge tube and incubated on ice for 30 min. After 15 minutes centrifuge at 15000 rpm at 4 degrees C, supernatant was transferred to a new 1.5 ml tube and protein concentration was measured with BCA protein assay according to manufacturer's standard protocol (Pierce). The cell lysate was diluted in 30 μl of H2O to 2 μg/μl protein concentration and mixed with 30 μl of 2x SDS sample buffer (BioRAD) to give a final concentration of 1 μgl/μl. The diluted lysate was heated at 70 degrees C for 10 minutes to denature the protein. The lysate was centrifuged at 15000 rpm for 1 minutes after heating and was loaded on a precast 4-12% NuPAGE Novex Bis-Tris Mini gel (Invitrogen) with 15 μl lysate for each well. The gel was run at a constant voltage of 150 V for 1.5 hours following transfer to a nitrocellulose membrane at a constant voltage of 30 V for 2 hours according to manufacturer's protocol (Invitrogen). The nitrocellulose membrane was blocked in 5% milk in western blot wash buffer (1xPBS plus 0.1% Tween-20) for 1 hours at room temperature. Separate membranes were further incubated with mouse anti-active β-catenin (1:1000) (Millipore), mouse anti-β-catenin (1:2000) (BD Transduction lab) and rabbit anti-GAPDH (1:2000) (ABcam) primary antibodies at 4 degrees C overnight. The next day, membranes were took out from 4 degrees C and further incubated at room temperature for 30 minutes following three times of wash in western blot wash buffer with 5 minutes for each wash. Membranes were incubated with goat anti-mouse (1:4000) and goat anti-rabbit (1:4000) secondary antibody (Sigma) for 1 hours at room temperature. Membranes were washed in western blot wash buffer 5 minutes for three times. To detect protein band on membranes, mixed western blot substrate (0.75ml peroxide solution mixed with 0.75 ml luminol enhancer solution) (Thermo SCIENTIFIC) was added to each membrane and incubated at room temperature for 1 minutes to cover the entire membrane. The membrane was exposed to Fuji image machine LAS-3000 (Fuji Film) for 2 minutes with chemiluminecence filter. The effect of Wnt3a treatment was demonstrated by increased protein levels of active β-catenin in Wnt3a treated samples compared to untreated samples. The effect of β-catenin siRNA was demonstrated by decreased protein levels of active and total β-catenin in β-catenin siRNA transfected samples compared to non target siRNA transfected samples.

**Protocol for total RNA isolation with SABiosciences RT$^2$ qPCR-Grade RNA Isolation Kit**

To harvest cells grown in 6-well plate for RNA isolation, cell culture medium was removed and 200 μl of lysis buffer G6 was added into each well. Cells were scrapped off plate and lysate was transferred to a 1.5 ml microcentrifuge tube for immediate RNA isolation or stored at -80 degrees C to isolate RNA later. To isolate RNA, one volume (200 μl) of 70% ethanol was added to the lysate and mixed 6 times by pipetting. The mixed sample was added to a spin column placed in a 2 ml collection tube and centrifuged for 1 minutes at 11,000 Xg. The column was washed with 350 μl of desalting buffer G15 by centrifuging for 1 minutes at 11,000xg. 100 μl of DNAse treatment solution (10 μl RNase-free DNase mixed with 90 μl of DNase Reaction buffer) was added into each spin column and incubated at room temperature for 15 min. Prewash buffer (G16, 200 μl) was added into spin column and centrifuged at 1 minutes at 11,000xg. Washing buffer G17 (600 μl) was added to the spin column and centrifuged for 1 minutes at 11,000xg to wash the spin column membrane. Another 250 μl Buffer G17 was added to the spin column and centrifuged for 3 minutes at 11,000xg to wash the spin column membrane. The spin column was placed in a new 1.5 ml collection tube and 30 μl RNase-free water was directly added to the spin column membrane. The spin column was sit at room temperature for 2 minutes and centrifuged for 1 minutes at 11,000xg to elute the RNA.

**Total RNA isolation with QIAGEN RNeasy Plus Mini Kit**

To harvest cells grown in 6-well plate for RNA isolation, cell culture medium was removed and 200 μl of RNeasy Plus buffer was added into each well. Cells were scrapped off plate and lysate was transferred to a 1.5 ml microcentrifuge tube for immediate RNA isolation or stored at -80 degrees C to isolate RNA later. To isolate RNA, transfer the homogenized lysate to a gDNA Eliminator spin column placed in a 2 ml collection tube. Centrifuge for 30 s at ≥8000 x g (≥10,000 rpm). Discard the column, and save the flowthrough. One volume (200 μl) of 70% ethanol was added to the flowthrough and mixed 6 times by pipetting. The mixed sample was added to an RNeasy spin column placed in a 2 ml collection tube and centrifuged for 1 minutes at ≥8000 x g (≥10,000 rpm). The column was washed with 700 μl of buffer RW1 by centrifuging for 1 minutes at ≥8000 x g (≥10,000 rpm). Buffer RPE (500 μl) was added to the RNeasy spin column and centrifuged for 1 minutes at ≥8000 x g (≥10,000 rpm) to wash the spin column membrane. Another 500 μl Buffer RPE was added to the RNeasy spin column and centrifuged for 2 minutes at ≥8000 x g (≥10,000 rpm) to wash the spin column membrane. The RNeasy spin column was placed in a new 2 ml collection tube and centrifuged at full speed for 1 min.

RNeasy spin column was transferred to a new 1.5 ml collection tube and 30 μl RNase-free water was directly added to the spin column membrane. The spin column was sit at room temperature for 2 minutes and centrifuged for 1 minutes at ≥8000 x g (≥10,000 rpm) to elute the RNA.

**Protocol for reverse transcription with RT$^2$ EZ First Strand Kit (SABiosciences, a QIAGEN company)**

Total RNA of 300-1000 ng was diluted with RNase-free H2O to 8 μl and mixed with 6 μl of GE2 (genomic DNA elimination) buffer. The reaction was incubated at 37 °C for 5 min, and immediately placed on ice for 1 minute. 6 μl of the BC5 (RT Master Mix) was added to each 14-μl Genomic DNA Elimination Mixture for a final volume of 20 μl. The reaction was incubated at 42 °C for exactly 15 minutes and then immediately stopped by heating at 95 °C for 5 minutes. Incubation at 37 °C, 42 °C and 95 °C was done on a thermal cycle GenAmp PCR System 2700 (Applied Systems). The finished reaction was put on ice until ready to use for real-time PCR, or placed at -20 °C for long-term storage.

**Protocol for reverse transcription with RT$^2$ First Strand Kit (SABiosciences, a QIAGEN company)**

Total RNA of 300-1000 ng was diluted with RNase-free H2O to 8 μl and mixed with 2 μl of GE (genomic DNA elimination) buffer. The reaction was incubated at 42 °C for 5 min, and immediately placed on ice for 1 minute. 10 μl of the RT cocktail (4 μl BC3, 1 μl P2, 2 μl of RE3 and 3 μl of H2O) was added to each 10-μl Genomic DNA Elimination Mixture for a final volume of 20 μl. The reaction was incubated at 42 °C for exactly 15 minutes and then immediately stop the reaction by heating at 95 °C for 5 minutes. Incubation at 42 °C and 95 °C was done on a thermal cycle GenAmp PCR System 2700 (Applied Systems). The finished reaction was put on ice until ready to use for real-time PCR, or placed at -20 °C for long-term storage.

**Protocol for Illumina Human HT-12 BeadChip**

Resuspended cRNA samples were dispensed onto BeadChips and incubated in the Illumina Hybridization Oven to hybridize the samples onto the BeadChips for 16-24 hours as follows. Hybridization was performed using HYB (10 μl), HCB (400 μl), and cRNA (750 ng in 5 μl; RNase-free water was added or samples were dried down as necessary to achieve this volume). The oven (with rocking platform) was preheated to 58°C. Samples were left at room temperature (~22°C) for 10 minutes to resuspend cRNA. The HYB and HCB tubes were placed in the 58°C oven for 10 minutes to dissolve any salts that may have precipitated in storage and if any salts remained undissolved, were incubated at 58°C for another 10 minutes. After cooling to room temperature, HYB and HCB tubes were mixed thoroughly before using. 10 μl HYB was added to each cRNA sample. The Illumina Hyb Chamber gaskets were placed into the BeadChip Hyb Chamber. 200 μl HCB was dispensed into the humidifying buffer reservoirs that were next to the loaded BeadChips. The Hyb Chamber was sealed with lid and kept on bench at room temperature until ready to load BeadChips into the Hyb Chamber. All BeadChips were removed from their packages. Holding the BeadChip by the coverseal tab with tweezers or with powder-free gloved hands, the BeadChip were slid into the Hyb Chamber insert so that the barcode lined up with barcode symbol on the insert. Assay sample were preheated at 65°C for 5 minutes, briefly vortexed, then briefly centrifuged to collect the liquid in the bottom of the tube, then allow to cool to room temperature before using. Sample were pipetted immediately after cooling to room temp. The Hyb Chamber inserts containing BeadChips were loaded into the Hyb Chamber. Assay samples (15 μl) were dispensed onto the large sample port of each array. The lid was sealed onto the Hyb Chamber carefully to avoid dislodging the Hyb Chamber insert(s) and incubated for 16-20 hours at 58°C with rocker speed at 5. BeadChips were removed from the overnight hybridization for washing and staining using High-Temp Wash Buffer (500 ml), Wash E1BC (2 L), Block E1 Buffer (6 ml/chip), Streptavidin-Cy3 stock (1 mg/ml in RNase-free water, 2μl per chip), and 100% Ethanol (250 ml). 1X High-Temp Wash buffer was prepared by adding 50 ml 10X stock to 450 ml RNase-free water. The waterbath insert was placed into the heat block, and 500 ml prepared 1X High-Temp Wash buffer was added. The heat block temp was set to 55°C and High-Temp Wash buffer was pre-warmed to that temperature. The heat block lid was closed and left overnight. The next day, The Wash E1BC solution was prepared by adding 6 ml E1BC buffer to 2 L RNase-free water. Block E1 buffer (4 ml/chip) was pre-warmed to room temperature. Block E1 buffer (2 ml/chip) was prepared with streptavidin-Cy3 (2 μl of 1 mg/ml stock per chip), using a a single conical tube for all BeadChips, and stored in the dark until detection step. 1 L of diluted Wash E1BC buffer was placed in a Pyrex No. 3140 beaker. The Hyb Chamber was removed from the oven and disassembled (all at one at a time). All BeadChips were processed in the first chamber as described in the following steps, then the second chamber was removed from the oven for processing all of its BeadChips, and so on until all chambers were processed. Using powder-free gloved hands, all BeadChips were removed from the Hyb Chamber and submerged face up at the bottom of the beaker, and the coverseal was removed from the first BeadChip, ensuring that the entire BeadChip remained submerged during removal. Using tweezers or powder-free gloved hands, each peeled BeadChip was transferred in turn into the slide rack submerged in the staining dish containing 250 ml Wash E1BC solution. The 250 ml Wash E1BC solution was saved to be used again in the 1st Room-Temp Wash below. The slide rack handles were then used to transfer the rack into the Hybex Waterbath insert containing High-Temp Wash buffer for the High-Temp Wash. Samples were incubated static for 10 minutes with the Hybex lid closed. After the 10-minute High-Temp Wash buffer incubation was complete, the slide rack was immediately transferred back into the staining dish containing the Wash E1BC used in the Seal Removal steps, and briefly agitated using rack, then shaken on an orbital shaker for 5 minutes at the highest speed possible without allowing solution to splash out of the dish. The rack was then transferred to a clean staining dish containing 250 ml 100% Ethanol (used fresh from the Ethanol source bottle) and briefly agitated using the rack handle, then shaken on the orbital shaker for 10 minutes. The rack was then transferred to a clean staining dish containing fresh 250 ml Wash E1BC solution and briefly agitated using rack handle, then shaken on orbital shaker for 2 minutes. 4 ml Block E1 buffer was then pipetted into the wash tray(s), and the BeadChip was transferred, face up, into BeadChip wash tray(s) on rocker and rocked at medium speed for 10 minutes. 2 ml Block E1 buffer + streptavidin-Cy3 was pipetted into fresh wash tray(s) and the BeadChip was transferred, face up, into wash tray(s) on the rocker, the cover was placed on the tray and rocked at medium speed for 10 minutes. 250 ml of Wash E1BC solution was added to a clean staining dish and the BeadChip was transferred to the slide rack submerged in the staining dish, briefly agitated using the rack, and then shaken at room temperature on an orbital shaker for 5 minutes. The centrifuge with then prepared plateholders, paper towels, and balance rack, and the speed was speed to 275 relative centrifugal force. The rack of BeadChips was centrifuged at room temperature for 4 minutes. If processing only one slide rack, BeadChips were redistributed between two racks, or counterbalanced with another rack loaded with an equal number of used BeadChips to maintain centrifuge balance. Dry chips were stored in slide box until scanned.

**Experimental data**

The data of the above-described experiments is contained in Figures 2A-C, 3A-B and 4A-B.

## Claims

1. A kit for effecting amplification analysis of a sample to detect the regulation status of Wnt/β-catenin signaling pathway in a cell sample or subject, wherein the kit comprises nucleic acids that amplify and provide for the detection of at least 5 of the genes selected from the group consisting of CALM1, CCND1, CCND2, CHSY1, CXADR, CYP4V2, FAM44B, HSPA12A, LEF1, MTP18, MYC, NAV2, SKP2, PRMT6, MTSS1 and MT1A or an ortholog or variant thereof.

2. The kit of claim 1 wherein the amplified genes are at least 90% identical or specifically hybridize to genes with Accession numbers selected from the group consisting of NM_006888.4, NM_053056.2, NM_001759.3, NM_014918.3, NM_001338.4, NM_207352.3, NM_138369.2, NM_025015.2, NM_016269.4, NM_016498.4, NM_002467.4, NM_145117.4, NM_005983.3, NM_018137.2, NM_014751.4, and NM_005946.2.

3. The kit of claim 1:

a) for effecting amplification analysis of a sample to detect the regulation status of Wnt/β-catenin signaling pathway in a cell sample or subject, wherein the kit comprises nucleic acids that amplify and provide for the detection of at least 10 of the genes selected from the group consisting of CALM1, CCND1, CCND2, CHSY1, CXADR, CYP4V2, FAM44B, HSPA12A, LEF1, MTP18, MYC, NAV2, SKP2, PRMT6, MTSS1 and MT1A;
b) which further comprises nucleic acids corresponding to from 1-10 housekeeping genes;
c) for effecting amplification analysis of a sample to detect the regulation status of Wnt/β-catenin signaling pathway in a cell sample or subject, wherein the kit comprises nucleic acids that amplify and provide for the detection of at least 10-15 of the genes selected from the group consisting of CALM1, CCND1, CCND2, CHSY1, CXADR, CYP4V2, FAM44B, HSPA12A, LEF1, MTP18, MYC, NAV2, SKP2, PRMT6, MTSS1 and MT1A;
d) for effecting amplification analysis of a sample to detect the regulation status of Wnt/β-catenin signaling pathway in a cell sample or subject, wherein the kit comprises nucleic acids that amplify and provide for the detection of at least 10-15 of the genes selected from the group consisting of CALM1, CCND1, CCND2, CHSY1, CXADR, CYP4V2, FAM44B, HSPA12A, LEF1, MTP18, MYC, NAV2, SKP2, PRMT6, MTSS1 and MT1A;
e) which is for effecting real-time PCR amplification;
f) which is for effecting real-time PCR amplification with detection by a SYBR Green method;
g) which is for effecting amplification by a method comprising: polymerase chain reaction (PCR), strand displacement amplification (SDA), loop-mediated isothermal amplification (LAMP), rolling circle amplification (RCA), transcription-mediated amplification (TMA), self-sustained sequence replication (3SR), nucleic acid sequence based amplification (NASBA), or reverse transcription polymerase chain reaction (RT-PCR); or
h) which is for effecting detection by a SYBR Green or MNAzyme method.

4. A method of using a kit according to any one of claims 1 - 3 to determine the regulation status of Wnt/β-catenin signaling pathway in a cell sample or subject.

5. The method of claim 4 wherein after running the amplification, the data is analyzed using a web based analysis tool.

6. The method of claim 5 wherein this tool provides a number that is used to determine the regulation status of Wnt/β-catenin signaling pathway in the target as compared to a control sample.

7. A method of determining the regulation status of Wnt/β-catenin signaling pathway in a cell sample or subject comprising detecting and comparing the expression of at least 5 of the genes selected from the group consisting of CALM1, CCND1, CCND2, CHSY1, CXADR, CYP4V2, FAM44B, HSPA12A, LEF1, MTP18, MYC, NAV2, SKP2, PRMT6, MTSS1 and MT1A to the expression of the same genes by a control cell sample and based on this comparison determining the regulation status of the Wnt/β-catenin signaling pathway in a cell sample or subject.

8. The method of claim 7 wherein gene expression is assayed by real time amplification.

9. The method of claim 8 wherein the detection method comprises SYBR Green based real-time PCR.

10. The method of claim 7 wherein the gene expression data is analyzed by a support vector machine method.

11. The method of claim 10 wherein the expression value of all 16 genes plus house keeping genes is measured on control and treatment sample and the ΔΔCt is calculated and the ΔΔCt value of those 16 genes is compared to ΔΔCt value of these 16 genes in a data pool that contains negatively regulated and positively regulated samples in terms of pathway activity.

**12.** The method of claim 7:

a) wherein a cell sample is obtained from a patient that is potentially to be treated with a compound that modulates Wnt/β-catenin signaling and the method is used to assess the treatment protocol;
b) wherein a cell sample is obtained from a patient that has been treated with a compound that modulates Wnt/β-catenin signaling and the method is used to assess the efficacy of the treatment protocol;
c) which is used to evaluate Wnt/β-catenin pathway deregulation status in a sample;
d) which is used to classify a cell sample as having a deregulated or regulated Wnt/β-catenin signaling pathway; or
e) which is used to determine whether an agent modulates the Wnt/β-catenin signaling pathway in sample.

**13.** The method of claim 7 which is used to predict the response of a subject to an agent that modulates the Wnt/β-catenin signaling pathway.

**14.** The method of claim 13 which is used to assign treatment to a subject.

**15.** The method of claim 7:

a) which is used to evaluate the pharmacodynamic effects of therapies designed to regulate Wnt/β-catenin pathway signaling;
b) which is used to identify a cancer **characterized by** Wnt/β-catenin pathway dysregulation; or
c) wherein the cancer is selected from colorectal carcinomas, melanomas, breast cancer and hepatocellular carcinomas.

**Patentansprüche**

**1.** Kit zum Durchführen einer Amplifikationsanalyse einer Probe zum Nachweisen des Regulationsstatus eines Wnt/β-Catein-Signalweges in einer Zellprobe oder einer Testperson, worin der Kit Nukleinsäuren umfasst, die mindestens 5 der Gene, ausgewählt aus der Gruppe, bestehend aus CALM1, CCND1, CCND2, CHSY1, CXADR, CYP4V2, FAM44B, HSPA12A, LEF1, MTP18, MYC, NAV2, SKP2, PRMT6, MTSS1 und MT1 A oder einem Orthologen oder einer Variante davon, amplifizieren und deren Nachweis bereitstellen.

**2.** Kit nach Anspruch 1, worin die amplifizierten Gene zu mindestens 90 % identisch sind mit oder spezifisch hybridisieren an Gene mit Hinterlegungsnummern, ausgewählt aus der Gruppe, bestehend aus NM_006888.4, NM_053056.2, NM_001759.3, NM_014918.3, NM_001338.4, NM 207352.3, NM 138369.2, NM 025015.2, NM 016269.4, NM 016498.4, NM_002467.4, NM_145117.4, NM_005983.3, NM_018137.2, NM_014751.4, und NM_005946.2.

**3.** Kit nach Anspruch 1,

a) zum Durchführen einer Amplifikationsanalyse einer Probe zum Nachweisen des Regulationsstatus eines Wnt/β-Catenin-Signalwegs in einer Zellprobe oder einer Testperson, worin der Kit Nukleinsäuren umfasst, die mindestens 10 der Gene, ausgewählt aus der Gruppe, bestehend aus CALM1, CCND1, CCND2, CHSY1, CXADR, CYP4V2, FAM44B, HSPA12A, LEF1, MTP18, MYC, NAV2, SKP2, PRMT6, MTSS1 und MT1 A, amplifizieren und deren Nachweis bereitstellen;
b) weiterhin umfassend Nukleinsäuren, die von 1-10 Haushaltsgenen entsprechen;
c) zum Durchführen einer Amplifikationsanalyse einer Probe zum Nachweisen des Regulationsstatus eines Wnt/β-Catenin-Signalwegs in einer Zellprobe oder einer Testperson, worin der Kit Nukleinsäuren umfasst, die mindestens 10-15 der Gene, ausgewählt aus der Gruppe, bestehend aus CALM1, CCND1, CCND2, CHSY1, CXADR, CYP4V2, FAM44B, HSPA12A, LEF1, MTP18, MYC, NAV2, SKP2, PRMT6, MTSS1 und MT1 A, amplifizieren und deren Nachweis bereitstellen;
d) zum Durchführen einer Amplifikationsanalyse einer Probe zum Nachweisen des Regulationsstatus eines Wnt/β-Catenin-Signalwegs in einer Zellprobe oder einer Testperson, worin der Kit Nukleinsäuren umfasst, die mindestens 10-15 der Gene, ausgewählt aus der Gruppe, bestehend aus CALM1, CCND1, CCND2, CHSY1, CXADR, CYP4V2, FAM44B, HSPA12A, LEF1, MTP18, MYC, NAV2, SKP2, PRMT6, MTSS1 and MT1 A amplifizieren und deren Nachweis bereitstellen;

e) zum Durchführen von Echtzeit-PCR-Amplifikation;
f) der geeignet ist zum Durchführen einer Echtzeit-PCR-Amplifikation mit Nachweis durch ein SYBR-Green-Verfahren;
g) zum Durchführen eines Verfahrens, umfassend:

Polymerasekettenreaktion (PCR), Strang-Displacement-Amplifikation (SDA), Schleifen-vermittelte isothermale Amplifikation (LAMP), Rolling-Circle-Amplifikation (RCA), Transkriptionsvermittelte Amplifikation (TMA), Selbstunterhaltende Sequenzreplikation (Self-sustained-Sequenzreplikation) (3SR), Nukleinsäuresequenzbasierende Amplifikation (NASBA) oder Reverse Transkription-Polymerase-Kettenreaktion (RT-PCR); oder

h) zum Durchführen eines Nachweises durch ein SYBR Green- oder MNAzym-Verfahren.

**4.** Verfahren zur Verwendung eines Kits nach einem der Ansprüche 1 - 3, zum Bestimmen des Regulationsstatus des Wnt/β-Catenin-Signalwegs in einer Zellprobe oder einer Testperson.

**5.** Verfahren nach Anspruch 4, worin nach Durchführen der Amplifikation die Daten unter Verwendung eines webbasierten Analysewerkzeugs analysiert werden.

**6.** Verfahren nach Anspruch 5, worin dieses Werkzeug Zahl bereitstellt, die verwendet wird um den Regulationsstatus des Wnt/β-Catenin-Signalwegs in dem Ziel im Vergleich zu einer Kontrollprobe zu bestimmen.

**7.** Verfahren zum Bestimmen des Regulationsstatus des Wnt/β-Catenin-Signalwegs in einer Zellprobe oder einer Testperson, umfassend das Nachweisen und Vergleichen der Expression von mindestens 5 der Gene, ausgewählt aus der Gruppe, bestehend aus CALM1, CCND1, CCND2, CHSY1, CXADR, CYP4V2, FAM44B, HSPA12A, LEF1, MTP18, MYC, NAV2, SKP2, PRMT6, MTSS1 und MT1 A mit der Expression der gleichen Gene durch eine Kontrollzellprobe und basierend auf diesem Vergleich Bestimmung des Regulationsstatus des Wnt/β-Catenin-Signalwegs in einer Zellprobe oder einer Testprobe.

**8.** Verfahren nach Anspruch 7, worin Genexpression durch Echtzeit-Amplifikation untersucht wird.

**9.** Verfahren nach Anspruch 8, worin das Nachweisverfahren SYBR Greenbasierende Echtzeit-PCR umfasst.

**10.** Verfahren nach Anspruch 7, worin die Genexpressiondaten durch ein Support-Vektor-Maschinen-Verfahren analysiert werden.

**11.** Verfahren nach Anspruch 10, worin der Expressionswert aller 16 Gene plus Haushaltsgene von Kontrolle und Behandlungsprobe gemessen wird und das ΔΔCt berechnet wird und der ΔΔCt-Wert dieser 16 Gene verglichen wird mit dem ΔΔCt-Wert dieser 16 Gene in einem Datenpool, der negativ regulierte und positiv regulierte Proben bezüglich Signalwegaktivität enthält.

**12.** Verfahren nach Anspruch 7:

a) worin eine Zellprobe erhalten wird von einem Patienten, der potentiell zu behandeln ist mit einer Verbindung, die Wnt/β-Catenin-Signalisierung moduliert, und wobei das Verfahren verwendet wird, um das Behandlungsprotokoll zu bewerten;
b) worin eine Zellprobe erhalten wird von einem Patienten, der behandelt worden ist mit einer Verbindung, die Wnt/β-Catenin-Signalisierung moduliert, und wobei das Verfahren verwendet wird, um die Wirksamkeit des Behandlungsprotokolls zu bewerten;
c) das verwendet wird, zum beurteilen des Wnt/β-Catenin-Signalweg-Deregulationsstatus in einer Probe.
d) das verwendet wird zum Klassifizieren einer Zellprobe, dass sie einen deregulierten oder regulierten Wnt/β-Catenin-Signalweg aufweist; oder
e) das verwendet wird, um zu bestimmen, ob ein Mittel den Wnt/β-Catenin-Signalweg in einer Probe moduliert.

**13.** Verfahren nach Anspruch 7, das verwendet wird, um die Reaktion einer Testperson auf ein Mittel vorherzusagen, das den Wnt/β-Catenin-Signalweg moduliert.

**14.** Verfahren nach Anspruch 13, das verwendet wird, um die Behandlung einer Testperson zuzuweisen.

15. Verfahren nach Anspruch 7:

a) das verwendet wird zum Beurteilen der pharmakodynamischen Wirkungen von Therapien, die vorgesehen sind zum Regulieren der Wnt/β-Catenin-Wegsignalisierung;
b) das verwendet wird zum Identifizieren von Krebs, der **gekennzeichnet ist durch** Wnt/β-Catenin-Signalweg-Dysregulation; oder
c) worin der Krebs ausgewählt ist aus kolorektalem Karzinom, Melanomen, Brustkrebs und hepatozellulären Karzinomen.

## Revendications

1. Kit pour effectuer une analyse d'amplification d'un échantillon afin de détecter l'état de régulation de la voie de signalisation Wnt/β-caténine dans un échantillon de cellules ou chez un sujet, dans lequel le kit comprend les acides nucléiques qui amplifient et assurent la détection d'au moins 5 des gènes choisis dans le groupe consistant en CALM1, CCND1, CCND2, CHSY1, CXADR, CYP4V2, FAM44B, HSPA12A, LEF1, MTP18, MYC, NAV2, SKP2, PRMT6, MTSS1 et MT1A ou l'un de leurs orthologues ou de leurs variants.

2. Kit de la revendication 1, dans lequel les gènes amplifiés sont identiques à au moins 90% ou s'hybrident spécifiquement à des gènes ayant des numéros d'accès choisis dans le groupe consistant en NM_006888.4, NM_053056.2, NM_001759.3, NM_014918.3, NM_001338.4, NM_207352.3, NM_138369.2, NM_025015.2, NM_016269.4, NM_016498.4, NM_002467.4, NM_145117.4, NM_005983.3, NM_018137.2, NM_014751.4 et NM_005946.2.

3. Kit de la revendication 1 :

a) pour effectuer une analyse d'amplification d'un échantillon afin de détecter l'état de régulation de la voie de signalisation Wnt/β-caténine dans un échantillon de cellules ou chez un sujet, dans lequel le kit comprend des acides nucléiques qui amplifient et assurent la détection d'au moins 10 des gènes choisis dans le groupe consistant en CALM1, CCND1, CCND2, CHSY1, CXADR, CYP4V2, FAM44B, HSPA12A, LEF1, MTP18, MYC, NAV2, SKP2, PRMT6, MTSS1 et MT1A,
b) qui comprend en outre des acides nucléiques correspondant à 1 à 10 gène(s) domestique(s) ;
c) pour effectuer une analyse d'amplification d'un échantillon afin de détecter l'état de régulation de la voie de signalisation Wnt/β-caténine dans un échantillon de cellules ou chez un sujet, dans lequel le kit comprend des acides nucléiques qui amplifient et assurent la détection d'au moins 10 à 15 des gènes choisis dans le groupe consistant en CALM1, CCND1, CCND2, CHSY1, CXADR, CYP4V2, FAM44B, HSPA12A, LEF1, MTP18, MYC, NAV2, SKP2, PRMT6, MTSS1 et MT1A,
d) pour effectuer une analyse d'amplification d'un échantillon afin de détecter l'état de régulation de la voie de signalisation Wnt/β-caténine dans un échantillon de cellules ou chez un sujet, dans lequel le kit comprend des acides nucléiques qui amplifient et assurent la détection d'au moins 10 à 15 des gènes choisis dans le groupe consistant en CALM1, CCND1, CCND2, CHSY1, CXADR, CYP4V2, FAM44B, HSPA12A, LEF1, MTP18, MYC, NAV2, SKP2, PRMT6, MTSS1 et MT1A,
e) qui est destiné à effectuer une amplification par PCR en temps réel ;
f) qui est destiné à effectuer une amplification par PCR en temps réel avec une détection par la méthode SYBR Green ;
g) qui est destiné à effectuer une amplification par un procédé comprenant : la réaction en chaîne par polymérase (PCR), l'amplification par déplacement de brin (SDA), l'amplification isotherme à médiation par boucle (LAMP), l'amplification en cercle roulant (RCA), l'amplification à médiation par transcription (TMA), la réplication de séquences auto-entretenues (3SR), l'amplification à base de séquences d'acides nucléiques (NASBA), ou la réaction en chaîne par polymérase par transcription inverse (RT-PCR), ou
h) qui est destiné à effectuer une détection par la méthode SYBR Green ou MNAzyme.

4. Procédé d'utilisation d'un kit selon l'une quelconque des revendications 1 à 3 pour déterminer l'état de régulation de la voie de signalisation Wnt/β-caténine dans un échantillon de cellules ou chez un sujet.

5. Procédé de la revendication 4, dans lequel, après la réalisation de l'amplification, les données sont analysées en utilisant un outil d'analyse basé sur le Web.

6. Procédé de la revendication 5, dans lequel cet outil fournit un nombre qui est utilisé pour déterminer l'état de régulation de la voie de signalisation Wnt/β-caténine dans la cible par rapport à un échantillon témoin.

**7.** Procédé de détermination de l'état de régulation de la voie de signalisation Wnt/β-caténine dans un échantillon de cellules ou chez un sujet comprenant le fait de détecter et de comparer l'expression d'au moins 5 des gènes choisis dans le groupe consistant en CALM1, CCND1, CCND2, CHSY1, CXADR, CYP4V2, FAM44B, HSPA12A, LEF1, MTP18, MYC, NAV2, SKP2, PRMT6, MTSS1 et MT1A à l'expression des mêmes gènes par un échantillon de cellules témoin et de déterminer, sur la base de cette comparaison, l'état de régulation de la voie de signalisation Wnt/β-caténine dans un échantillon de cellules ou chez un sujet.

**8.** Procédé de la revendication 7, dans lequel l'expression de gène est testée par une amplification en temps réel.

**9.** Procédé de la revendication 8, dans lequel le procédé de détection comprend une PCR en temps réel basée sur la méthode SYBR Green.

**10.** Procédé de la revendication 7, dans lequel les données d'expression de gène sont analysées par un procédé de machines à vecteur de support.

**11.** Procédé de la revendication 10, dans lequel la valeur d'expression de tous les 16 gènes plus les gènes domestiques est mesurée sur les échantillons témoin et de traitement et la ΔΔCt est calculée et la valeur de ΔΔCt de ces 16 gènes est comparée à la valeur de ΔΔCt de ces 16 gènes dans un ensemble de données qui contient des échantillons régulés négativement et régulés positivement en termes d'activité de voie.

**12.** Procédé de la revendication 7 :

a) dans lequel un échantillon de cellules est obtenu à partir d'un patient qui doit être traité potentiellement par un composé qui module la signalisation Wnt/β-caténine et le procédé est utilisé pour évaluer le protocole de traitement ;
b) dans lequel un échantillon de cellules est obtenu à partir d'un patient qui a été traité par un composé qui module la signalisation Wnt/β-caténine et le procédé est utilisé pour évaluer l'efficacité du protocole de traitement ;
c) qui est utilisé pour évaluer l'état de dérégulation de la voie Wnt/β-caténine dans un échantillon ;
d) qui est utilisé pour classer un échantillon de cellules comme ayant une voie de signalisation Wnt/β-caténine dérégulée ou régulée ; ou
e) qui est utilisé pour déterminer si un agent module la voie de signalisation Wnt/β-caténine dans l'échantillon.

**13.** Procédé de la revendication 7 qui est utilisé pour prévoir la réponse d'un sujet à un agent qui module la voie de signalisation Wnt/β-caténine.

**14.** Procédé de la revendication 13 qui est utilisé pour attribuer un traitement à un sujet.

**15.** Procédé de la revendication 7 :

a) qui est utilisé pour évaluer les effets pharmacodynamiques de thérapies conçues pour réguler la signalisation de la voie Wnt/β-caténine ;
b) qui est utilisé pour identifier un cancer **caractérisé par** un dérèglement de la voie Wnt/β-caténine ; ou
c) dans lequel le cancer est choisi entre les carcinomes colorectaux, les mélanomes, le cancer du sein et les carcinomes hépatocellulaires.

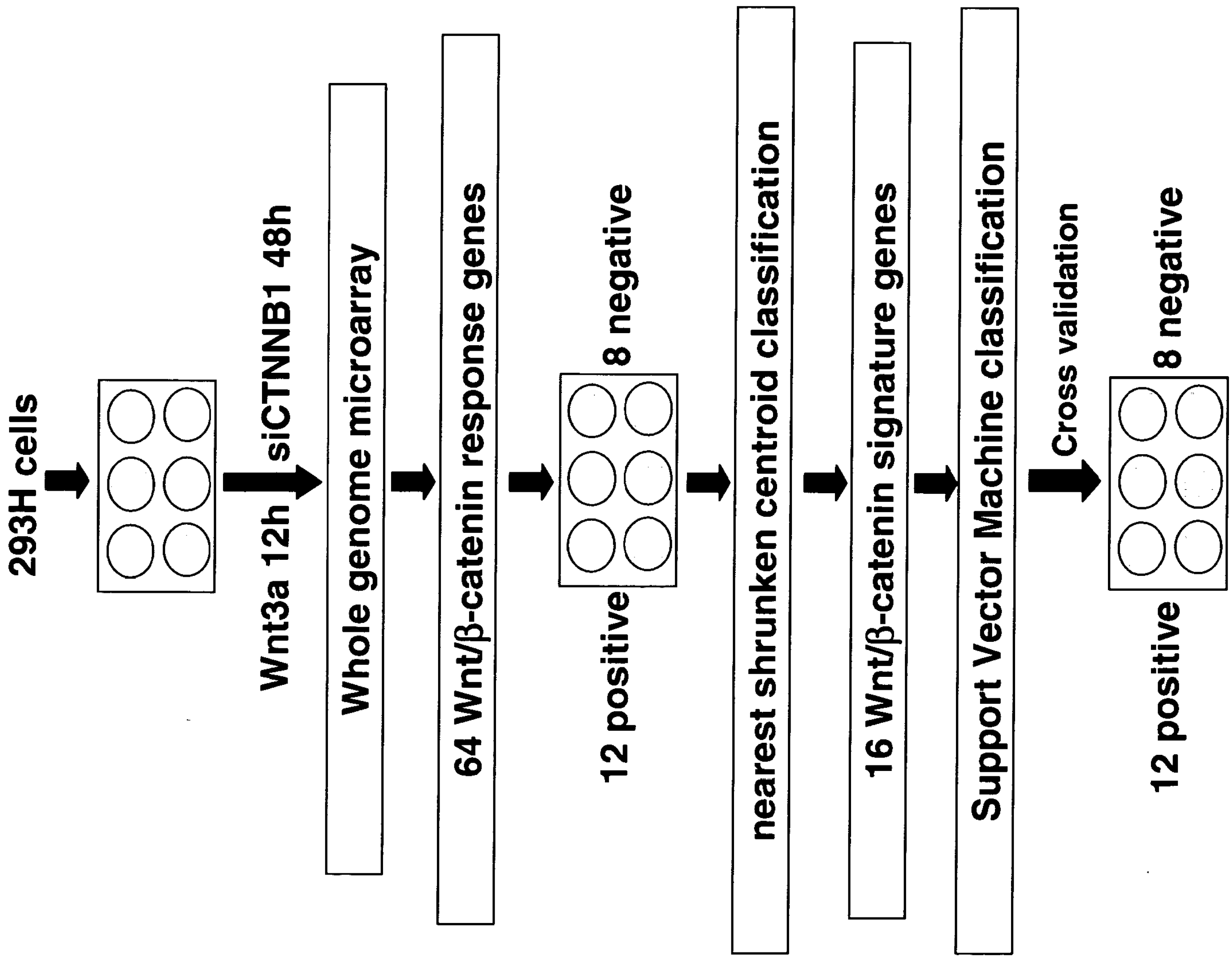
293H cells
Wnt3a 12h
siCTNNB1 48h
Whole genome microarray
64 Wnt/β-catenin response genes
12 positive
8 negative
nearest shrunken centroid classification
16 Wnt/β-catenin signature genes
Support Vector Machine classification
Cross validation
12 positive
8 negative

Figure 1

Figure 2

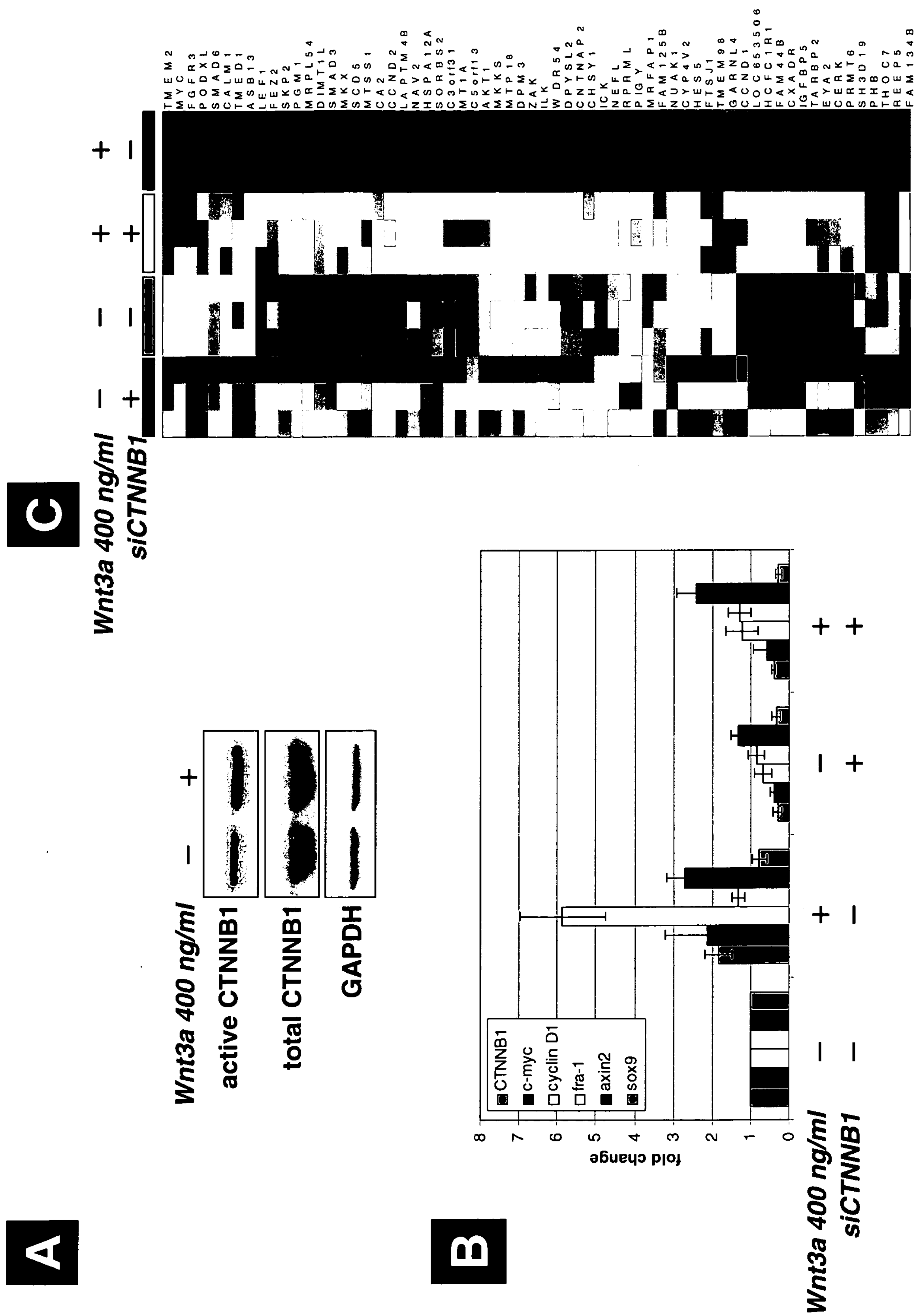
A
B
C
Wnt3a 400 ng/ml
active CTNNB1
total CTNNB1
GAPDH
siCTNNB1
fold change
CTNNB1
c-myc
cyclin D1
fra-1
axin2
sox9

Figure 3

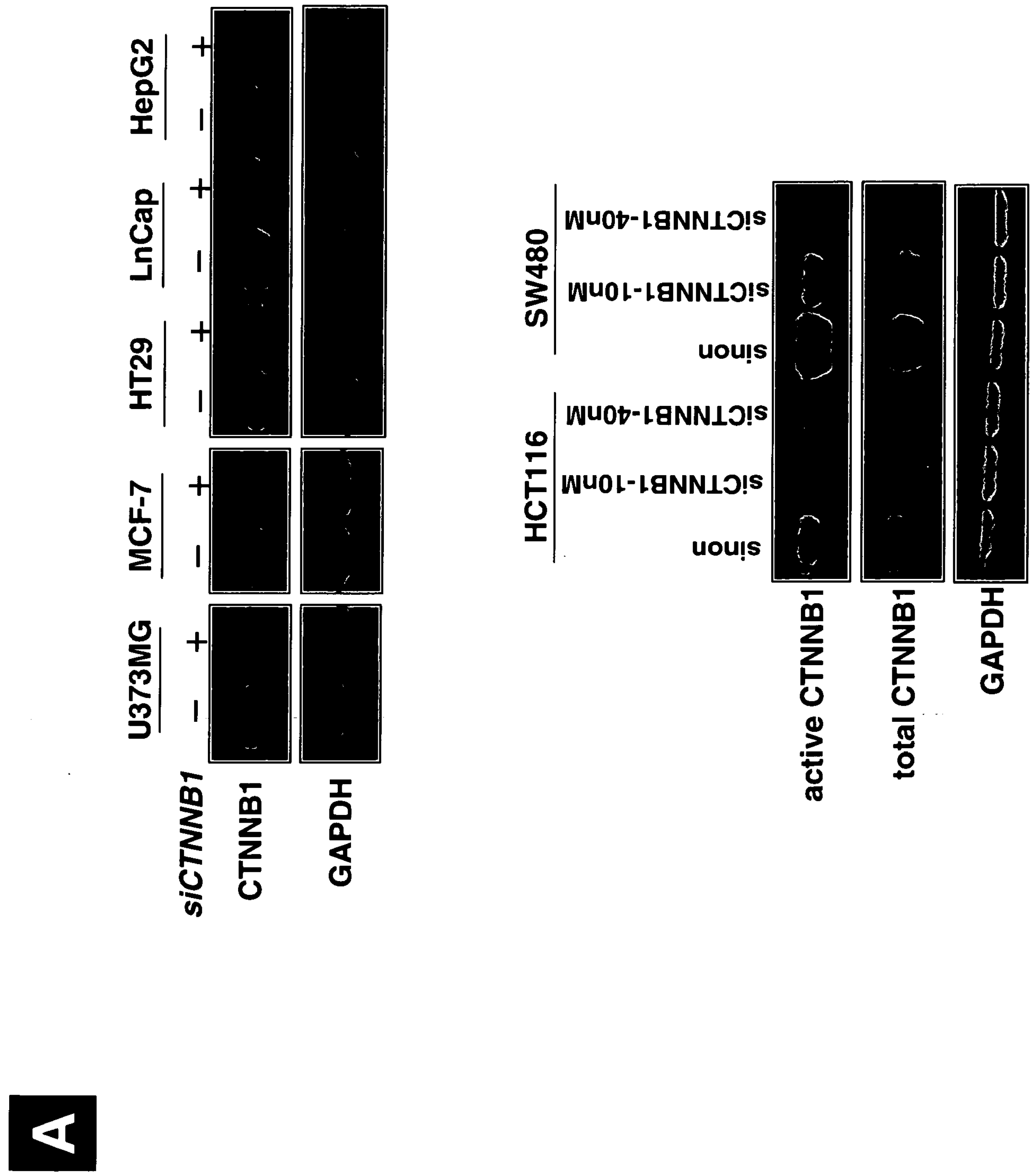
A
siCTNNB1
CTNNB1
GAPDH
U373MG
MCF-7
HT29
LnCap
HepG2
- +
HCT116
SW480
sinon
siCTNNB1-10nM
siCTNNB1-40nM
active CTNNB1
total CTNNB1
GAPDH

Figure 3

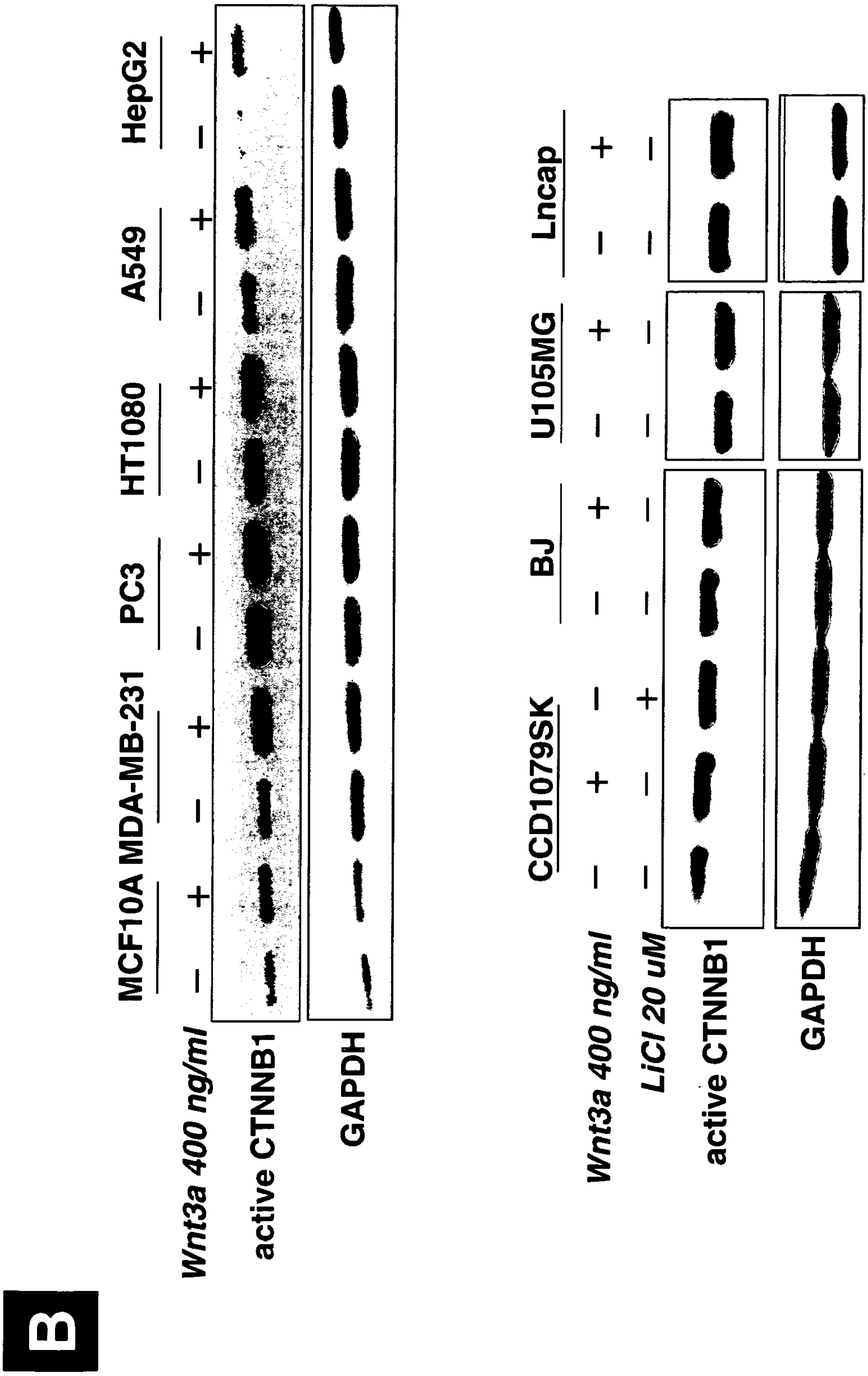
B
MCF10A MDA-MB-231 PC3 HT1080 A549 HepG2
Wnt3a 400 ng/ml
active CTNNB1
GAPDH
CCD1079SK BJ U105MG Lncap
Wnt3a 400 ng/ml
LiCl 20 uM
active CTNNB1
GAPDH

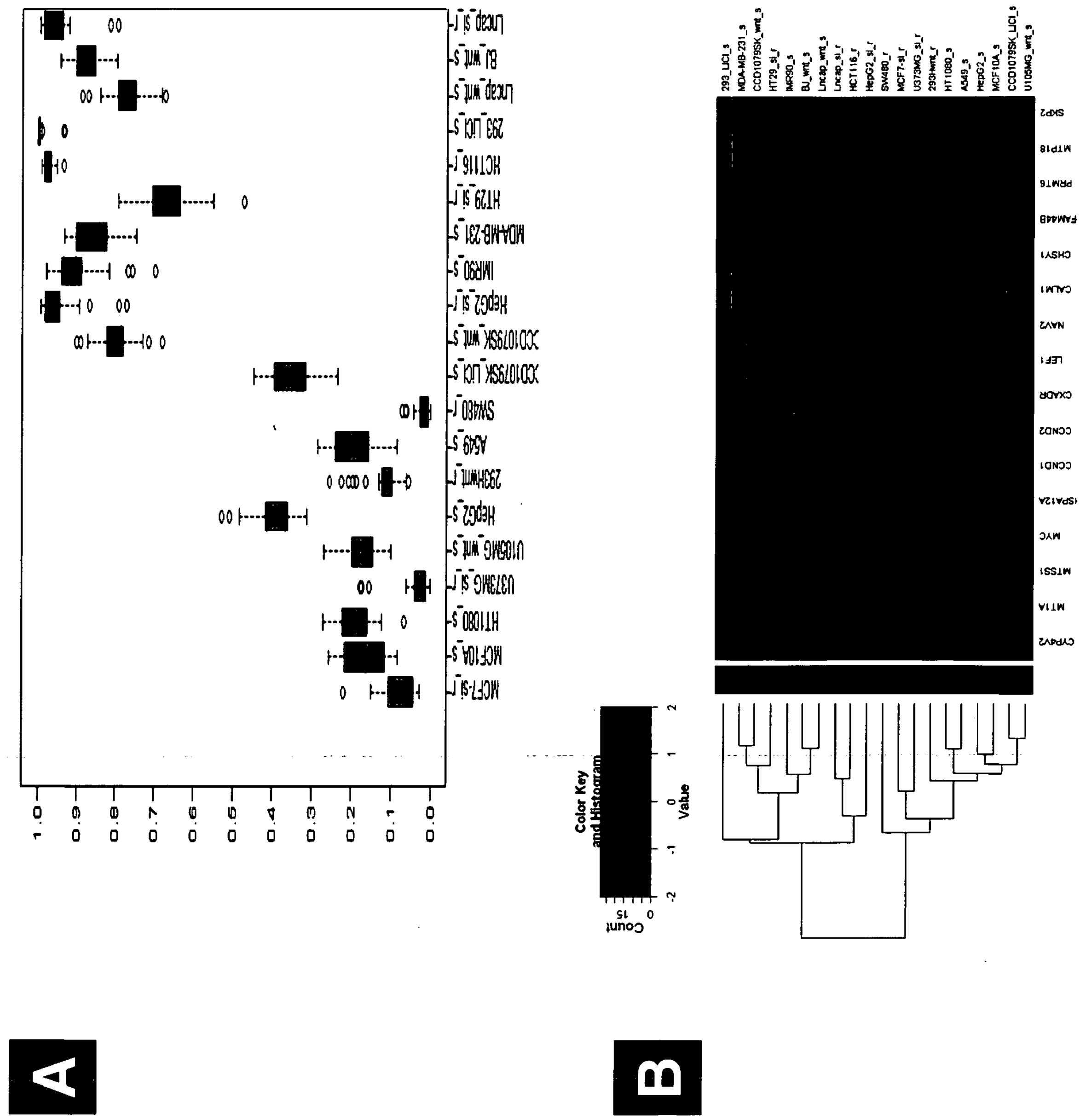


Figure 4

Figure 5A

| No | Symbol | REFSEQ_ID | seq_5 | seq_3 |
|---|---|---|---|---|
| 1 | TMEM2 | NM_013390 | AGCTATTTACCAGTCCTGCTG | GGTCTCTTCTGCGGACAGTG |
| 2 | MYC | NM_002467 | ACAGCTACGGAACTCTTGTGC | AAGGTTGTGAGGTTGCATTTG |
| 3 | FGFR3 | NM_022965 | CTGGCACTCTTGTTCCCAC | ACCTTCTCGGTTGTCAATAAG |
| 4 | PODXL | NM_001018111 | AGAATCTGACACCTTGCCTTG | AACTGTCTTTGCTACAACCTG |
| 5 | SMAD6 | NM_005585 | ATTAAGGATTCCCAGCAGCTC | AAGGCAGGCTTGTTGATAC |
| 6 | CALM1 | NM_006888 | GACCTTCCTGTAAACTCTTGC | AAGCCCATCTGAAATATCGAG |
| 7 | TMED1 | NM_006858 | CATGAGTCTCCTTCCGTCCTC | GTTTCCTTGTTAGGCCCTGC |
| 8 | ASB13 | NM_024701 | TGGATCAGTCAGTGTTTCTGC | TGCGCTAAGTGCTCTACTGTG |
| 9 | LEF1 | NM_016269 | CCTGTAACACATAGTGGCTTC | CTGCAAACCAGTCTGCTGAAC |
| 10 | FEZ2 | NM_005102 | TGAAACAGAATAAACGGGCTG | CACAGACACATGAAGCAGAAC |
| 11 | PGM1 | NM_002633 | ATGATGGTGCAAGTTCCTTTC | ATCTGTAGCCAAGTACGTCTC |
| 12 | MRPL54 | NM_172251 | CGACGTATGCACAGATCCTG | GTACTCAGCATCCGGTTTC |
| 13 | DIMT1L | NM_014473 | AGAATTTGCCCTCCGACTG | TTCATTAGATGGTCCACACG |
| 14 | SMAD3 | NM_005902 | TCTAGGAGCATGAAGTTTGAG | TGTTACATACGCCCAAAGCAC |
| 15 | MKX | NM_173576 | CTTGGGAAGACCTGAAACTTG | ACCACTGGCTGCACTATTGAC |
| 16 | SCD5 | NM_001037582 | ATATTGGGTGGCTGTTTGTTC | GAGTGAGATGGTATAGCGGAG |
| 17 | MTSS1 | NM_014751 | AGAGTAACGTACCGTATGGTC | ATCAAGATTGGTGGGCTAAAC |
| 18 | CA2 | NM_000067 | TTGGTGCTTTGTTTATGGTAG | TATCTTGTGCTATGGATTTGC |
| 19 | CCND2 | NM_001759 | CTATTGGTGTGGACTCAGAGC | AATGTAGTATCGTTGTGCCTG |
| 20 | LAPTM4B | NM_018407 | TGTTCTTGTGGATCTTGTGTC | CCTTGAACCAATCTAGGGCAG |
| 21 | NAV2 | NM_145117 | TTAGGGCTCTATCGGAAATGC | TTATGGCCTGTATCATTCTTG |
| 22 | HSPA12A | NM_025015 | TCATCTGCTTGAAATATGGAC | TTGCTTTATTAGGCGTCTGTG |
| 23 | SORBS2 | NM_003603 | GTCTTTATCCTGCGACAACAC | ATGAAGCTGGTTATGAACTTG |
| 24 | C3orf31 | NM_138807 | ATCTCAGTGAACGAGGATGTC | AATAGGAGAGACCGGCAATC |
| 25 | MT1A | NM_005946 | GGCATCAGAGAAGTGCAGC | ACAGTAAATGGGTCAGGGTTG |
| 26 | C5orf13 | NM_004772 | TGTGGCATTTATGTAGAGTTG | CAGCTTGCGTATTTATTGAAC |
| 27 | AKT1 | NM_005163 | AGTACATCAAGACCTGGCGG | CTCACGTTGGTCCACATCC |
| 28 | MKKS | NM_170784 | AATAGCCAGGAAGAACTCAAC | AGTCAAACAGTCCAAGGTCAG |
| 29 | MTP18 | NM_016498 | AGATGCTGAGTGGAAGTGGAC | CCTGAGTTTCTCCCTGAGTTG |
| 30 | DPM3 | NM_153741 | ATGACGAAATTAGCGCAGTG | CTCGGGCCTCCTGTATCTG |
| 31 | ILK | NM_004517 | CTCGCATCCAAATGTGCTC | ATCCATACGGCATCCAGTG |
| 32 | WDR54 | NM_032118 | ATTCCTCAGGCAACTCCTTTG | GGTGGAGCGGGTACTTTATG |

Figure 5B

| No | Symbol | REFSEQ_ID | seq_5 | seq_3 |
|---|---|---|---|---|
| 33 | DPYSL2 | NM_001386 | TTGCTGACTTGTGTTGCATTG | GGTGGTGATGGAATGACCTAC |
| 34 | CNTNAP2 | NM_014141 | TCACCACATTATACCCTGCTC | TATCTTTGGCTGGCTTACTTG |
| 35 | CHSY1 | NM_014918 | ATGTGGCTGTTCTCGTGGTAG | ATGTGTGTGTGTTTCAGTCTG |
| 36 | ICK | NM_016513 | AGATAAAGCCAAAGAGTAGGAG | CCAAGTCATCCAAGTCAGC |
| 37 | NEFL | NM_006158 | GGCTCTCAGTGTATTGGCTTC | GCATCCAGCTATTTCCATTTG |
| 38 | RPRML | NM_203400 | CTTCGGCGTCTTCTTCCTG | AGATGGCGCTCAGTACAGC |
| 39 | PIGY | NM_001042616 | TGTACTCTTGTTCTGCCCTTG | TATTCCAACATCACTCCTTTG |
| 40 | MRFAP1 | NM_033296 | AGAAAGGACCAGACTTCAACG | CCAAACAAACTCACCAGACAG |
| 41 | FAM125B | NM_033446 | AGTCCACCATGCCTGAAGTC | TACGTCATAGCCTGTCGGG |
| 42 | NUAK1 | NM_014840 | CCTGGGTGTGTTGCTTTAC | GTACTCTCCGCTGCTGATTTG |
| 43 | CYP4V2 | NM_207352 | AATAAACGCAGGGCCTTTC | GAAGGTGTCAACTTCTTCTCG |
| 44 | HES5 | NM_001010926 | CTCAGAGAATGTGTGTGCAG | CTGAAGAAAGTCCTCTACAGG |
| 45 | FTSJ1 | NM_012280 | AACAACCCTGAAGACAACAAG | GTTAAGAATCCCTGCCTTTAG |
| 46 | TMEM98 | NM_001033504 | TGAGCCACCGTCTAAGAAATC | GGCCAGGTGAGAGAGTACAAG |
| 47 | GARNL4 | NM_015085 | GTGACCCACGGACAGACAG | GTCGGTAAATGGCAGCTTTG |
| 48 | CCND1 | NM_053056 | TTGCTGCTATTGGAGGATCAG | AAGCATGAGGTATTGTGAAAC |
| 49 | LOC653506 | XM_927769 | GAGCTTCCAGCCAAGGATG | AATAACAACTCTGAATGGCAC |
| 50 | HCFC1R1 | NM_001002018 | CTGAGTGCTGGTGGACAGTG | CTCTGTTGAGGGAAGGTCTTG |
| 51 | FAM44B | NM_138369 | TGGGAGTAGTCTTTGACAGTG | CCTTATGCCTAATGTCTGAAC |
| 52 | CXADR | NM_001338 | CAGTGCTTTATGTTGTTGTTG | AGCTTGATTTGCTGGAACTAC |
| 53 | IGFBP5 | NM_000599 | ATGGGAACCTCAGAGAAGAGC | AAGTGGAGAAACAGTGCAATG |
| 54 | TARBP2 | NM_134323 | AGTACCTCAAGATCATGGCAG | GGTAGAGATGGCACCCACAG |
| 55 | EYA2 | NM_172112 | CTTAGTCGTTGGTTTGGTTTG | CAAGATGTAGCGTAAGGCACG |
| 56 | CERK | NM_022766 | GAGTGGCAAGTCGTCTGTG | AATTGAACCTGGAGCATTTC |
| 57 | PRMT6 | NM_018137 | GAGGAGGTTTCTGAGCCTAGC | CACAACTTTCACATTTGGCTG |
| 58 | SH3D19 | NM_001009555 | CTCACACACAGAAGTTGCACG | CTGGTAATAAGGCTGATATGC |
| 59 | PHB | NM_002634 | ACCTTGGTCCCTCTCAGATAC | ACACATTTGTTTCCTTCCCAG |
| 60 | THOC7 | NM_025075 | GGAAACAGATCCTAAGCCATAG | CTTTGTGAGAGGAAATATGAATG |
| 61 | REEP5 | NM_005669 | CTCGGGAAGACTCCTCTACTC | TTCCATTCAGTGTATCACATC |
| 62 | FAM134B | NM_001034850 | TGCTTATGTCATTAGTGCCTC | CCTGTCCAGAAACTGTTATTG |
| 63 | ZAK | NM_133646 | TATCTTTGTGCGTCTGTTTGC | GCCTGGTGCTGTGTTAAAGTG |
| 64 | SKP2 | NM_005983 | GCACATGGACCTATCGAACTC | GAATCCAGAACACCCAGAAAG |

FIG. 6A

| ProbeID | Symbol | REFSEQ_ID | A | B | C | D | E | F | G | H | I | J |
|---|---|---|---|---|---|---|---|---|---|---|---|---|
| 3360112 | TMEM2 | NM_013390.1 | 0.05917025 | 0.07092667 | 0.10030079 | 0.47719765 | 0.42887878 | 0.55118036 | -0.05917025 | -0.22509623 | -0.30704212 | -0.3019781 |
| 5420095 | MYC | NM_002467.3 | 0.20625567 | 0.21063638 | 0.06156182 | 0.7410009 | 0.84007144 | 0.8650806 | -0.06156182 | -0.21605468 | -0.66159797 | -0.0749104 |
| 6520139 | FGFR3 | NM_022965.1 | -0.76498103 | -0.6427262 | -0.570616 | 0.5769112 | 0.79322934 | 0.5746224 | 0.02032971 | -0.21731305 | -0.95063186 | -0.00745082 |
| 1850092 | PODXL | NM_001018111.1 | 0.00686765 | 0.05131698 | -0.00686765 | 0.531646 | 0.46554208 | 0.5777118 | -0.10511041 | -0.04944921 | -0.28466344 | 0.051965 |
| 580711 | SMAD6 | NM_005585.3 | -0.2259934 | -0.200701 | -0.2720759 | 0.65493464 | 0.7765405 | 0.8151233 | -0.1305902 | -0.07872748 | -0.48185325 | 0.32706332 |
| 1660477 | CALM1 | NM_006888.3 | -0.8044803 | -0.645653 | -0.83822274 | 0.452554 | 0.48014283 | 0.52111363 | 0.16068769 | -0.3049872 | -0.89174104 | 0.01787782 |
| 1580356 | TMED1 | NM_006858.2 | -0.24305677 | -0.26611233 | -0.32296228 | 0.46665955 | 0.38094234 | 0.5993824 | 0.11636877 | -0.16662455 | -0.25905943 | 0.10828018 |
| 5050575 | ASB13 | NM_024701.2 | -0.12304735 | -0.11277676 | -0.05149078 | 0.58461 | 0.60237455 | 0.4867158 | 0.26428318 | -0.02244425 | -0.18726778 | -0.00437737 |
| 4570255 | LEF1 | NM_016269.2 | -0.09144282 | 0.00630736 | -0.06978917 | 0.5172856 | 0.5945637 | 0.50818086 | 0.07064176 | -0.25030303 | -0.56051564 | -0.00630736 |
| 360608 | FEZ2 | NM_005102.2 | -0.0362463 | 0.03749704 | 0.00163794 | 0.33221722 | 0.290699 | 0.3703947 | 0.20532036 | -0.00163794 | -0.34493208 | -0.19547653 |
| 4730674 | SKP2 | NM_005983.2 | -0.47193408 | -0.30542445 | -0.44274068 | 0.38648582 | 0.5755317 | 0.6130874 | 0.38207698 | -0.04981732 | -0.3378694 | 0.03439593 |
| 2070291 | PGM1 | NM_002633.2 | -0.19329643 | -0.11338186 | -0.23791838 | 0.59883404 | 0.68519974 | 0.69089603 | 0.096982 | -0.04221106 | -0.5803752 | 0.26068687 |
| 3710039 | MRPL54 | NM_172251.1 | -0.5936072 | -0.49861836 | -0.63307023 | 0.7285645 | 0.8170922 | 0.6309912 | 0.03993344 | -0.28558564 | -0.5834544 | -0.00721812 |
| 1110114 | DIMT1L | NM_014473.2 | -0.33409786 | -0.26688242 | -0.40218115 | 0.5723934 | 0.6242218 | 0.38954544 | 0.14261341 | -0.01338911 | -0.64574003 | -0.02239513 |
| 6840328 | SMAD3 | NM_005902.3 | -0.07373691 | -0.01145959 | -0.11283088 | 0.4184115 | 0.5731528 | 0.49140477 | 0.24212575 | -0.04780412 | -0.21205497 | 0.09286189 |
| 6620017 | MKX | NM_173576.1 | -0.07914281 | -0.06778979 | -0.23514056 | 0.50093627 | 0.57159543 | 0.4866488 | 0.00276446 | -0.14128757 | -0.25075603 | 0.03751063 |
| 3610671 | SCD5 | NM_001037582.1 | -0.1524446 | -0.17809558 | -0.2196362 | 0.3135941 | 0.34924626 | 0.3123181 | 0.24417806 | -0.04195428 | -0.30082774 | 0.05566621 |
| 3170300 | MTSS1 | NM_014751.2 | -0.17462277 | -0.07818818 | -0.17667794 | 0.39518237 | 0.5199888 | 0.39271283 | 0.14023328 | 0.04321837 | -0.24197125 | 0.01764703 |
| 460358 | CA2 | NM_000067.1 | -0.45094204 | -0.5066428 | -0.489264 | 0.39216518 | 0.65472984 | 0.58955574 | 0.36338234 | 0.01333571 | -0.62090254 | 0.21032429 |
| 3460520 | CCND2 | NM_001759.2 | 0.16456175 | 0.38134384 | 0.17584991 | 1.0843368 | 1.1285462 | 1.1319556 | -0.16456175 | -0.57943726 | -1.2683325 | -0.59840345 |
| 580132 | LAPTM4B | NM_018407.4 | 0.01931644 | -0.0266521 | -0.01931644 | 0.24721599 | 0.2537477 | 0.24630237 | 0.24631476 | 0.062567 | -0.5228317 | -0.20315099 |
| 4230195 | NAV2 | NM_145117.3 | -0.11939454 | -0.02305484 | -6.00E-04 | 0.51540065 | 0.4800651 | 0.54786944 | 0.06181073 | -0.07527947 | -0.15971541 | 6.00E-04 |
| 1400044 | HSPA12A | NM_025015.2 | -0.22657514 | -0.10123372 | -0.2845328 | 0.50144696 | 0.53909993 | 0.4886191 | 0.20332742 | -0.09775472 | -0.44564652 | 0.14246678 |
| 2000630 | SORBS2 | NM_003603.4 | -0.11959338 | -0.16299796 | -0.20247388 | 0.53998494 | 0.6003082 | 0.44393325 | 0.01632237 | -0.02408719 | -0.01632237 | -0.07294822 |
| 7000746 | C3orf31 | NM_138807.2 | -0.82351804 | -0.7613447 | -0.86028075 | 0.353791 | 0.5662401 | 0.39403605 | 0.06317687 | -0.3011639 | -0.5796397 | 0.0882442 |
| 6200402 | MT1A | NM_005946.2 | -0.40774655 | -0.46245885 | -0.37805724 | 0.40116 | 0.60611606 | 0.48149753 | 0.11547351 | -0.00766349 | -0.2454989 | 0.00766349 |
| 940471 | C5orf13 | NM_004772.1 | -0.39075017 | -0.3810742 | -0.33057237 | 0.4434321 | 0.4365723 | 0.4948151 | 0.19850945 | 0.01188636 | -0.3181603 | 0.2270348 |
| 6200431 | AKT1 | NM_001014431.1 | -0.14864588 | -0.04942131 | -0.20472622 | 0.45786905 | 0.55393744 | 0.56376505 | 0.3598237 | -0.00263977 | -0.14773464 | -0.00345182 |
| 3830519 | MKKS | NM_170784.1 | -0.7423017 | -0.5109055 | -0.704015 | 0.38032794 | 0.5061891 | 0.39974093 | 0.04272246 | -0.21505237 | -0.54116225 | 0.05390811 |
| 7400025 | MTP18 | NM_016498.3 | -0.57140636 | -0.51407576 | -0.7428651 | 0.5846834 | 0.42000866 | 0.37310696 | 0.22951603 | -0.09628916 | -0.65704775 | 0.22219563 |
| 7320435 | DPM3 | NM_153741.1 | -0.09732151 | -0.00737906 | -0.17918634 | 0.50263023 | 0.50131893 | 0.5172119 | 0.18942165 | -0.12952471 | -0.3106761 | 0.11035442 |
| 4670059 | ZAK | NM_133646.2 | -0.01893067 | 0.01893067 | -0.09943938 | 0.477803 | 0.43636203 | 0.5190017 | 0.24975848 | -0.06792235 | -0.17298675 | 0.08762908 |

FIG. 6B

| | | | | | | | | | | | | |
|---|---|---|---|---|---|---|---|---|---|---|---|---|
| 4230132 | ILK | NM_004517.2 | -0.28403997 | -0.29379654 | -0.26579475 | 0.45678568 | 0.44190836 | 0.5515094 | 0.317019 | -0.16221237 | -0.5469055 | 0.08060217 |
| 4210050 | WDR54 | NM_032118.2 | -0.27396822 | -0.2966919 | -0.26280022 | 0.5797286 | 0.73473597 | 0.57958364 | 0.22984743 | -0.11513138 | -0.7657194 | 0.16087961 |
| 3290685 | DPYSL2 | NM_001386.4 | 0.15356684 | 0.2962923 | 0.22008991 | 0.8032708 | 0.7986617 | 0.70381784 | -0.15356684 | -0.5180054 | -0.9191189 | -0.31133986 |
| 1400520 | CNTNAP2 | NM_014141.4 | -0.62819266 | -0.33051038 | -0.52470326 | 1.1980612 | 1.2911551 | 1.1916621 | 0.01324678 | -0.5513909 | -1.1547339 | 0.01778054 |
| 3060128 | CHSY1 | NM_014918.3 | 0.17371964 | 0.14724231 | 0.1341312 | 0.3736441 | 0.3820498 | 0.39279675 | -0.1341312 | -0.5212271 | -0.7432635 | -0.519613 |
| 4570524 | ICK | NM_016513.3 | 0.10064411 | 0.09588337 | 0.08099413 | 0.37888813 | 0.33072376 | 0.4014578 | -0.28502274 | -0.32924223 | -0.6184287 | -0.08099413 |
| 3140139 | NEFL | NM_006158.2 | -0.06109953 | 0.01105356 | -0.10892534 | 0.48640776 | 0.47588968 | 0.39379358 | -0.1027565 | -0.19219351 | -0.08963013 | 0.02886057 |
| 7560497 | RPRML | NM_203400.1 | -0.17151403 | -0.20159149 | -0.1645608 | 0.39767075 | 0.47506475 | 0.3678589 | 0.02845955 | 0.01255751 | -0.04900932 | 0.03068113 |
| 2320093 | PIGY | NM_001042616.1 | -0.17731237 | -0.05455494 | -0.09842777 | 0.4170146 | 0.3795743 | 0.49765825 | 0.31376505 | 0.05455494 | -0.3881569 | 0.24772596 |
| 2340372 | MRFAP1 | NM_033296.1 | -0.0919199 | -0.05082083 | -0.10750532 | 0.36676025 | 0.43453503 | 0.3325615 | 0.5261925 | 0.03342485 | -0.39957285 | -0.03342533 |
| 650619 | FAM125B | NM_033446.1 | 0.01272321 | -0.02433324 | -0.00691676 | 0.43962693 | 0.45145297 | 0.40305448 | 0.04469371 | -0.23243213 | -0.11730313 | 0.00691676 |
| 1690156 | NUAK1 | NM_014840.2 | 0.05442143 | 0.09787417 | 0.1319189 | 0.52485275 | 0.5211916 | 0.5817747 | -0.20394802 | -0.47769213 | -0.61529684 | -0.19596958 |
| 2650598 | CYP4V2 | NM_207352.2 | -0.4010818 | -0.38692737 | -0.29648232 | 0.3822515 | 0.5828645 | 0.5345571 | 0.07066656 | 0.07740521 | -0.3311298 | 0.01757836 |
| 6590300 | HES5 | NM_001010926.2 | 0.00126433 | -0.16841865 | 3.56E-04 | 0.80008197 | 0.7498424 | 0.706187 | -0.30463767 | -0.18252873 | -0.42113042 | 0.0744431 |
| 1110605 | FTSJ1 | NM_012280.2 | -0.23364282 | -0.3216951 | -0.40345073 | 0.4616096 | 0.33336616 | 0.47136855 | 0.3320539 | 0.06173968 | -0.2139318 | 0.16628718 |
| 5390333 | TMEM98 | NM_001033504.1 | -0.47827554 | -0.27277637 | -0.38760638 | 0.63498473 | 0.9491279 | 0.796129 | 0.13022208 | -0.14812541 | -0.8135469 | 0.19442916 |
| 2850291 | GARNL4 | NM_015085.3 | -0.14655137 | -0.17149377 | -0.18679309 | 0.41003203 | 0.3753121 | 0.507036 | 0.05116868 | -0.04025865 | -0.2673385 | 0.09249854 |
| 5820601 | CCND1 | NM_053056.2 | -0.25157404 | -0.01787949 | -0.1779809 | 1.2034063 | 1.15131 | 1.0072899 | -0.04000187 | -0.21530294 | -0.42514563 | 0.14800596 |
| 2030132 | LOC653506 | XM_927769.1 | -0.09368753 | -0.06354666 | -0.06232834 | 0.44838572 | 0.5069728 | 0.48317194 | 0.1511836 | -0.02635098 | -0.17952108 | 0.06579971 |
| 5420347 | HCFC1R1 | NM_001002018.1 | -0.447145 | -0.30883694 | -0.31431198 | 0.4007945 | 0.37067842 | 0.51739645 | 0.2851987 | -0.14147758 | -0.2627616 | 0.16804266 |
| 3780762 | FAM44B | NM_138369.1 | -0.4200151 | -0.3115294 | -0.4600861 | 0.3668635 | 0.3414786 | 0.5041077 | 0.0079186 | -0.0079186 | -0.4552281 | 0.10892606 |
| 870161 | CXADR | NM_001338.3 | -0.06940198 | 0.05747438 | 0.05618024 | 0.74545264 | 0.5984905 | 0.6801288 | -0.2458899 | -0.22135377 | -0.46954322 | 0.01680827 |
| 2190674 | IGFBP5 | NM_000599.2 | -0.20485854 | -0.27005792 | -0.2338221 | 0.69652534 | 0.78981566 | 0.54021907 | -0.01966548 | -0.09686637 | -0.5820396 | 0.06907296 |
| 7570446 | TARBP2 | NM_134323.1 | -0.24840665 | -0.27859664 | -0.15607476 | 0.38426614 | 0.2984531 | 0.3170855 | 0.05110049 | 0.10654378 | -0.13683915 | 0.05623221 |
| 4290706 | EYA2 | NM_172112.1 | -0.14592719 | -0.19804978 | -0.15167308 | 0.4094522 | 0.46912646 | 0.52224135 | 0.00160718 | -0.09882188 | -0.36732793 | 0.07026887 |
| 2230367 | CERK | NM_022766.4 | -0.12929296 | -0.15110731 | -0.28905725 | 0.6331639 | 0.6641345 | 0.57462454 | 0.3566928 | -0.18384027 | -0.38283587 | 0.06344223 |
| 3890187 | PRMT6 | NM_018137.1 | -0.45528865 | -0.54608417 | -0.5539601 | 0.37588334 | 0.33577466 | 0.2530892 | 0.4401648 | 0.06941104 | -0.17455173 | 0.00197673 |
| 780133 | SH3D19 | NM_001009555.2 | -0.01176882 | 0.01176882 | 0.1000495 | 0.76189375 | 0.8915143 | 0.8910823 | 0.1544118 | -0.07160139 | -0.34567642 | -0.26836538 |
| 4280204 | PHB | NM_002634.2 | -0.05875063 | -0.01142597 | -0.09996128 | 0.4671607 | 0.61903715 | 0.4727807 | 0.26115847 | 0.01142597 | -0.5489807 | -0.03035498 |
| 5870113 | THOC7 | NM_025075.1 | -0.79957986 | -0.7073324 | -0.8958566 | 0.3745916 | 0.4327562 | 0.33682323 | 0.30562568 | 0.00872541 | -0.63857245 | -0.00872541 |
| 520324 | REEP5 | NM_005669.4 | -0.47876716 | -0.5589411 | -0.6944411 | 0.48814893 | 0.46968102 | 0.54176736 | 0.25787282 | 0.01686168 | -0.5424559 | -0.01686168 |
| 6420309 | FAM134B | NM_001034850.1 | -0.17534304 | -0.11143732 | -0.1377101 | 0.45166254 | 0.49799204 | 0.45121527 | 0.19206858 | 0.04558849 | -0.04973841 | -0.02552319 |
| | group 2 vs group1 p<=0.05 | | | group 1 (A,B,C) nownt-sinon | | | | | | | | |
| | group 2 vs group 4 fold>=1.3 | | | group 2 (D,E,F) wnt-sinon | | | | | | | | |
| | exclude opposite genes | | | group 3 (G,H,I) nownt-siCTNNB1 | | | | | | | | |
| | | | | group 4 (J,K,L) wnt-siCTNNB1 | | | | | | | | |

FIG. 6C

| ProbeID | K | L | FCAbsolute (wnt-sinon vs nownt-sinon) | Corrected p-value | p-value | regulation | FCAbsolute( nownt-siCTNNB1 vs nownt-sinon) | Corrected p-value | p-value | regulation | FCAbsolute( wnt-siCTNNB1 vs nownt-sinon) | Corrected p-value |
|---|---|---|---|---|---|---|---|---|---|---|---|---|
| 3360112 | -0.23238564 | -0.1642971 | 1.327722 | 0.04584246 | 4.06E-04 | up | 1.2090737 | 0.33510348 | 0.02074598 | down | 1.239438 | 0.05052814 |
| 5420095 | -0.0924356 | -0.14044642 | 1.5755982 | 0.04755635 | 4.50E-04 | up | 1.3875657 | 0.49119106 | 0.06433625 | down | 1.1992081 | 0.10226964 |
| 6520139 | 0.00745082 | 0.03320479 | 2.475458 | 0.03916105 | 1.43E-04 | up | 1.2115909 | 0.8538606 | 0.40490547 | up | 1.5916348 | 0.02940745 |
| 1850092 | -0.22222447 | -0.15516257 | 1.4219404 | 0.03939719 | 1.63E-04 | up | 1.1200113 | 0.55163676 | 0.08896487 | down | 1.090946 | 0.5450103 |
| 580711 | 0.12437606 | 0.07872748 | 1.9749137 | 0.02931408 | 4.85E-05 | up | 1.0017574 | 0.9985851 | 0.9851971 | up | 1.3283596 | 0.09529766 |
| 1660477 | -0.01787782 | 0.06876445 | 2.3741133 | 0.02928034 | 3.75E-05 | up | 1.3355542 | 0.7524505 | 0.2496311 | up | 1.7239368 | 0.02825333 |
| 1580356 | -0.09421492 | 0.09421492 | 1.6931446 | 0.044535 | 3.62E-04 | up | 1.128395 | 0.71332294 | 0.20553076 | up | 1.2426935 | 0.12137282 |
| 5050575 | 0.22088432 | 0.00437737 | 1.5731668 | 0.03632479 | 1.03E-04 | up | 1.0821961 | 0.8691683 | 0.44236168 | up | 1.1245905 | 0.37431133 |
| 4570255 | -0.08585668 | 0.01680255 | 1.5069709 | 0.03792959 | 1.26E-04 | up | 1.1447909 | 0.82713675 | 0.35021287 | down | 1.018553 | 0.82232887 |
| 360608 | -0.25392675 | -0.21935892 | 1.257136 | 0.04813069 | 4.60E-04 | up | 1.0338638 | 0.96754456 | 0.78137606 | down | 1.1678731 | 0.0435276 |
| 4730674 | 0.03397203 | -0.03397203 | 1.9075682 | 0.04669999 | 4.29E-04 | up | 1.3239328 | 0.63026845 | 0.1333884 | up | 1.3362269 | 0.05052814 |
| 2070291 | -0.13789797 | 0.04221106 | 1.7898543 | 0.02935696 | 5.79E-05 | up | 1.0043979 | 0.9979157 | 0.97736424 | up | 1.1781573 | 0.42824 |
| 3710039 | 0.00721812 | 0.08169293 | 2.4633949 | 0.02928034 | 4.14E-05 | up | 1.2300609 | 0.6894125 | 0.18051621 | up | 1.518166 | 0.02787541 |
| 1110114 | 0.01338911 | 0.13400126 | 1.8189518 | 0.04713156 | 4.43E-04 | up | 1.119004 | 0.9063808 | 0.54280806 | up | 1.2977858 | 0.07010672 |
| 6840328 | 0.01145959 | -0.05219245 | 1.4746087 | 0.04853779 | 4.72E-04 | up | 1.0425366 | 0.9449641 | 0.6814354 | up | 1.0595014 | 0.5091888 |
| 6620017 | 0.07804799 | -0.00276446 | 1.5660003 | 0.04637714 | 4.20E-04 | up | 1.0016663 | 0.997946 | 0.980234 | down | 1.1211317 | 0.26724494 |
| 3610671 | -0.29953313 | 0.04195428 | 1.4225161 | 0.02837349 | 2.49E-05 | up | 1.1099727 | 0.85104114 | 0.39634374 | up | 1.083792 | 0.7006543 |
| 3170300 | -0.01764703 | -0.13905406 | 1.4939421 | 0.0457843 | 4.02E-04 | up | 1.0894927 | 0.8307811 | 0.3582106 | up | 1.0694075 | 0.49405703 |
| 460358 | -0.04493952 | -0.01333571 | 2.0388656 | 0.03939719 | 2.18E-04 | up | 1.3203205 | 0.74168926 | 0.23704667 | up | 1.4469008 | 0.06336355 |
| 3460520 | -0.5458927 | -0.5191746 | 1.833196 | 0.04138748 | 2.65E-04 | up | 1.8808206 | 0.45059112 | 0.05054658 | down | 1.7351681 | 0.03181534 |
| 580132 | -0.16750121 | -0.07313418 | 1.1957972 | 0.02935696 | 5.78E-05 | up | 1.044225 | 0.97065854 | 0.80144376 | down | 1.1011758 | 0.20118825 |
| 4230195 | 0.11609387 | -0.00358081 | 1.4764457 | 0.03939719 | 1.70E-04 | up | 1.0069869 | 0.98633754 | 0.89874893 | down | 1.0609727 | 0.5166221 |
| 1400044 | -0.09006143 | 0.09006143 | 1.6401592 | 0.03957788 | 2.21E-04 | up | 1.064928 | 0.9414256 | 0.66608196 | up | 1.1905291 | 0.26314378 |
| 2000630 | 0.13324904 | 0.09557462 | 1.6130195 | 0.03939719 | 1.79E-04 | up | 1.1123874 | 0.19864501 | 0.00467485 | up | 1.1596158 | 0.22305924 |
| 7000746 | -0.06317687 | 0.09068179 | 2.383481 | 0.02935696 | 6.14E-05 | up | 1.4564998 | 0.43284675 | 0.044994 | up | 1.807043 | 0.02396943 |
| 6200402 | -0.03036714 | 0.1961739 | 1.8821027 | 0.03916105 | 1.45E-04 | up | 1.2925241 | 0.3634721 | 0.02720557 | up | 1.3888694 | 0.06549862 |
| 940471 | -0.06404042 | -0.01188636 | 1.7724425 | 0.02175117 | 6.03E-06 | up | 1.2583594 | 0.5619125 | 0.09492132 | up | 1.3359212 | 0.11836423 |
| 6200431 | 0.00263977 | 0.28256416 | 1.5794859 | 0.04252872 | 3.10E-04 | up | 1.1519523 | 0.7647253 | 0.26506728 | up | 1.1713579 | 0.37644837 |
| 3830519 | -0.04272246 | 0.04701877 | 2.1157367 | 0.03939719 | 1.88E-04 | up | 1.3329076 | 0.54618734 | 0.08673544 | up | 1.5930692 | 0.0402488 |
| 7400025 | -0.14395094 | 0.09628916 | 2.097565 | 0.04376151 | 3.42E-04 | up | 1.3517629 | 0.68873525 | 0.17984633 | up | 1.5884581 | 0.09332971 |
| 7320435 | -0.01013708 | 0.00737906 | 1.5174854 | 0.04138748 | 2.71E-04 | up | 1.0076789 | 0.99377257 | 0.94644 | up | 1.0946687 | 0.3968176 |
| 4670059 | -0.04622817 | -0.06655383 | 1.4249079 | 0.04138748 | 2.69E-04 | up | 1.0253357 | 0.9697186 | 0.7977182 | up | 1.0173119 | 0.88788944 |

FIG. 6D

| | | | | | | | | | | | | |
|---|---|---|---|---|---|---|---|---|---|---|---|---|
| 4230132 | -0.05283642 | 0.05283642 | 1.6989125 | 0.02837349 | 2.68E-05 | up | 1.1099623 | 0.91677004 | 0.57964367 | up | 1.238057 | 0.05072185 |
| 4210050 | -0.11497116 | 0.11497116 | 1.8779641 | 0.02964491 | 6.60E-05 | up | 1.0430577 | 0.977488 | 0.8452059 | up | 1.2582744 | 0.15978105 |
| 3290685 | -0.63078403 | -0.27594376 | 1.4592905 | 0.04907665 | 4.83E-04 | up | 1.6859324 | 0.36919326 | 0.02855038 | down | 1.546856 | 0.09323008 |
| 1400520 | -0.01324678 | 0.07472014 | 3.2976272 | 0.02931408 | 5.01E-05 | up | 1.0495886 | 0.97840023 | 0.8509069 | down | 1.434837 | 0.0790943 |
| 3060128 | -0.6403086 | -0.31365132 | 1.173756 | 0.02935696 | 5.69E-05 | up | 1.534645 | 0.35778385 | 0.02571546 | down | 1.5614525 | 0.06029106 |
| 4570524 | -0.37675953 | -0.2677927 | 1.2123863 | 0.03939719 | 2.14E-04 | up | 1.4175553 | 0.2404566 | 0.00860171 | down | 1.2608144 | 0.15928072 |
| 3140139 | -0.01105356 | 0.09854746 | 1.4191438 | 0.04461852 | 3.76E-04 | up | 1.0535092 | 0.69687545 | 0.18839476 | down | 1.0656807 | 0.43184814 |
| 7560497 | -0.07278776 | -0.01255751 | 1.5081224 | 0.02935696 | 6.29E-05 | up | 1.1301843 | 0.1684062 | 0.00253196 | up | 1.1180624 | 0.10044543 |
| 2320093 | -0.07782364 | -0.43376637 | 1.4554992 | 0.04598351 | 4.14E-04 | up | 1.0743663 | 0.9350069 | 0.6449944 | up | 1.0154673 | 0.97423655 |
| 2340372 | -0.09048128 | 0.09461069 | 1.3768464 | 0.03939719 | 1.79E-04 | up | 1.0999441 | 0.9326782 | 0.6357878 | up | 1.0523758 | 0.6044486 |
| 650619 | -0.14050078 | -0.0094173 | 1.354306 | 0.0263472 | 1.73E-05 | up | 1.0684392 | 0.79676306 | 0.3041747 | down | 1.0291773 | 0.7402011 |
| 1690156 | -0.24929857 | -0.05442143 | 1.3640232 | 0.03762268 | 1.14E-04 | up | 1.4409802 | 0.27788353 | 0.01278197 | down | 1.1985594 | 0.13757123 |
| 2650598 | -0.11213327 | -0.01757836 | 1.8167857 | 0.04079708 | 2.34E-04 | up | 1.2315522 | 0.56437767 | 0.09668005 | up | 1.2519001 | 0.06534282 |
| 6590300 | -3.56E-04 | -0.28031564 | 1.7503414 | 0.03939719 | 2.08E-04 | up | 1.1868737 | 0.44964975 | 0.05001978 | down | 1.0091522 | 0.97500706 |
| 1110605 | -0.06173968 | -0.08557963 | 1.6721578 | 0.044535 | 3.61E-04 | up | 1.3009361 | 0.5373977 | 0.08299412 | up | 1.2534624 | 0.19232263 |
| 5390333 | -0.13022208 | 0.15008283 | 2.2547486 | 0.04598351 | 4.15E-04 | up | 1.0735599 | 0.95754987 | 0.7385916 | up | 1.3669711 | 0.16184144 |
| 2850291 | 0.02267289 | -0.02267289 | 1.5147427 | 0.03800846 | 1.29E-04 | up | 1.0590739 | 0.8664861 | 0.4344215 | up | 1.1479918 | 0.08195241 |
| 5820601 | 0.01985979 | 0.01787949 | 2.411304 | 0.03916105 | 1.50E-04 | up | 1.0553137 | 0.9183297 | 0.5850553 | down | 1.1575383 | 0.29987517 |
| 2030132 | -0.01776266 | 0.01776266 | 1.466826 | 0.02411978 | 9.93E-06 | up | 1.0388288 | 0.9215054 | 0.5980097 | up | 1.0681548 | 0.17852132 |
| 5420347 | -0.17981243 | 0.14147758 | 1.7247506 | 0.04106888 | 2.46E-04 | up | 1.2458104 | 0.63901186 | 0.13939735 | up | 1.3195084 | 0.20614144 |
| 3780762 | -0.25180173 | 0.01717544 | 1.7427434 | 0.04138748 | 2.84E-04 | up | 1.1854769 | 0.70411855 | 0.19561668 | up | 1.2792605 | 0.23664463 |
| 870161 | -0.01680827 | -0.04405427 | 1.5800166 | 0.0449633 | 3.84E-04 | up | 1.2544136 | 0.338788 | 0.02167633 | down | 1.0206128 | 0.8122856 |
| 2190674 | 0.07706666 | 0.01966548 | 1.8813473 | 0.04138748 | 2.66E-04 | up | 1.0023519 | 0.9987309 | 0.985642 | up | 1.2239245 | 0.03061705 |
| 7570446 | -0.17562938 | -0.05110049 | 1.4752516 | 0.0410251 | 2.41E-04 | up | 1.1766031 | 0.4377019 | 0.04644178 | up | 1.1257294 | 0.36694217 |
| 4290706 | 0.05508733 | -0.00160718 | 1.5498803 | 0.02964491 | 6.54E-05 | up | 1.0072132 | 0.9920474 | 0.9302869 | up | 1.1538585 | 0.04971791 |
| 2230367 | -0.06344223 | 0.08139563 | 1.7578275 | 0.03898787 | 1.35E-04 | up | 1.0866028 | 0.9302095 | 0.62483114 | up | 1.1622747 | 0.21742521 |
| 3890187 | -0.18649077 | -0.00197673 | 1.7900833 | 0.02935696 | 6.29E-05 | up | 1.5476927 | 0.3566294 | 0.02553887 | up | 1.3720002 | 0.06255896 |
| 780133 | -0.08470726 | -0.13083887 | 1.7590709 | 0.03773382 | 1.20E-04 | up | 1.0874672 | 0.8766153 | 0.46110985 | down | 1.1444494 | 0.24068375 |
| 4280204 | -0.0128994 | 0.09385777 | 1.491095 | 0.04980087 | 4.99E-04 | up | 1.0248548 | 0.9852621 | 0.8902181 | down | 1.052325 | 0.51937115 |
| 5870113 | -0.15443969 | 0.08154416 | 2.2694037 | 0.02928034 | 4.22E-05 | up | 1.6164727 | 0.509155 | 0.07124914 | up | 1.7096677 | 0.03899507 |
| 520324 | -0.13825917 | 0.10818839 | 2.1100085 | 0.03283544 | 8.52E-05 | up | 1.402638 | 0.60513604 | 0.11741041 | up | 1.4760473 | 0.07462262 |
| 6420309 | -0.0963397 | 0.02552319 | 1.524624 | 0.02579014 | 1.48E-05 | up | 1.1519965 | 0.44481388 | 0.04846948 | up | 1.0787672 | 0.27708542 |
| | | | | | | | | | | | | |
| | | | | | | | | | | | | |
| | | | | | | | | | | | | |
| | | | | | | | | | | | | |

FIG. 6E

| ProbeID | p-value | regulation | FCAbsolute( nownt-siCTNNB1 vs wnt-siCTNNB1) | Corrected p-value | p-value | regulation | FCAbsolute( wnt-sinon vs wnt-siCTNNB1) | Corrected p-value | p-value | regulation | FCAbsolute( nownt-sinon vs wnt-siCTNNB1) | Corrected p-value |
|---|---|---|---|---|---|---|---|---|---|---|---|---|
| 3360112 | 0.00173675 | down | 1.0251138 | 0.9776424 | 0.68872917 | up | 1.645629 | 0.07005717 | 1.76E-04 | up | 1.239438 | 0.05052814 |
| 5420095 | 0.00766023 | down | 1.1570683 | 0.9434789 | 0.30940694 | down | 1.8894702 | 0.05458321 | 2.76E-05 | up | 1.1992081 | 0.10226964 |
| 6520139 | 3.19E-04 | up | 1.3136734 | 0.93085265 | 0.24956486 | down | 1.5552926 | 0.09226806 | 9.72E-04 | up | 1.5916348 | 0.02940745 |
| 1850092 | 0.21086007 | down | 1.0266423 | 0.9808855 | 0.7450221 | down | 1.5512602 | 0.11355642 | 0.0020346 | up | 1.090946 | 0.5450103 |
| 580711 | 0.00661913 | up | 1.3260293 | 0.8896548 | 0.05117799 | down | 1.4867312 | 0.12901047 | 0.00317684 | up | 1.3283596 | 0.09529766 |
| 1660477 | 2.60E-04 | up | 1.2908025 | 0.9396131 | 0.2944793 | down | 1.3771465 | 0.06909774 | 1.35E-04 | up | 1.7239368 | 0.02825333 |
| 1580356 | 0.01071826 | up | 1.1012931 | 0.94541866 | 0.34604692 | down | 1.3624797 | 0.17296569 | 0.00805395 | up | 1.2426935 | 0.12137282 |
| 5050575 | 0.09260461 | up | 1.0391743 | 0.9790928 | 0.7322863 | down | 1.3988799 | 0.1408517 | 0.00411036 | up | 1.1245905 | 0.37431133 |
| 4570255 | 0.5703936 | up | 1.1660302 | 0.9401564 | 0.29673773 | down | 1.4795214 | 0.07001147 | 1.68E-04 | up | 1.018553 | 0.82232887 |
| 360608 | 0.00119191 | down | 1.12962 | 0.94541866 | 0.33707538 | up | 1.4681754 | 0.05486843 | 4.18E-05 | up | 1.1678731 | 0.0435276 |
| 4730674 | 0.00173498 | up | 1.0092862 | 0.99793786 | 0.9525132 | down | 1.4275781 | 0.11746222 | 0.00223037 | up | 1.3362269 | 0.05052814 |
| 2070291 | 0.12196246 | up | 1.1729987 | 0.94874126 | 0.38550234 | down | 1.5191981 | 0.16805871 | 0.00713671 | up | 1.1781573 | 0.42824 |
| 3710039 | 2.41E-04 | up | 1.2342203 | 0.9171619 | 0.17081934 | down | 1.6226122 | 0.0777968 | 3.20E-04 | up | 1.518166 | 0.02787541 |
| 1110114 | 0.00358929 | up | 1.1597686 | 0.95723855 | 0.43311572 | down | 1.401581 | 0.14573903 | 0.00468529 | up | 1.2977858 | 0.07010672 |
| 6840328 | 0.17950614 | up | 1.0162727 | 0.99230796 | 0.87530047 | down | 1.3917949 | 0.10217137 | 0.00147291 | up | 1.0595014 | 0.5091888 |
| 6620017 | 0.04858149 | up | 1.1229999 | 0.91095686 | 0.0955261 | down | 1.3968031 | 0.07001147 | 1.63E-04 | up | 1.1211317 | 0.26724494 |
| 3610671 | 0.38025782 | up | 1.0241566 | 0.9921743 | 0.8688255 | up | 1.312536 | 0.2580598 | 0.02833327 | up | 1.083792 | 0.7006543 |
| 3170300 | 0.1676194 | up | 1.0187817 | 0.98999447 | 0.83936834 | up | 1.3969812 | 0.10491759 | 0.00160109 | up | 1.0694075 | 0.49405703 |
| 460358 | 0.00289173 | up | 1.0958709 | 0.9776424 | 0.6815655 | down | 1.4091259 | 0.19434664 | 0.01175317 | up | 1.4469008 | 0.06336355 |
| 3460520 | 4.30E-04 | down | 1.0839415 | 0.97932905 | 0.7368251 | down | 3.1809032 | 0.00951164 | 4.65E-07 | up | 1.7351681 | 0.03181534 |
| 580132 | 0.02818301 | down | 1.0545387 | 0.9831688 | 0.76088345 | up | 1.3167828 | 0.08469696 | 5.17E-04 | up | 1.1011758 | 0.20118825 |
| 4230195 | 0.18590397 | up | 1.0683856 | 0.9340403 | 0.27501068 | down | 1.3915962 | 0.08036991 | 4.06E-04 | up | 1.0609727 | 0.5166221 |
| 1400044 | 0.04717905 | up | 1.1179432 | 0.9597624 | 0.46694535 | down | 1.3776726 | 0.12753457 | 0.00303067 | up | 1.1905291 | 0.26314378 |
| 2000630 | 0.03441646 | up | 1.0424567 | 0.954574 | 0.40559804 | down | 1.3909948 | 0.13427287 | 0.0036538 | up | 1.1596158 | 0.22305924 |
| 7000746 | 1.28E-04 | up | 1.240675 | 0.9172609 | 0.18214966 | down | 1.3189952 | 0.17459506 | 0.00845032 | up | 1.807043 | 0.02396943 |
| 6200402 | 0.00309573 | up | 1.0745404 | 0.9582638 | 0.45995954 | down | 1.355133 | 0.17771208 | 0.00886283 | up | 1.3888694 | 0.06549862 |
| 940471 | 0.01029901 | up | 1.0616372 | 0.97510695 | 0.64894 | down | 1.3267567 | 0.19097805 | 0.01116598 | up | 1.3359212 | 0.11836423 |
| 6200431 | 0.09480499 | up | 1.0168458 | 0.9940102 | 0.8988314 | down | 1.348423 | 0.19972035 | 0.01260669 | up | 1.1713579 | 0.37844837 |
| 3830519 | 0.00100281 | up | 1.1951835 | 0.9246163 | 0.20862894 | down | 1.3280884 | 0.09634664 | 0.00121174 | up | 1.5930692 | 0.0402488 |
| 7400025 | 0.00635819 | up | 1.1751012 | 0.95741194 | 0.45295835 | down | 1.3205037 | 0.27230486 | 0.03271113 | up | 1.5884581 | 0.09332971 |
| 7320435 | 0.10406482 | up | 1.0863271 | 0.960001 | 0.47296694 | down | 1.3862507 | 0.0723593 | 2.41E-04 | up | 1.0946687 | 0.3968176 |
| 4670059 | 0.6993156 | up | 1.0078871 | 0.9965293 | 0.9375719 | up | 1.4006598 | 0.08965625 | 8.39E-04 | up | 1.0173119 | 0.88788944 |

FIG. 6F

| | | | | | | | | | | | | |
|---|---|---|---|---|---|---|---|---|---|---|---|---|
| 4230132 | 0.00175277 | up | 1.1154045 | 0.9689514 | 0.56742424 | down | 1.3722409 | 0.09278597 | 0.00101314 | up | 1.238057 | 0.05072185 |
| 4210050 | 0.0181805 | up | 1.2063326 | 0.95638555 | 0.4237809 | down | 1.4924916 | 0.14405657 | 0.00442092 | up | 1.2582744 | 0.15978105 |
| 3290685 | 0.00634756 | down | 1.0899091 | 0.9750697 | 0.6430687 | down | 2.2573123 | 0.08469696 | 5.61E-04 | up | 1.546856 | 0.09323008 |
| 1400520 | 0.00460625 | up | 1.5059884 | 0.91235435 | 0.15563779 | down | 2.2982593 | 0.04432925 | 8.25E-06 | up | 1.434837 | 0.0790943 |
| 3060128 | 0.00259379 | down | 1.0174682 | 0.99448794 | 0.9074959 | up | 1.8327643 | 0.08886172 | 7.92E-04 | up | 1.5614525 | 0.06029106 |
| 4570524 | 0.01810717 | down | 1.1243172 | 0.9361705 | 0.28054518 | down | 1.528594 | 0.11921038 | 0.00232501 | up | 1.2608144 | 0.15928072 |
| 3140139 | 0.1246087 | up | 1.1227045 | 0.8896548 | 0.02129006 | down | 1.3316782 | 0.08603825 | 6.79E-04 | up | 1.0656807 | 0.43184814 |
| 7560497 | 0.00739417 | up | 1.0108418 | 0.9785845 | 0.70457315 | up | 1.3488715 | 0.08469696 | 5.96E-04 | up | 1.1180624 | 0.10044543 |
| 2320093 | 0.91719127 | up | 1.058002 | 0.9854832 | 0.7888642 | up | 1.4333296 | 0.3341827 | 0.06012831 | up | 1.0154673 | 0.97423655 |
| 2340372 | 0.26788586 | up | 1.0452008 | 0.9884926 | 0.8270851 | up | 1.308322 | 0.13178104 | 0.0034128 | up | 1.0523758 | 0.6044486 |
| 650619 | 0.434956 | down | 1.0381489 | 0.97363573 | 0.59229976 | down | 1.393821 | 0.08469696 | 6.07E-04 | up | 1.0291773 | 0.7402011 |
| 1690156 | 0.01377497 | down | 1.2022601 | 0.9116602 | 0.11866931 | down | 1.6348628 | 0.0777968 | 3.20E-04 | up | 1.1985594 | 0.13757123 |
| 2650598 | 0.00308029 | up | 1.016522 | 0.99230796 | 0.8745494 | down | 1.4512225 | 0.10648412 | 0.00170499 | up | 1.2519001 | 0.06534282 |
| 6590300 | 0.9192721 | down | 1.1761097 | 0.9116602 | 0.14167212 | down | 1.7663609 | 0.10826653 | 0.00180352 | up | 1.0091522 | 0.97500706 |
| 1110605 | 0.02570582 | up | 1.037874 | 0.9842285 | 0.77684915 | up | 1.334031 | 0.18746106 | 0.01057043 | up | 1.2534624 | 0.19232263 |
| 5390333 | 0.01861825 | up | 1.2733067 | 0.9424701 | 0.30684584 | down | 1.6494488 | 0.15897574 | 0.00608637 | up | 1.3669711 | 0.16184144 |
| 2850291 | 0.00495886 | up | 1.0839581 | 0.9439195 | 0.3112752 | down | 1.3194718 | 0.1025667 | 0.00150668 | up | 1.1479918 | 0.08195241 |
| 5820601 | 0.060329 | up | 1.2215661 | 0.9035627 | 0.07284764 | down | 2.0831313 | 0.06909774 | 1.30E-04 | up | 1.1575383 | 0.29987517 |
| 2030132 | 0.02240777 | up | 1.0282298 | 0.9785845 | 0.704545 | down | 1.3732334 | 0.06803054 | 1.02E-04 | up | 1.0681548 | 0.17852132 |
| 5420347 | 0.02945752 | up | 1.0591568 | 0.9779646 | 0.70009834 | down | 1.307116 | 0.2719499 | 0.03262045 | up | 1.3195084 | 0.20614144 |
| 3780762 | 0.03850586 | up | 1.0791105 | 0.97321945 | 0.58743685 | down | 1.3623053 | 0.23081851 | 0.02022164 | up | 1.2792605 | 0.23664463 |
| 870161 | 0.5538471 | down | 1.2290789 | 0.8896548 | 0.02123229 | down | 1.6125852 | 0.06813176 | 1.16E-04 | up | 1.0206128 | 0.8122856 |
| 2190674 | 3.62E-04 | up | 1.2210528 | 0.9171619 | 0.17873828 | down | 1.5371432 | 0.09598332 | 0.00116626 | up | 1.2239245 | 0.03061705 |
| 7570446 | 0.08915674 | up | 1.0451918 | 0.96848273 | 0.55669963 | up | 1.310485 | 0.15533009 | 0.00559172 | up | 1.1257294 | 0.36694217 |
| 4290706 | 0.00166065 | up | 1.1455951 | 0.91235435 | 0.15563919 | down | 1.3432151 | 0.0805364 | 4.10E-04 | up | 1.1538585 | 0.04971791 |
| 2230367 | 0.03271554 | up | 1.0696408 | 0.9776424 | 0.68899107 | down | 1.5124029 | 0.07901091 | 3.44E-04 | up | 1.1622747 | 0.21742521 |
| 3890187 | 0.00282538 | up | 1.1280557 | 0.95571494 | 0.41021937 | up | 1.3047252 | 0.15767321 | 0.00594234 | up | 1.3720002 | 0.06255896 |
| 780133 | 0.03981937 | down | 1.052399 | 0.97710776 | 0.65881985 | up | 2.0131676 | 0.06909774 | 1.35E-04 | up | 1.1444494 | 0.24068375 |
| 4280204 | 0.1886676 | up | 1.0784804 | 0.9776424 | 0.6765545 | down | 1.4169528 | 0.09996143 | 0.00134236 | up | 1.052325 | 0.51937115 |
| 5870113 | 9.10E-04 | up | 1.0576533 | 0.9862591 | 0.7921729 | down | 1.3273946 | 0.15281498 | 0.00530291 | up | 1.7096677 | 0.03899507 |
| 520324 | 0.00409405 | up | 1.0523367 | 0.9850848 | 0.78099096 | down | 1.4294993 | 0.11837966 | 0.00227275 | up | 1.4760473 | 0.07462262 |
| 6420309 | 0.0518426 | up | 1.0678824 | 0.9399604 | 0.2949708 | up | 1.4133021 | 0.07009397 | 2.06E-04 | up | 1.0787672 | 0.27708542 |
| | | | | | | | | | | | | |
| | | | | | | | | | | | | |
| | | | | | | | | | | | | |
| | | | | | | | | | | | | |

FIG. 6G

| ProbeID | p-value | regulation | Entrez_Gene_ID | Definition | Ontology_Process | Ontology_Component |
|---|---|---|---|---|---|---|
| 3360112 | 0.00173675 | up | 23670 | Homo sapiens transmembrane protein 2 (TMEM2), mRNA. | | GO:0016020\|GO:0016021 |
| 5420095 | 0.00766023 | up | 4609 | Homo sapiens v-myc myelocytomatosis v | GO:0006355\|GO:0006355\|GO:0006355\|G | GO:0005634\|GO:0005634\|GO:0005634\|G |
| 6520139 | 3.19E-04 | down | 2261 | Homo sapiens fibroblast growth factor rec | GO:0000165\|GO:0001501\|GO:0006468\|G | GO:0005886\|GO:0005886\|GO:0005887 |
| 1850092 | 0.21086007 | up | 5420 | Homo sapiens podocalyxin-like (PODXL), | GO:0050900 | GO:0005886\|GO:0005887\|GO:0016020 |
| 580711 | 0.00661913 | down | 4091 | Homo sapiens SMAD family member 6 (S | GO:0006350\|GO:0006355\|GO:0006955\|G | GO:0005622\|GO:0005634\|GO:0005667\|G |
| 1660477 | 2.60E-04 | down | 801 | Homo sapiens calmodulin 1 (phosphoryla | GO:0007049\|GO:0007186\|GO:0032465\|G | GO:0000922\|GO:0005737\|GO:0005813\|G |
| 1580356 | 0.01071826 | down | 11018 | Homo sapiens transmembrane emp24 pr | GO:0006810\|GO:0007165\|GO:0007267 | GO:0005886\|GO:0016021 |
| 5050575 | 0.09260461 | down | 79754 | Homo sapiens ankyrin repeat and SOCS | GO:0006511\|GO:0007242 | |
| 4570255 | 0.5703936 | down | 51176 | Homo sapiens lymphoid enhancer-binding | GO:0006350\|GO:0006355\|GO:0016055\|G | GO:0005634\|GO:0005730\|GO:0005737\|G |
| 360608 | 0.00119191 | up | 9637 | Homo sapiens fasciculation and elongatio | GO:0007165\|GO:0007399\|GO:0007411 | |
| 4730674 | 0.00173498 | down | 6502 | Homo sapiens S-phase kinase-associated | GO:0000082\|GO:0006511\|GO:0008283 | |
| 2070291 | 0.12196246 | down | 5236 | Homo sapiens phosphoglucomutase 1 (P | GO:0005975\|GO:0006006\|GO:0006006 | GO:0005737\|GO:0005829 |
| 3710039 | 2.41E-04 | down | 116541 | Homo sapiens mitochondrial ribosomal protein L54 (MRPL54), nuclear gene encodi | | GO:0005739\|GO:0005840 |
| 1110114 | 0.00358929 | down | 27292 | Homo sapiens DIM1 dimethyladenosine t | GO:0000154 | GO:0005634 |
| 6840328 | 0.17950614 | down | 4088 | Homo sapiens SMAD family member 3 (S | GO:0000122\|GO:0001666\|GO:0001707\|G | GO:0005622\|GO:0005634\|GO:0005654\|G |
| 6620017 | 0.04858149 | down | 283078 | Homo sapiens mohawk homeobox (MKX) | GO:0006355\|GO:0007275\|GO:0007517 | GO:0005634\|GO:0005730 |
| 3610671 | 0.38025782 | down | 79966 | Homo sapiens stearoyl-CoA desaturase 5 | GO:0006629\|GO:0006633\|GO:0055114\|G | GO:0005783\|GO:0005789\|GO:0016020\|G |
| 3170300 | 0.1676194 | down | 9788 | Homo sapiens metastasis suppressor 1 (I | GO:0006928\|GO:0007155\|GO:0007165\|G | GO:0001726\|GO:0005737\|GO:0015629\|G |
| 460358 | 0.00289173 | down | 760 | Homo sapiens carbonic anhydrase II (CA | GO:0002009\|GO:0006730\|GO:0015670\|G | GO:0005634\|GO:0005737 |
| 3460520 | 4.30E-04 | up | 894 | Homo sapiens cyclin D2 (CCND2), mRNA | GO:0001934\|GO:0045737\|GO:0051301\|G | GO:0000307\|GO:0005634 |
| 580132 | 0.02818301 | up | 55353 | Homo sapiens lysosomal protein transme | GO:0006810 | GO:0012505\|GO:0016020\|GO:0016021 |
| 4230195 | 0.18590397 | down | 89797 | Homo sapiens neuron navigator 2 (NAV2), transcript variant 2, mRNA. | | GO:0005634 |
| 1400044 | 0.04717905 | down | 259217 | Homo sapiens heat shock 70kDa protein 12A (HSPA12A), mRNA. | | |
| 2000630 | 0.03441646 | down | 8470 | Homo sapiens sorbin and SH3 domain co | GO:0008150 | GO:0005622\|GO:0005634\|GO:0005737\|G |
| 7000746 | 1.28E-04 | down | 132001 | Homo sapiens chromosome 3 open readi | GO:0015031\|GO:0030150\|GO:0065002 | GO:0005739\|GO:0031314 |
| 6200402 | 0.00309573 | down | 4489 | Homo sapiens metallothionein 1A (MT1A) | GO:0008150 | GO:0005737 |
| 940471 | 0.01029901 | down | 9315 | Homo sapiens chromosome 5 open readi | GO:0017015 | |
| 6200431 | 0.09480499 | down | 207 | Homo sapiens v-akt murine thymoma vira | GO:0000060\|GO:0001568\|GO:0001890\|G | GO:0005634\|GO:0005654\|GO:0005654\|G |
| 3830519 | 0.00100281 | down | 8195 | Homo sapiens McKusick-Kaufman syndro | GO:0006457\|GO:0007286\|GO:0007507\|G | GO:0005622 |
| 7400025 | 0.00635819 | down | 51537 | Homo sapiens mitochondrial protein 18 kI | GO:0006915\|GO:0015976 | GO:0005739 |
| 7320435 | 0.10406482 | down | 54344 | Homo sapiens dolichyl-phosphate manno | GO:0005975\|GO:0006506\|GO:0018406\|G | GO:0005783\|GO:0005789\|GO:0005789\|G |
| 4670059 | 0.6993156 | down | 51776 | Homo sapiens sterile alpha motif and leucine zipper containing kinase AZK (ZAK), transcript variant 2, mRNA. | | |

FIG. 6H

| | | | | | | | |
|---|---|---|---|---|---|---|---|
| 4230132 | 0.00175277 | down | | 3611 | Homo sapiens integrin-linked kinase (ILK) | GO:0001658\|GO:0006468\|GO:0007160\|( | GO:0005737\|GO:0005886\|GO:0005925\|( |
| 4210050 | 0.0181805 | down | | 84058 | Homo sapiens WD repeat domain 54 (WDR54), mRNA. | | |
| 3290685 | 0.00634756 | up | | 1808 | Homo sapiens dihydropyrimidinase-like 2 | GO:0006139\|GO:0007165\|GO:0007275\|( | GO:0005737\|GO:0005739\|GO:0005856\|( |
| 1400520 | 0.00460625 | down | | 26047 | Homo sapiens contactin associated prote | GO:0007155\|GO:0007165\|GO:0008038\|( | GO:0016020\|GO:0016021 |
| 3060128 | 0.00259379 | up | | 22856 | Homo sapiens chondroitin sulfate synthase 1 (CHSY1), mRNA. | | GO:0005794\|GO:0016020\|GO:0016021 |
| 4570524 | 0.01810717 | up | | 22858 | Homo sapiens intestinal cell (MAK-like) ki | GO:0006468\|GO:0007165\|GO:0007243\|( | GO:0005737\|GO:0043231 |
| 3140139 | 0.1246087 | down | | 4747 | Homo sapiens neurofilament, light polype | GO:0008089\|GO:0008090\|GO:0019896\|( | GO:0005882\|GO:0005883\|GO:0030424\|( |
| 7560497 | 0.00739417 | down | | 388394 | Homo sapiens reprimo-like (RPRML), mRNA. | | GO:0016020\|GO:0016021 |
| 2320093 | 0.91719127 | down | | 84992 | Homo sapiens phosphatidylinositol glycar | GO:0006506 | GO:0000506\|GO:0005783\|GO:0005789\|( |
| 2340372 | 0.26788586 | down | | 93621 | Homo sapiens Mof4 family associated protein 1 (MRFAP1), mRNA. | | GO:0005634\|GO:0005737 |
| 650619 | 0.434956 | up | | 89853 | Homo sapiens family with sequence similarity 125, member B (FAM125B), transcript variant 1, mRNA. | | |
| 1690156 | 0.01377497 | up | | 9891 | Homo sapiens NUAK family, SNF1-like ki | GO:0006468 | |
| 2650598 | 0.00308029 | down | | 285440 | Homo sapiens cytochrome P450, family 4 | GO:0007601\|GO:0050896\|GO:0055114 | GO:0005783\|GO:0005789\|GO:0016020\|( |
| 6590300 | 0.9192721 | up | | 388585 | Homo sapiens hairy and enhancer of split | GO:0006355\|GO:0006355\|GO:0007155\|( | GO:0005634\|GO:0005634 |
| 1110605 | 0.02570582 | down | | 24140 | Homo sapiens FtsJ homolog 1 (E. coli) (F | GO:0006364 | |
| 5390333 | 0.01861825 | down | | 26022 | Homo sapiens transmembrane protein 98 (TMEM98), transcript variant 2, mRNA. | | GO:0005783\|GO:0016020\|GO:0016021 |
| 2850291 | 0.00495886 | down | | 23108 | Homo sapiens GTPase activating Rap/Ra | GO:0051056 | GO:0005622\|GO:0005737 |
| 5820601 | 0.060329 | down | | 595 | Homo sapiens cyclin D1 (CCND1), mRNA | GO:0000082\|GO:0000320\|GO:0001934\|( | GO:0000307\|GO:0005575\|GO:0005622\|( |
| 2030132 | 0.02240777 | down | | 653506 | PREDICTED: Homo sapiens similar to meteorin, glial cell differentiation regulator-like (LOC653506), mRNA. | | |
| 5420347 | 0.02945752 | down | | 54985 | Homo sapiens host cell factor C1 regulator 1 (XPO1 dependent) (HCFC1R1), trans( | | GO:0005634\|GO:0005737 |
| 3780762 | 0.03850586 | down | | 91272 | Homo sapiens family with sequence similarity 44, member B (FAM44B), mRNA. | | |
| 870161 | 0.5538471 | up | | 1525 | Homo sapiens coxsackie virus and adeno | GO:0007005\|GO:0007155\|GO:0007507\|( | GO:0005737\|GO:0005794\|GO:0005886\|( |
| 2190674 | 3.62E-04 | down | | 3488 | Homo sapiens insulin-like growth factor b | GO:0001558\|GO:0007165\|GO:0014912\|( | GO:0005783\|GO:0005576\|GO:0016942 |
| 7570446 | 0.08915674 | down | | 6895 | Homo sapiens TAR (HIV-1) RNA binding | GO:0006469\|GO:0045070\|GO:0046726\|( | GO:0005622\|GO:0005622\|GO:0005622\|( |
| 4290706 | 0.00166065 | down | | 2139 | Homo sapiens eyes absent homolog 2 (D | GO:0007275\|GO:0008152\|GO:0006350\|( | GO:0005634\|GO:0005737 |
| 2230367 | 0.03271554 | down | | 64781 | Homo sapiens ceramide kinase (CERK), ( | GO:0006672\|GO:0007205 | GO:0000299\|GO:0005737\|GO:0016020 |
| 3890187 | 0.00282538 | down | | 55170 | Homo sapiens protein arginine methyltrar | GO:0044419 | GO:0005634 |
| 780133 | 0.03981937 | up | | 152503 | Homo sapiens SH3 domain containing 19 (SH3D19), mRNA. | | GO:0005634\|GO:0005737 |
| 4280204 | 0.1886676 | down | | 5245 | Homo sapiens prohibitin (PHB), mRNA. | GO:0006260\|GO:0007165\|GO:0008285\|( | GO:0016020\|GO:0005634\|GO:0005654\|( |
| 5870113 | 9.10E-04 | down | | 80145 | Homo sapiens THO complex 7 homolog (Drosophila) (THOC7), mRNA. | | GO:0005634\|GO:0005737 |
| 520324 | 0.00409405 | down | | 7905 | Homo sapiens receptor accessory protein | GO:0008150 | GO:0016020\|GO:0016021 |
| 6420309 | 0.0518426 | down | | 54463 | Homo sapiens family with sequence similarity 134, member B (FAM134B), transcrip | | GO:0016020\|GO:0016021 |
| | | | | | | | |
| | | | | | | | |
| | | | | | | | |
| | | | | | | | |

FIG. 6I

| ProbeID | Ontology_Function | Chromosome | Chromosome Start Index(Avadis) | Chromosome End Index(Avadis) | Probe_Sequence | SEQ ID NO. |
|---|---|---|---|---|---|---|
| 3360112 | | chr9 | 73488479 | 73488528 | GGCTGATGGCCTCTACCTTTGTATCCAGGAGAAACTGCAGAGCAGCCCTG | 1 |
| 5420095 | GO:0003700\|GO:0003700\|GO:0003700\|( | chr8 | 128822194 | 128822243 | CCTGCGTGACCAGATCCCGGAGTTGGAAAACAATGAAAAGGCCCCCAAGG | 2 |
| 6520139 | GO:0004872\|GO:0004872\|GO:0000166\|( | chr4 | 1780312 | 1780361 | TCACCCAAACCGGCAGGTGCGATTTTGTTAACCCAGCGACGAACTTTCCG | 3 |
| 1850092 | | chr7 | 130835794 | 130835843 | GTCACGGTCACTAGCTGATCCCTCAGGTCTGCTGCAAACACAGCATGGAG | 4 |
| 580711 | GO:0003700\|GO:0004871\|GO:0005515\|( | chr15 | 64861002 | 64861051 | CCAGAGACACAGCCCCCACGGACAAAACCCCCCAGATATCATCTACCTAG | 5 |
| 1660477 | GO:0005509\|GO:0005509\|GO:0019904\|( | chr14 | 89944051 | 89944100 | TGCTGTCTGTATCCCTTGGAGTAAGAAGGTAGTGGCATGGGTGGAGTGTG | 6 |
| 1580356 | GO:0005102 | chr19 | 10804151 | 10804200 | GCCCGGCCAAAGTCTAGGCAGAAGCCTCCTATAACAAAGGGTGGTGTGGC | 7 |
| 5050575 | GO:0005515 | chr10 | 5721023 | 5721072 | CTTCATGCTGGCCGCCGTGAGGTTTTACTCCAGCGTTCCCGCCATTGTCA | 8 |
| 4570255 | GO:0005515\|GO:0005515\|GO:0008301\|( | chr4 | 109188515 | 109188564 | GTAATAGCCAAACCCCACTCTGTTGGTAGCAATTGGCAGCCCTATTTCAG | 9 |
| 360608 | GO:0005515\|GO:0005515 | chr2 | 36633209 | 36633258 | CTGTTGGTGGTGATGGATTTTGTAGCTTGCTGCTTGTTTCACCACTGGTC | 10 |
| 4730674 | GO:0005515\|GO:0005515\|GO:0005515\|( | chr5 | 36207525 | 36207574 | GGCTGTTGCGCATGTGTCAGAGACCATCACCCAGCTGAATCTTAGCGGCT | 11 |
| 2070291 | GO:0000287\|GO:0004614\|GO:0004614\|( | chr1 | 63898163 | 63898212 | GCCCTCCTGCATTGCTGCTGCGTGGGTATTTGTCTCCTTAGCCATCAGGT | 12 |
| 3710039 | | chr19 | 3716282 | 3718260 | GGAAGGGCAGGATGTACCCCTGAAACCGGATGCTGAGTACCCTGAATGGC | 13 |
| 1110114 | GO:0000179\|GO:0003723\|GO:0008168\|( | chr5 | 61720154 | 61720203 | CTAGTCACACATTCCCCATACCATTTCGTGTTATTCACATTCCCCGTACC | 14 |
| 6840328 | GO:0003690\|GO:0003700\|GO:0008134\|( | chr15 | 65274286 | 65274335 | TGCAACTCGGCTGTTCTGGACTCTGATGTGTGTGGAGGGATGGGGAATAG | 15 |
| 6620017 | GO:0003700\|GO:0043565 | chr10 | 28001951 | 28002000 | ATCCCCCCAGTCCTTTAAAAGAATCTTGAACAATGCTGAGCCGGCAGCTG | 16 |
| 3610671 | GO:0004768\|GO:0005506\|GO:0016491\|( | chr4 | 83770373 | 83770422 | GTTTCAAGAGAGATTTGTTCACTGCCCAGCTCGTTTTGTGTCCTGAGCCC | 17 |
| 3170300 | GO:0003785\|GO:0005102 | chr8 | 125632315 | 125632364 | TTGTGCTTTAGTTGCTAGTTTGTACTGAGAGTTGACCTCTCCCTGTGCAG | 18 |
| 460358 | GO:0004089\|GO:0008270\|GO:0016829\|( | chr8 | 86580426 | 86580475 | GACTAGACCAATTGTCATGCTTGACACAACTGCTGTGGCTGGTTGGTGCT | 19 |
| 3460520 | GO:0005515\|GO:0019901 | chr12 | 4284480 | 4284529 | CTCTCACAGTGTACTCTATAAGAGGTGTGGGTGTCTGTTTGGTCAGGATG | 20 |
| 580132 | | chr8 | 98933560 | 98933609 | GAGGGTGGAATGGATGTGTTTGGCGCTGCATGGGATCTGGTGCCCCTCTT | 21 |
| 4230195 | GO:0000166\|GO:0004386\|GO:0005524\|( | chr11 | 20099593 | 20099642 | TTGTCATGAGGTTTTTGGATTTGCCAATGATCTGCTGGACATCATGCCCC | 22 |
| 1400044 | GO:0000166\|GO:0005524 | chr10 | 118420717 | 118420766 | CACCACCAGACGGGACTGTGTCTGCAAACTCTCTTGTCTTCACAGACGCC | 23 |
| 2000630 | GO:0005200\|GO:0005515\|GO:0008093\|( | chr4 | 186744115 | 186744164 | GCAGACTAGCCTCTCCTTATCTCACAGATCACAAGCACCCCTAGATAGTG | 24 |
| 7000746 | | chr3 | 11807042 | 11826026 | GCTCATGATCCCGACTGTGGAGATGTGGTGCGACTAGGCCTGAAGAAGTC | 25 |
| 6200402 | GO:0005507\|GO:0008270\|GO:0046870\|( | chr16 | 55231274 | 55231323 | TGCTGCTCCTGCTGCCCCATGAGCTGTGCCAAGTGTGCCCAGGGCTGCAT | 26 |
| 940471 | GO:0005515 | chr5 | 111093165 | 111093214 | CAGGGCAGGCCTTGTTCTGAACTGTTTCGCTTCTGACTGTTAAACACCGA | 27 |
| 6200431 | GO:0000166\|GO:0004674\|GO:0004674\|( | chr14 | 104306911 | 104306960 | CCACGGTAGCACTTGACCTTTTCGACGCTTAACCTTTCCGCTGTCGCCCC | 28 |
| 3830519 | GO:0000166\|GO:0005524\|GO:0051082\|( | chr20 | 10333950 | 10333999 | CCTGACCTTGGACTGTTTGACTGCAAAGCTTAGTGGCCTACAGGTGGCTG | 29 |
| 7400025 | GO:0004089\|GO:0008270 | chr22 | 29154916 | 29154965 | GGTGGTATGGCTGAACAAGGAGCGGCAGACAACTCAGGGAGAAACTCAGG | 30 |
| 7320435 | GO:0004582\|GO:0005515 | chr1 | 153379297 | 153379591 | AGTGACCATGACGAAATTAGCGCAGTGGCTTTGGGGACTAGCGATCCTGG | 31 |
| 4670059 | | chr2 | 173799586 | 173799635 | CTCCACCTTTGTTTGCACTCTGTTGCCTGTGAGGAGCTTTCTGGCATGTG | 32 |

FIG. 6J

| | | | | | | |
|---|---|---|---|---|---|---|
| 4230132 | GO:0000166\|GO:0004674\|GO:0004713\| | chr11 | 6588580 | 6588629 | CAAGAGGGGCGGGCTCAGAGCTTTGTCACTTGCCACATGGTGTCTCCCAA | 33 |
| 4210050 | | chr2 | 74506263 | 74506312 | GTGATTCCTCAGGCAACTCCTTTGCTGTGACTGGCTATGACCTTGCGGAG | 34 |
| 3290685 | GO:0016810\|GO:0004157\|GO:0005515\| | chr8 | 26571233 | 26571282 | CTGCAGGCTGTGTAAGCATGTTTACCTGTTGGCTTGCTTTGTGTGTCTGT | 35 |
| 1400520 | GO:0005102 | chr7 | 147453078 | 147453127 | TTTTCAGTGAGCGTGGTGACTGCAGAGGTTAGTGCTGTGAAAAGCTGGGC | 36 |
| 3060128 | GO:0016740\|GO:0046872\|GO:0047238\| | chr15 | 99533553 | 99533602 | ACACGTCATTGGAGGGCTGCGTATTTGTAAATAGCCTGATGCTCATTTGG | 37 |
| 4570524 | GO:0000166\|GO:0000287\|GO:0004674\| | chr6 | 52974628 | 52974677 | AGTAGCATTTCCCTACCATCAAGCCATTGTTTTGTGCCATTCAGGAGAGG | 38 |
| 3140139 | GO:0005198\|GO:0005200\|GO:0008022\| | chr8 | 24867064 | 24867113 | CAGACCAGCTCCTATCTGATGTCCACCCGCTCCTTCCCGTCCTACTACAC | 39 |
| 7560497 | | chr17 | 42410556 | 42410605 | GCGTCAGAGTCGCTGAGCTTGTTGGCCTGATCTTGCGTTTGGAAAGAAAT | 40 |
| 2320093 | O:0005886\|GO:0016021 | chr4 | 89661596 | 89661645 | GCTTTGCGCATATCAGGCTTAGGACTGTGGGAGGCTTAAGTTGCAGATGC | 41 |
| 2340372 | GO:0005515\|GO:0005515\|GO:0005515 | chr4 | 6694849 | 6694898 | CCGACCTGGCTGGGACTCGTGAATCTGGAGAAGAGCTGGAGAATGGATAG | 42 |
| 650619 | | chr9 | 128308937 | 128308986 | ACCAGAGACGGCTTTGTTCTCCCAGCTAAGGCCGTGGAGCTGCTGTGTGA | 43 |
| 1690156 | GO:0000166\|GO:0000287\|GO:0004674\| | chr12 | 104981731 | 104981780 | GATGGGGAGCACTTTTCAGGGTGAAATTCAGGCGAGTTTTGCCCAGGCCT | 44 |
| 2650598 | GO:0004497\|GO:0005506\|GO:0009055\| | chr4 | 187371417 | 187371466 | GATGATCAAGGTTTGTGTGCCCATTACCTTTCCTCTGCCTGAAAGACGTG | 45 |
| 6590300 | GO:0003677\|GO:0030528\|GO:0003677\| | chr1 | 2450120 | 2450169 | GGATTCCTCTGTGTGGGTGGATGCGTGTGGGCACGACTTTGTACTCAGAA | 46 |
| 1110605 | GO:0008168\|GO:0016740 | chrX | 48229320 | 48229369 | TCCCTAAAGGCAGGGATTCTTAACCTGGATAGAAGCCAGGGGTAACCATG | 47 |
| 5390333 | | chr17 | 28292595 | 28292644 | GGCCTGTTTCATTTATTTGTATTATCTGCCTGGTCCCTGAGGCGTCTGGG | 48 |
| 2850291 | GO:0005096 | chr17 | 2887454 | 2887503 | CTCTCTCCTGTTTTGGGTGTTACCCTTGGACACTCCAGCTCGGGGACTGC | 49 |
| 5820601 | GO:0004672\|GO:0005515\|GO:0005515\| | chr11 | 69178329 | 69178378 | CGGCGCTTCCCAGCACCAACATGTAACCGGCATGTTTCCAGCAGAAGACA | 50 |
| 2030132 | | chr17 | 7533 | 7582 | TTTCAGGCCGCCGGTTGTTTCCGTTCCCGAGAATAAAGACGAGGATCCGA | 51 |
| 5420347 | | chr16 | 3012708 | 3012757 | CTGGGCACCAAGACCTTCCCTCAACAGAGGACACTGAGCCCAACGGAGTT | 52 |
| 3780762 | | chr5 | 172967249 | 172967298 | TAGGCATAAGGAAACTCGTTTGCAGGCTCTCTGTCCAGGGCTGCTTCCTG | 53 |
| 870161 | GO:0004872\|GO:0005515 | chr21 | 17860695 | 17860744 | GGTGGCCAGCCAGATCAAGGATGTAGTATCTCATAGTTCCCAGGTGATAT | 54 |
| 2190674 | GO:0031994 | chr2 | 217245529 | 217245578 | GCACAGTTGTCAGACAAGATTCCTTCAGATTCCGAGTTGCCTACCGGTTG | 55 |
| 7570446 | GO:0003725\|GO:0046982\|GO:0003725\| | chr12 | 52186326 | 52186375 | CTTGAAGCTGAGAAGGCACAGGGCAAGGAGCCAAGGACCACAGAGCCTCA | 56 |
| 4290706 | GO:0003824\|GO:0000287\|GO:0004725\| | chr20 | 45250552 | 45250601 | CTCCGGAATTATGCTCTTGTACCTGTGTGGCTGGGTTTCTTAGTCGTTGG | 57 |
| 2230367 | GO:0000287\|GO:0001729\|GO:0004143\| | chr22 | 45459303 | 45459352 | GCTCTGATTTCCGGGGCAGCCTTTCAGATGCGGCAGACATACAACACCTG | 58 |
| 3890187 | GO:0005515\|GO:0008168\|GO:0016740 | chr1 | 107402806 | 107402855 | TTCTTATGAAACAGGCTGGTAGAGGAGGTTTCTGAGCCTAGCCCAAGGGC | 59 |
| 780133 | | chr4 | 152261295 | 152261344 | CAAGTGGCCACACTTTTACGTGACTACAACCTGGAGTTCTGCAAAGAAGG | 60 |
| 4280204 | GO:0005515\|GO:0016563\|GO:0016564 | chr17 | 44836462 | 44836511 | TTCAGAGACTGCCCTTCTCACGGGCTCTATGCCTGCACTGGGAAGGAAAC | 61 |
| 5870113 | | chr3 | 63794820 | 63794869 | TGGAATCAGATCCTAAGCCATAGACAGGCTAATTGCCCACCACTCCCAGG | 62 |
| 520324 | GO:0003674\|GO:0005515 | chr5 | 112240078 | 112240127 | TGGATCAACAACTGCTACTCTCGGGAAGACTCCTCTACTCACAGCTGAAG | 63 |
| 6420309 | | chr5 | 16526306 | 16526355 | GCCTCATGACTGTGTTTGATGTCCTTTATTGATACAAAGTGAGCCTGTGC | 64 |
| | | | | | | |
| | | | | | | |
| | | | | | | |
| | | | | | | |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20100169025 A, Arthur
- US 5569588 A
- US 61470919 B
- US 20110143338 A
- US 20070231810 A
- WO 2008122084 A
- WO 2007041774 A
- US 7232822 B
- WO 2005021025 A
- WO 2007062243 A
- WO 2008061020 A
- WO 0039339 A
- WO 2004065545 A
- US 7171311 B
- WO 2002103320 A
- WO 2005086891 A
- WO 2006015312 A
- WO 2006084272 A
- US 20030104426 A, Linsley
- US 20070154931 A, Radish


### Non-patent literature cited in the description


- **HUELSKEN ; BIRCHMEIER.** *Curr. Opin. Genet. Dev.,* 2001, vol. 11, 547-553
- **CLEVERS.** *Cell,* 2006, vol. 127, 469-480
- **WILLERT et al.** *EMBO J.,* 1997, vol. 16, 3089-3096
- **STAAL et al.** *EMBO Rep.,* 2002, vol. 3, 63-68
- **VLAD et al.** *Cellular Signaling,* 2008, vol. 20, 795-802
- **KORINEK et al.** *Nat. Genet.,* 1998, vol. 19, 1-5
- **VAN ES et al.** *Nature,* 2005, vol. 435, 959-963
- **VAN GENDEREN et al.** *Genes Dev.,* 1994, vol. 8, 2691-2703
- **CLEVERS.** *Cell,* 2006, 469-480
- **GREGORIEFF ; CLEVERS.** *Genes Dev.,* 2005, vol. 19, 877-890
- **LOGAN ; NUSSE.** *Annu. Rev. Cell Dev. Biol.,* 2004, vol. 20, 781-810
- **FODDE ; BRABLETZ.** *Curr. Opin. Cell Biol.,* 2007, vol. 19, 150-158
- **ROTHENBERG et al.** *Nat. Rev. Cancer,* 2003, vol. 3, 303-309
- **DRACOPOLI.** *Curr. Mol. Med.,* 2005, vol. 5, 103-110
- **FOLGUERIA et al.** *Clin. Cancer Res.,* 2005, vol. 11, 7434-7443
- **CHANG et al.** *Lancet,* 2003, vol. 362, 362-369
- **ROUZIER et al.** *Proc. Natl. Acad. Sci. USA,* 2005, vol. 102, 8315-8320
- **HAHN ; WEINBERG.** *Nat. Rev. Cancer,* 2002, vol. 2, 331-341
- **HANAHAN ; WEINBERG.** *Cell,* 2000, vol. 100, 57-70
- **TROSKO et al.** *Ann. N.Y. Acad. Sci.,* 2004, vol. 1028, 192-201
- **ADJEI ; HILDALGO.** *J. Clin. Oncol.,* 2005, vol. 23, 5386-5403
- **POLAKIS.** *Genes Dev.,* 2000, vol. 14, 1837-1851
- **MORIN et al.** *Science,* 1997, vol. 275, 1787-1790
- **RUBINFELD et al.** *Science,* 1997, vol. 275, 1790-1792
- **KOCH et al.** *Cancer Res.,* 1999, vol. 59, 269-273
- **ZURAWEL et al.** *Cancer Res.,* 1998, vol. 58, 896-899
- **VOELLER et al.** *Cancer Res.,* 1998, vol. 58, 2520-2523
- **SCHLOSSHAUER et al.** *Mod. Pathol.,* 2000, vol. 13, 1066-1071
- **MIRABELLI-PRIMDAHL et al.** *Cancer Res.,* 1999, vol. 59, 3346-3351
- **SAEGUSA ; OKAYASU.** *J. Pathol.,* 2001, vol. 194, 59-67
- **WU et al.** *Cancer Res.,* 2001, vol. 61, 8247-8255
- **HAYES et al.** *Clin. Cancer Res.,* 2008, vol. 14, 4038-4044
- **HUANG et al.** *BMC Cancer,* 2006, vol. 6 (1), 221
- **LEE et al.** *Life Sciences,* 2007, vol. 80 (7), 690-698
- **SHIN et al.** *Journal of dermatological Science,* 2010, vol. 58 (2), 91-96
- **MOKANY et al.** *J Am Chem Soc.,* 27 January 2010, vol. 132 (3), 1051-1059
- **WANG et al.** *J. Surg. Res.,* 27 June 2008

- **DOGHMAN et al.** *J. Clin. Endocrinol. Metab.,* 2008
- **YAMAKAWA et al.** *Biol Pharm. Bull.,* 2008, vol. 31, 916-920
- **HANDELI ; SIMON.** *Mol. Cancer Ther.,* 2008, vol. 7, 521-529
- **HAN et al.** *Eur. J. Pharmacol.,* 2008, vol. 583, 26-31
- **TUYNMAN et al.** *Cancer Res.,* 2008, vol. 68, 1213-1220
- **SAMBROOK et al.** MOLECULAR CLONING--A LABORATORY MANUAL. Cold Spring Harbor Laboratory, 1989, vol. 1-3
- **AUSUBEL et al.** CURRENT PROTOCOLS IN MOLECULAR BIOLOGY. Current Protocols Publishing, 1994, vol. 2
- **CHIRGWIN et al.** *Biochemistry,* 1979, vol. 18, 5294-5299
- **LUU et al.** *Curr. Cancer Drug Targets,* 2004, vol. 4, 653-671
- **REYA ; CLEVERS.** *Nature,* 2005, vol. 434, 843-850
- **MOON et al.** *Nat. Rev. Genet.,* 2004, vol. 5, 691-701
- **BOYDEN et al.** *N. Engl. J. Med.,* 2002, vol. 346, 1513-1521
- **GONG et al.** *Cell,* 2001, vol. 107, 513-523
- **LITTLE et al.** *Am. J. Hum. Genet.,* 2002, vol. 70, 11-19
- **DIANA et al.** *Nat. Med.,* 2007, vol. 13, 156-163
- **BARON ; RAWADI.** *Endocrin.,* 2007, vol. 148, 2635-2643
- **KIM et al.** *J. Bone Mineral Res,* 2007, vol. 22, 1913-1923
- **JIN.** *Diabetologia,* 12 August 2008
- **LAD et al.** *Stem Cells Dev.,* 08 August 2008
- **CARACI et al.** *Neurochem. Res.,* 22 April 2008
- **DIARRA et al.** *Nat. Med.,* 2007, vol. 13, 156-163
- **KIM et al.** *J. Bone Mineral Res.,* 2007, vol. 22, 1913-1923
- **HAN et al.** *Fur. J. Pharmacol.,* 2008, vol. 583, 26-31
- **CRISTIANINI ; SHAWE-TAYLOR.** An Introduction to Support Vector Machines. Cambridge University Press, 2000
- A training algorithm for optimal margin classifiers. **BASER et al.** Proceedings of the 5th Annual ACM Workshop on Computational Learning Theory. ACM Press, 1992, 142-152
- **VAPNIK.** Statistical Learning Theory. Wiley, 1998
- **DUDA.** Pattern Classification. John Wiley & Sons, Inc, 2001
- **HASTIE.** The Elements of Statistical Learning. Springer, 2001
- **FUREY et al.** *Bioinformatics,* 2000, vol. 16, 906-914
- **JAAKKOLA et al.** Proceedings of the 7th International Conference on Intelligent Systems for Molecular Biology. AAAI Press, 1999
- **BROWN et al.** *Proc. Natl. Acad. Sci.,* 2000, vol. 97 (1), 262-67
- **ZIEN et al.** *Bioinformatics,* 2000, vol. 16 (9), 799-807
- **FUREY et al.** *Bioinformatics,* 2000, vol. 16 (10), 906-914
- **AGRESTI.** An Introduction to Categorical Data Analysis. John Wiley & Sons, Inc, 1996
- **DUDA.** Pattern Classification. John Wiley & Sons, Inc, 2001
- **VENABLES ; RIPLEY.** Modern Applied Statistics with s-plus. Springer, 1997
- **DUDA ; HART.** Pattern Classification and Scene Analysis. John Wiley & Sons, Inc, 1973, 211-256
- **DUDA et al.** Pattern Classification. John Wiley & Sons, Inc, 537-563
- **KAUFMAN ; ROUSSEEUW.** Finding Groups in Data: An Introduction to Cluster Analysis. Wiley, 1990
- **EVERITT.** Cluster analysis. Wiley, 1993
- **BACKER.** Computer-Assisted Reasoning in Cluster Analysis. Prentice Hall, 1995
- **KUBINYI.** 3D QSAR in drug design theory methods and applications. Pergamon Press, 1990, 589-638
- **ANDERSON.** Cluster Analysis for applications. Academic Press, 1973
- **GORDON.** Classification. Chapman and Hall, CRC, 1999
- **BREIMAN.** *Machine Learning,* 1996, vol. 24, 123-140
- **EFRON ; TIBSHIRANI.** An Introduction to Bootstrap. Chapman & Hall, 1993
- **FREUND ; SCHAPIRE.** Experiments with a new boosting algorithm. *Proceedings 13th International Conference on Machine Learning,* 1996, 148-156
- **FREUND ; SCHAPIRE.** *Journal of Computer and System Sciences,* 1997, vol. 55, 119-139
- **PARK et al.** *Pac. Symp. Biocomput.,* 2002, vol. 6, 52-63
- **FRIEDMAN et al.** *Ann Stat,* 2000, vol. 28, 337-407
- **BEN-DOR et al.** *Journal of Computational Biology,* 2000, vol. 7, 559-583
- **HO.** The Random subspace method for constructing decision forests. *IEEE Trans Pattern Analysis and Machine Intelligence,* 1998, vol. 20 (8), 832-844

- *Proc Natl Acad Sci USA.,* 14 May 2002, vol. 99 (10), 6567-72

SEQUENCE LISTING

<110> QIAGEN
Wu, Zhong
DiCarlo, John
Wang, Yexun

<120> GENE EXPRESSION SIGNATURE FOR WNT/B-CATENIN SIGNALING PATHWAY AND USE THEREOF

<130> 74708.010002

<140> tba
<141> 2012-04-02

<150> 61/470,919
<151> 2011-04-01

<160> 80

<170> PatentIn version 3.5

<210> 1
<211> 50
<212> DNA
<213> ARTIFICIAL

<220>
<223> PCR PRIMER

<400> 1
ggctgatggc ctctaccttt gtatccagga gaaactgcag agcagccctg 50

<210> 2
<211> 50
<212> DNA
<213> ARTIFICIAL

<220>
<223> PCR PRIMER

<400> 2
cctgcgtgac cagatcccgg agttggaaaa caatgaaaag gcccccaagg 50

<210> 3
<211> 50
<212> DNA
<213> ARTIFICIAL

<220>
<223> PCR PRIMER

<400> 3
tcacccaaac cggcaggtgc gattttgtta acccagcgac gaactttccg 50

<210> 4
<211> 50
<212> DNA
<213> ARTIFICIAL

<220>
<223> PCR PRIMER

<400> 4
gtcacggtca ctagctgatc cctcaggtct gctgcaaaca cagcatggag 50

<210> 5
<211> 50
<212> DNA
<213> ARTIFICIAL

<220>
<223> PCR PRIMER

<400> 5
ccagagacac agcccccacg gacaaaaccc cccagatatc atctacctag 50

<210> 6
<211> 50
<212> DNA
<213> ARTIFICIAL

<220>
<223> PCR PRIMER

<400> 6
tgctgtctgt atcccttgga gtaagaaggt agtggcatgg gtggagtgtg 50

<210> 7
<211> 50
<212> DNA

<213> ARTIFICIAL

<220>
<223> PCR PRIMER

<400> 7
gcccggccaa agtctaggca gaagcctcct ataacaaagg gtggtgtggc 50

<210> 8
<211> 50
<212> DNA
<213> ARTIFICIAL

<220>
<223> PCR PRIMER

<400> 8
cttcatgctg gccgccgtga ggttttactc cagcgttccc gccattgtca 50

<210> 9
<211> 50
<212> DNA
<213> ARTIFICIAL

<220>
<223> PCR PRIMER

<400> 9
gtaatagcca aaccccactc tgttggtagc aattggcagc cctatttcag 50

<210> 10
<211> 50
<212> DNA
<213> ARTIFICIAL

<220>
<223> PCR PRIMER

<400> 10
ctgttggtgg tgatggattt tgtagcttgc tgcttgtttc accactggtc 50

<210> 11
<211> 50
<212> DNA
<213> ARTIFICIAL

<220>
<223> PCR PRIMER

<400> 11
ggctgttgcg catgtgtcag agaccatcac ccagctgaat cttagcggct 50

<210> 12
<211> 50
<212> DNA
<213> ARTIFICIAL

<220>

<223> PCR PRIMER

<400> 12
gccctcctgc attgctgctg cgtgggtatt tgtctcctta gccatcaggt 50

<210> 13
<211> 50
<212> DNA
<213> ARTIFICIAL

<220>
<223> PCR PRIMER

<400> 13
ggaagggcag gatgtacccc tgaaaccgga tgctgagtac cctgaatggc 50

<210> 14
<211> 50
<212> DNA
<213> ARTIFICIAL

<220>
<223> PCR PRIMER

<400> 14
ctagtcacac attccccata ccatttcgtg ttattcacat tccccgtacc 50

<210> 15
<211> 50
<212> DNA
<213> ARTIFICIAL

<220>
<223> PCR PRIMER

<400> 15
tgcaactcgg ctgttctgga ctctgatgtg tgtggaggga tggggaatag 50

<210> 16
<211> 50
<212> DNA
<213> ARTIFICIAL

<220>
<223> PCR PRIMER

<400> 16
atccccccag tcctttaaaa gaatcttgaa caatgctgag ccggcagctg 50

<210> 17
<211> 50
<212> DNA
<213> ARTIFICIAL

<220>
<223> PCR PRIMER

<400> 17

gtttcaagag agatttgttc actgcccagc tcgttttgtg tcctgagccc 50

<210> 18
<211> 50
<212> DNA
<213> ARTIFICIAL

<220>
<223> PCR PRIMER

<400> 18
ttgtgcttta gttgctagtt tgtactgaga gttgacctct ccctgtgcag 50

<210> 19
<211> 50
<212> DNA
<213> ARTIFICIAL

<220>
<223> PCR PRIMER

<400> 19
gactagacca attgtcatgc ttgacacaac tgctgtggct ggttggtgct 50

<210> 20
<211> 50
<212> DNA
<213> ARTIFICIAL

<220>
<223> PCR PRIMER

<400> 20
ctctcacagt gtactctata agaggtgtgg gtgtctgttt ggtcaggatg 50

<210> 21
<211> 50
<212> DNA
<213> ARTIFICIAL

<220>
<223> PCR PRIMER

<400> 21
gagggtggaa tggatgtgtt tggcgctgca tgggatctgg tgcccctctt 50

<210> 22
<211> 50
<212> DNA
<213> ARTIFICIAL

<220>
<223> PCR PRIMER

<400> 22
ttgtcatgag gtttttggat ttgccaatga tctgctggac atcatgcccc 50

<210> 23

<211> 50
<212> DNA
<213> ARTIFICIAL

<220>
<223> PCR PRIMER

<400> 23
caccaccaga cgggactgtg tctgcaaact ctcttgtctt cacagacgcc 50

<210> 24
<211> 50
<212> DNA
<213> ARTIFICIAL

<220>
<223> PCR PRIMER

<400> 24
gcagactagc ctctccttat ctcacagatc acaagcaccc ctagatagtg 50

<210> 25
<211> 50
<212> DNA
<213> ARTIFICIAL

<220>
<223> PCR PRIMER

<400> 25
gctcatgatc ccgactgtgg agatgtggtg cgactaggcc tgaagaagtc 50

<210> 26
<211> 50
<212> DNA
<213> ARTIFICIAL

<220>
<223> PCR PRIMER

<400> 26
tgctgctcct gctgccccat gagctgtgcc aagtgtgccc agggctgcat 50

<210> 27
<211> 50
<212> DNA
<213> ARTIFICIAL

<220>
<223> PCR PRIMER

<400> 27
cagggcaggc cttgttctga actgtttcgc ttctgactgt taaacaccga 50

<210> 28
<211> 50
<212> DNA
<213> ARTIFICIAL

<220>
<223> PCR PRIMER

<400> 28
ccacggtagc acttgacctt ttcgacgctt aacctttccg ctgtcgcccc 50

<210> 29
<211> 50
<212> DNA
<213> ARTIFICIAL

<220>
<223> PCR PRIMER

<400> 29
cctgaccttg gactgtttga ctgcaaagct tagtggccta caggtggctg 50

<210> 30
<211> 50
<212> DNA
<213> ARTIFICIAL

<220>
<223> PCR PRIMER

<400> 30
ggtggtatgg ctgaacaagg agcggcagac aactcaggga gaaactcagg 50

<210> 31
<211> 50
<212> DNA
<213> ARTIFICIAL

<220>
<223> PCR PRIMER

<400> 31
agtgaccatg acgaaattag cgcagtggct ttggggacta gcgatcctgg 50

<210> 32
<211> 50
<212> DNA
<213> ARTIFICIAL

<220>
<223> PCR PRIMER

<400> 32
ctccaccttt gtttgcactc tgttgcctgt gaggagcttt ctggcatgtg 50

<210> 33
<211> 50
<212> DNA
<213> ARTIFICIAL

<220>
<223> PCR PRIMER

<400> 33
caagaggggc gggctcagag ctttgtcact tgccacatgg tgtctcccaa 50

<210> 34
<211> 50
<212> DNA
<213> ARTIFICIAL

<220>
<223> PCR PRIMER

<400> 34
gtgattcctc aggcaactcc tttgctgtga ctggctatga ccttgcggag 50

<210> 35
<211> 50
<212> DNA
<213> ARTIFICIAL

<220>
<223> PCR PRIMER

<400> 35
ctgcaggctg tgtaagcatg tttacctgtt ggcttgcttt gtgtgtctgt 50

<210> 36
<211> 50
<212> DNA
<213> ARTIFICIAL

<220>
<223> PCR PRIMER

<400> 36
ttttcagtga gcgtggtgac tgcagaggtt agtgctgtga aaagctgggc 50

<210> 37
<211> 50
<212> DNA
<213> ARTIFICIAL

<220>
<223> PCR PRIMER

<400> 37
acacgtcatt ggagggctgc gtatttgtaa atagcctgat gctcatttgg 50

<210> 38
<211> 50
<212> DNA
<213> ARTIFICIAL

<220>
<223> PCR PRIMER

<400> 38
agtagcattt ccctaccatc aagccattgt tttgtgccat tcaggagagg 50

<210> 39
<211> 50
<212> DNA
<213> ARTIFICIAL

<220>
<223> PCR PRIMER

<400> 39
cagaccagct cctatctgat gtccacccgc tccttcccgt cctactacac 50

<210> 40
<211> 50
<212> DNA
<213> ARTIFICIAL

<220>
<223> PCR PRIMER

<400> 40
gcgtcagagt cgctgagctt gttggcctga tcttgcgttt ggaaagaaat 50

<210> 41
<211> 50
<212> DNA
<213> ARTIFICIAL

<220>
<223> PCR PRIMER

<400> 41
gctttgcgca tatcaggctt aggactgtgg gaggcttaag ttgcagatgc 50

<210> 42
<211> 50
<212> DNA
<213> ARTIFICIAL

<220>
<223> PCR PRIMER

<400> 42
ccgacctggc tgggactcgt gaatctggag aagagctgga gaatggatag 50

<210> 43
<211> 50
<212> DNA
<213> ARTIFICIAL

<220>
<223> PCR PRIMER

<400> 43
accagagacg gctttgttct cccagctaag gccgtggagc tgctgtgtga 50

<210> 44
<211> 50
<212> DNA

<213> ARTIFICIAL

<220>
<223> PCR PRIMER

<400> 44
gatggggagc acttttcagg gtgaaattca ggcgagtttt gcccaggcct 50

<210> 45
<211> 50
<212> DNA
<213> ARTIFICIAL

<220>
<223> PCR PRIMER

<400> 45
gatgatcaag gtttgtgtgc ccattacctt tcctctgcct gaaagacgtg 50

<210> 46
<211> 50
<212> DNA
<213> ARTIFICIAL

<220>
<223> PCR PRIMER

<400> 46
ggattcctct gtgtgggtgg atgcgtgtgg gcacgacttt gtactcagaa 50

<210> 47
<211> 50
<212> DNA
<213> ARTIFICIAL

<220>
<223> PCR PRIMER

<400> 47
tccctaaagg cagggattct taacctggat agaagccagg ggtaaccatg 50

<210> 48
<211> 50
<212> DNA
<213> ARTIFICIAL

<220>
<223> PCR PRIMER

<400> 48
ggcctgtttc atttatttgt attatctgcc tggtccctga ggcgtctggg 50

<210> 49
<211> 50
<212> DNA
<213> ARTIFICIAL

<220>

<223> PCR PRIMER

<400> 49
ctctctcctg ttttgggtgt tacccttgga cactccagct cggggactgc 50

<210> 50
<211> 50
<212> DNA
<213> ARTIFICIAL

<220>
<223> PCR PRIMER

<400> 50
cggcgcttcc cagcaccaac atgtaaccgg catgtttcca gcagaagaca 50

<210> 51
<211> 50
<212> DNA
<213> ARTIFICIAL

<220>
<223> PCR PRIMER

<400> 51
tttcaggccg ccggttgttt ccgttcccga gaataaagac gaggatccga 50

<210> 52
<211> 50
<212> DNA
<213> ARTIFICIAL

<220>
<223> PCR PRIMER

<400> 52
ctgggcacca agaccttccc tcaacagagg acactgagcc caacggagtt 50

<210> 53
<211> 50
<212> DNA
<213> ARTIFICIAL

<220>
<223> PCR PRIMER

<400> 53
taggcataag gaaactcgtt tgcaggctct ctgtccaggg ctgcttcctg 50

<210> 54
<211> 50
<212> DNA
<213> ARTIFICIAL

<220>
<223> PCR PRIMER

<400> 54

ggtggccagc cagatcaagg atgtagtatc tcatagttcc caggtgatat 50

<210> 55
<211> 50
<212> DNA
<213> ARTIFICIAL

<220>
<223> PCR PRIMER

<400> 55
gcacagttgt cagacaagat tccttcagat tccgagttgc ctaccggttg 50

<210> 56
<211> 50
<212> DNA
<213> ARTIFICIAL

<220>
<223> PCR PRIMER

<400> 56
cttgaagctg agaaggcaca gggcaaggag ccaaggacca cagagcctca 50

<210> 57
<211> 50
<212> DNA
<213> ARTIFICIAL

<220>
<223> PCR PRIMER

<400> 57
ctccggaatt atgctcttgt acctgtgtgg ctgggtttct tagtcgttgg 50

<210> 58
<211> 50
<212> DNA
<213> ARTIFICIAL

<220>
<223> PCR PRIMER

<400> 58
gctctgattt ccggggcagc ctttcagatg cggcagacat acaacacctg 50

<210> 59
<211> 50
<212> DNA
<213> ARTIFICIAL

<220>
<223> PCR PRIMER

<400> 59
ttcttatgaa acaggctggt agaggaggtt tctgagccta gcccaagggc 50

<210> 60

<211> 50
<212> DNA
<213> ARTIFICIAL

<220>
<223> PCR PRIMER

<400> 60
caagtggcca cacttttacg tgactacaac ctggagttct gcaaagaagg 50

<210> 61
<211> 50
<212> DNA
<213> ARTIFICIAL

<220>
<223> PCR PRIMER

<400> 61
ttcagagact gcccttctca cgggctctat gcctgcactg ggaaggaaac 50

<210> 62
<211> 50
<212> DNA
<213> ARTIFICIAL

<220>
<223> PCR PRIMER

<400> 62
tggaatcaga tcctaagcca tagacaggct aattgcccac cactcccagg 50

<210> 63
<211> 50
<212> DNA
<213> ARTIFICIAL

<220>
<223> PCR PRIMER

<400> 63
tggatcaaca actgctactc tcgggaagac tcctctactc acagctgaag 50

<210> 64
<211> 50
<212> DNA
<213> ARTIFICIAL

<220>
<223> PCR PRIMER

<400> 64
gcctcatgac tgtgtttgat gtcctttatt gatacaaagt gagcctgtgc 50

<210> 65
<211> 21
<212> DNA
<213> ARTIFICIAL

<220>
<223> siRNA duplex

<400> 65
gttccgaatg tctgaggaca a 21

<210> 66
<211> 21
<212> DNA
<213> ARTIFICIAL

<220>
<223> siRNA duplex

<400> 66
acactaagta cgtcgtatta c 21

<210> 67
<211> 21
<212> DNA
<213> ARTIFICIAL

<220>
<223> PCR PRIMER

<400> 67
acttgcattg tgattggcct g 21

<210> 68
<211> 25
<212> DNA
<213> ARTIFICIAL

<220>
<223> PCR PRIMER

<400> 68
aatccatttg tattgttact cctcg 25

<210> 69
<211> 19
<212> DNA
<213> ARTIFICIAL

<220>
<223> PCR PRIMER

<400> 69
agatccggag cgaataggg 19

<210> 70
<211> 21
<212> DNA
<213> ARTIFICIAL

<220>
<223> PCR PRIMER

<400> 70
ccttgctcgg gtgttgtaag t 21

<210> 71
<211> 24
<212> DNA
<213> ARTIFICIAL

<220>
<223> PCR PRIMER

<400> 71
ttgtacctgt aggactctca ttcg 24

<210> 72
<211> 21
<212> DNA
<213> ARTIFICIAL

<220>
<223> PCR PRIMER

<400> 72
cactgtgagc tggcttcatt g 21

<210> 73
<211> 21
<212> DNA
<213> ARTIFICIAL

<220>
<223> PCR PRIMER

<400> 73
ggacagtatc ccacatccaa c 21

<210> 74
<211> 22
<212> DNA
<213> ARTIFICIAL

<220>
<223> PCR PRIMER

<400> 74
caaattgtgc tagagaggcc ag 22

<210> 75
<211> 19
<212> DNA
<213> ARTIFICIAL

<220>
<223> PCR PRIMER

<400> 75
ggcctgctag aatcactgc 19

<210> 76
<211> 25
<212> DNA
<213> ARTIFICIAL

<220>
<223> PCR PRIMER

<400> 76
ctccctacaa ctgttcattt ctttg 25

<210> 77
<211> 21
<212> DNA
<213> ARTIFICIAL

<220>
<223> PCR PRIMER

<400> 77
ccagctccta ctacagccac g 21

<210> 78
<211> 21
<212> DNA
<213> ARTIFICIAL

<220>
<223> PCR PRIMER

<400> 78
tgtgtgtaga cgggttgttc c 21

<210> 79
<211> 21
<212> DNA
<213> ARTIFICIAL

<220>
<223> siRNA duplex

<400> 79
gcagttcgcc ttcactatgg a 21

<210> 80
<211> 21
<212> DNA
<213> ARTIFICIAL

<220>
<223> siRNA duplex

<400> 80
acactaagta cgtcgtatta c 21